# EUROPEAN PATENT SPECIFICATION

(11) **EP 4 096 675 B1**
(45) Date of publication and mention of the grant of the patent: **22.10.2025**
(21) Application number: 21818146.9
(22) Date of filing: 07.06.2021
(51) Int. Cl.: A61K 31/70, A61K 31/713, C12N 15/11, C12N 15/113, C12N 15/117, A61P 25/00, A61P 31/12

(54) **COMPOSITIONS FOR TREATING LONG COVID**
ZUSAMMENSETZUNGEN ZUR BEHANDLUNG VON LONG COVID
COMPOSITIONS POUR LE TRAITEMENT DE LA COVID LONGUE

(30) Priority: 05.06.2020 US 202063035681 P; 14.12.2020 US 202063125195 P
(43) Date of publication of application: 07.12.2022
(73) Proprietor: AIM ImmunoTech Inc., Ocala, FL 34473 (US)
(72) Inventor: EQUELS, Thomas K., Ocala, FL 34473 (US); STRAYER, David R., Ocala, FL 34473 (US); YOUNG, Diane L., Ocala, FL 34473 (US)
(74) Representative: V.O.
(86) International application number: PCT/US2021/036235
(87) International publication number: WO 2021/248134

(56) References cited:
- US-A1- 2007 141 080
- US-A1- 2011 196 020
- US-A1- 2016 333 347
- WILLIAM M MITCHELL: "Efficacy of rintatolimod in the treatment of chronic fatigue syndrome/myalgic encephalomyelitis (CFS/ME)", EXPERT REVIEW OF CLINICAL PHARMACOLOGY 20141101 EXPERT REVIEWS LTD. GBR, vol. 9, no. 6, 25 May 2016 (2016-05-25), UK, pages 755 - 770, XP055556867, ISSN: 1751-2433, DOI: 10.1586/17512433.2016.1172960
- "Mismatched double-stranded RNA: polyI:polyC12U", DRUGS IN R&D, ADIS INTERNATIONAL LTD , NZL, vol. 5, no. 5, 1 January 2004 (2004-01-01), pages 297 - 304, XP009129540, ISSN: 1174-5886
- XU JIABAO, ZHAO SHIZHE, TENG TIESHAN, ABDALLA ABUALGASIM ELGAILI, ZHU WAN, XIE LONGXIANG, WANG YUNLONG, GUO XIANGQIAN: "Systematic Comparison of Two Animal-to-Human Transmitted Human Coronaviruses: SARS-CoV-2 and SARS-CoV", VIRUSES, vol. 12, no. 2, pages 1 - 17, XP055853415, DOI: 10.3390/v12020244
- STRAYER DAVID R., YOUNG DIANE, MITCHELL WILLIAM M.: "Effect of disease duration in a randomized Phase III trial of rintatolimod, an immune modulator for Myalgic Encephalomyelitis/Chronic Fatigue Syndrome", PLOS ONE, vol. 15, no. 10, 29 October 2020 (2020-10-29), pages e0240403, XP055880667, DOI: 10.1371/journal.pone.0240403

## Description

### BACKGROUND

At the end of 2019, a novel coronavirus was identified as the cause of a cluster of pneumonia cases in Wuhan, China. It rapidly spread and became a global pandemic. The disease was named novel coronavirus disease 2019 (COVID-19) and the causative agent was discovered to be a coronavirus later named SARS-CoV-2. SARS-CoV-2 is a member of the β coronavirus family. It is the seventh known coronavirus to infect humans; four of these coronaviruses (229E, NL63, OC43, and HKU1) cause slight symptoms of the common cold. The other three β coronaviruses, SARS-CoV, MERS-CoV, and SARS-CoV-2 can cause severe symptoms and even death, with fatality rates of 10%, 37%, and 5%, respectively. Over 600,000 people in the U.S. and over 3.5 million people worldwide have died from SARS-CoV-2 infection.

Over 80% of hospitalized patients reported at least one lingering symptom of COVID even after the acute phase of the infection has passed. Further, in many patients, persistent symptoms of COVID remain after 6 weeks, 12 weeks, or over 6 months. This disorder/disease has been called long-haulers and other names. At present, the term "Long Covid" has been commonly used to describe the disorder/disease. (See, e.g., World Health Organization . 2020. Covid-19 Virtual Press Conference 21 August 2020. p.22. Available from:https://www. who. int/ docs/ default-source/coronaviruse/transcripts/covid-19-virtual-press-conference---21-august.pdf?sfvrsn=ada7ae85_0; see, also, Mahase E. Covid-19: what Do We Know about "Long Covid"? BMJ. 2020; 370 https:// www. bmj. com/ content/ 370/ bmj.m2815 Accessed 31 July 2020.).

This disclosure refers to compositions and methods for treating Long Covid or at least one symptom of Long Covid.

### SUMMARY

One embodiment is directed to a compositioncomprising tdsRNA for use in treating Long COVID in a subject previously infected with a virus wherein the virus is SARS-CoV-2, the treatment comprising administering to the subject a therapeutically effective amount of a composition comprising tdsRNA, as defined in the claim 1.

To be considered to have post-viral long-term symptoms (e.g., Long Covid), a subject should satisfy two conditions. The first condition is that the subject exhibits at least one symptom selected from the group consisting of: fatigue including severe fatigue, inability to exercise or be active because of fatigue, and low exercise tolerance because of fatigue. The second condition is that the subject should exhibit at least 4 symptoms selected from the group consisting of: shortness of breath or difficulty breathing, persistent chest pain or pressure, cough, heart palpitations, diarrhea, partial or complete loss of sense of smell, tachycardia, hair loss, blurry vision, neuropathy in feet and hands, partial or complete loss of sense of taste, nausea or vomiting, clogged ears, dry eyes, tremors or shakiness, floaters or flashes of light in vision, rash, tinnitus or humming in ears, changed sense of taste, dry or peeling skin, phantom smells, costochondritis, low blood oxygen, COVID toes, thrush, dyspnea, phlegm in back of throat, constant thirst, muscle twitching, heat intolerance, abnormally low temperature, cold burning feeling in lungs, goiter or lump in throat, dry scalp or dandruff, anemia, elevated thyroid, sicca syndrome, red eyes, dysgeusia, sputum production, lack of appetite, vertigo, muscle pain, cognitive problems, brain fog, problems with concentration, problems with thinking, chills, sweats, sleep problems, muscle or body aches, difficulty concentrating or focusing, headache, difficulty sleeping, anxiety, memory problems, dizziness, joint pain, sore throat, night sweats, fever or chills, congested or runny nose, sadness, reflux or heartburn, changing symptoms, abdominal pain, lower back pain, shortness of breath or exhaustion from bending over, weight gain, calf cramps, sleeping more than normal, upper back pain, nerve sensations, sharp or sudden chest pain, confusion, feeling irritable, weight loss, post nasal drip, dry throat, high blood pressure, swollen hands or feet, mouth sores or sore tongue, neck muscle pain, hot blood rush, bone aches in extremities, feeling of burning skin, extreme pressure at base of head or occipital nerve, swollen lymph nodes, brain pressure, kidney pain, spikes in blood pressure, hand or wrist pain, bulging veins, mid-back pain at base of ribs, burning sensations, painful scalp, jaw pain, arrhythmia, cracked or dry lips, foot pain, eye stye or infection, low blood pressure, kidney issues or protein in urine, urinary tract infection, hormone imbalances, drastic personality change, gastroesophageal reflux disease with excessive salivation, herpes infection, EBV infection, trigeminal neuralgia, bilateral neck throbbing around lymph nodes, syncope, sadness, chest pain, rhinitis, and myalgia.

Post-viral long-term symptoms (e.g., Long Covid) can be exhibited and defined as follows: (a) the subject has COVID-19, a previous diagnosed presence of SARS-CoV-2, or a positive serum antibody test for SARS-CoV-2; (b) the subject meets the Diagnosis of Chronic Fatigue Syndrome (CFS) as defined by the 1988 or the 1994 CDC case definition for CFS for at least 3 months but CFS does not precede the (a); and (c) the subject has concurrent occurrence of at least one or more of Long Covid symptoms which persisted or recurred during 3 or more consecutive months of illness but the Long Covid symptoms do not precede (a), wherein the Long Covid symptoms is at least one selected from the group consisting of: fatigue; post-exertional malaise; headache; sleep disturbance; memory problems; problems with concentration; brain fog; fever; chills; cough; shortness of breath; difficulty breathing; loss of taste; loss of smell; and chest pain.

In accordance with the invention, the post-viral fatigue is Long COVID (previously also called Post COVID-19 Chronic Fatigue (PCCF)) and wherein the post-viral long-term symptoms are symptoms of Long COVID.

The subject may be seropositive for anti-virus antibody or seronegative for anti-virus antibody. The subject may not be infected with the virus during the administering step. The subject may be infected with the virus during the administering step. The subject may not be hospitalized for the virus infection during the administering step. The subject may not have (i.e., be free of) post-viral fatigue symptoms before being infected with the virus. The subject may not have (may be free of) post-viral fatigue symptoms for at least 1 year, 2 years, 3 years, 4 years, or 5 years before being infected with the virus.

The subject may develop the symptoms of post-viral fatigue during or after being infected with the virus.

The virus may be SARS-CoV-2. For example, SARS-CoV-2 caused a subject to be sick and Long Covid symptoms continue in the subject even after the SARS-CoV-2b infection is treated or there is an undetectable level of SARS-CoV-2 in the subject.

The virus may be at least one selected from the group consisting of: influenza virus; adenovirus; herpes virus; rhinovirus; respiratory syncytial virus; coronavirus; Ebola Virus; West Niles Virus; Zika Virus; H5 influenza; H7 influenza; H5N1 influenza; West Niles Virus; Zika Virus; Human coronavirus 229E (HCoV-229E); Human coronavirus OC43 (HCoV-OC43); Severe acute respiratory syndrome-related coronavirus (SARS-CoV); Human coronavirus NL63 (HCoV-NL63, New Haven coronavirus); Human coronavirus HKU1; Middle East respiratory syndrome-related coronavirus (MERS-CoV, novel coronavirus 2012, HCoV-EMC); SARS-CoV-2; Influenza A; Influenza B; H1N1 influenza; H3N2 influenza; H7N9 influenza; H5N6 influenza; H10N8 influenza; H9N2 influenza; H6N1 influenza; a variant thereof; a strain thereof; a mutant thereof; a type thereof; a subtype thereof; a lineage thereof; a clade thereof; a subclade thereof; and a combination thereof.

The post-viral long-term symptoms may have lasted from the time of infection to the administering step, for a time selected from the group consisting of: 1 month, 2 months, 3 months, 4 months, 5 months, 6 months, 1 year, 2 years, and more than 2 years.

The administrating step may be performed after the primary COVID-19 symptoms of acute illness have been resolved.

The administering is performed after the subject was infected for a time selected from the group consisting of 30 days, 50 days, 2 months, 3 months, 4 months, 5 months, 6 months, 1 year, 2 years, and more than 2 years.

The COVID-19 symptoms of acute illness may at least one be selected from the group consisting of: fever, chills, cough, shortness of breath, difficulty breathing, fatigue, muscle aches, body aches, headache, loss of taste, loss of smell, sore throat, congestion, runny nose, nausea, vomiting, diarrhea, and a combination thereof.

The composition may be administered at a dosage of about 25-700 milligrams of tdsRNA.

The composition may be administered at a rate selected from the group consisting of: one dose per day, one dose every 2 days, one dose every 3 days, one dose every 4 days, one dose every 5 days, once a week, twice a week, thrice (3 times) a week, once every two weeks, once every 3 weeks, once every 4 weeks, and once a month.

The subject may be a mammal, such as, for example, a human.

The administering may be systemic administration, optionally by intravenous administration.

The administering may be at least one selected from the group consisting of: intravenous administration; intradermal administration; subcutaneous administration; intramuscular administration; intranasal administration; intraperitoneal administration; intracranial administration; intravesical administration; oral administration (through the mouth, by breathing through the mouth); topical administration; inhalation administration; aerosol administration; intra-airway administration; tracheal administration; bronchial administration; instillation; bronchoscopic instillation; intratracheal administration; mucosal administration; dry powder administration; spray administration; contact administration; swab administration; intratracheal deposition administration; intrabronchial deposition administration; bronchoscopic deposition administration; lung administration; nasal passage administration; respirable solid administration; respirable liquid administration; and dry powder inhalants administration.

The administering may be by nasal administration (pulmonary airway administration); oral and nasal administration; or nasal and intravenous administration.

The administering may be administering to at least one tissue selected from the group consisting of: an airway tissue; nose tissue; oral tissue; alveoli tissue; pharynx tissue; trachea tissue; bronchi tissue; carina tissue; bronchi tissue; bronchioles tissue; lung tissue; lobe of lung tissue; alveoli tissue; nasal passage tissue; nasal epithelium tissue; larynx tissue; bronchi tissue; inhalation tissue; and a combination thereof.

The administering may be administering to at least one cell selected from the group consisting of: an epithelium cell; an airway epithelium cell; a ciliated cell; a goblet cell; a non-ciliated cell; a basal cell; a lung cell; a nasal cell; a tracheal cell; a bronchial cell; a bronchiolar epithelial cell; an alveolar epithelial cell; a sinus cell; and a combination thereof.

The administering may be by at least one delivery system (device) selected from the group consisting of: a nebulizer; a sprayer; a nasal pump; a squeeze bottle; a nasal spray; a syringe sprayer or plunger sprayer (a syringe providing pressure to an attached sprayer or nozzle); a nasal aerosol device; a controlled particle dispersion device; a nasal aerosol device; a nasal nebulization device; a pressure-driven jet nebulizer; ultrasonic nebulizer; a breath-powered nasal delivery device; a atomized nasal medication device; an inhaler; a powder dispenser; a dry powder generator; an aerosolizer; an intrapulmonary aerosolizer; a sub-miniature aerosolizer; a propellant based metered-dose inhalers; a dry powder inhalation devices; an instillation device; an intranasal instillation device; an intravesical instillation device; a swab; a pipette; a nasal irrigation device; a nasal rinse; an aerosol device; a metered aerosol device; a pressurized dosage device; a powdered aerosol; a spray aerosol; a spray device; a metered spray device; a suspension spray device; and a combination thereof.

The tdsRNA is rIₙ•r(C₁₂U)ₙ, or a combination of rIₙ•r(C₁₂U)ₙ and rugged dsRNA, wherein the rugged dsRNA is an isolated double stranded ribonucleic acid (dsRNA) enzymatically active under thermal stress comprising: each strand with a molecular weight of 250 kDa to 500 kDa, 400-800 basepairs, 30 to 60 helical turns of duplex RNA, a single strand comprised of poly(ribocytosinic4-29 uracilic acid) and an opposite strand comprised of poly(riboinosinic acid),
wherein the two strands do not base pair the position of the uracil base,
wherein the two strands base pair the position of the cytosine base, and
wherein said strands are partially hybridized,
wherein n is a number with a value from 40 to 50 000,
wherein rIₙ represents a poly ribo-inosine strand of n bases in length, and
wherein r(C₁₂U)ₙ represents a ssRNA strand of random sequence of C and U bases being n bases in length and a ratio of C bases to U bases in the random sequence ssRNA is about 12.

The tdsRNA, including Rugged dsRNA which is a type of tdsRNA, may have the following characteristics: The tdsRNA preferably contains a minimum of 90 weight percent of dsRNA which is larger than a size selected from the group consisting of: 40 basepairs; 50 basepairs; 60 basepairs; 70 basepairs; and 80 basepairs. The tdsRNA may contain a minimum of 90 weight percent of dsRNA which is smaller than a size selected from the group consisting of: 10,000 basepairs; 9000 basepairs; 8000 basepairs; and 7000 basepairs. "n" in formula 1 to formula 5 may be from 40 to 40,000. The tdsRNA may have 4 to 4000 helical turns of duplexed RNA strands. The tdsRNA may have a molecular weight from 25 kDa to 2500 kDa. The tdsRNA is a linear structure without a branching RNA structure. The tdsRNA is complexed with a stabilizing polymer. The stabilizing polymer is at least one selected from the group consisting of polylysine; polylysine plus carboxymethylcellulose; polyarginine; polyarginine plus carboxymethylcellulose; carboxymethylcellulose; and a combination thereof. In one embodiment, the tdsRNA is r(Iₙ)•r(C₁₂U)ₙ, wherein n is from 40 to 50,000; 50 to 10,000; 60 to 9000; 70 to 8000; 80 to 7000; or 380 to 450.

The composition or the methods comprising the composition may have the characteristic that at least 30 weight percent of total dsRNA in the composition is a linear structure; at least 40 weight percent of total dsRNA in the composition is a linear structure; at least 50 weight percent of total dsRNA in the composition is a linear structure; at least 60 weight percent of total dsRNA in the composition is a linear structure; at least 70 weight percent of total dsRNA in the composition is a linear structure; at least 80 weight percent of total dsRNA in the composition is a linear structure; or at least 90 weight percent of total dsRNA in the composition is a linear structure. A linear structure is a linear nucleic acid molecule without, for example, branching or Y structure or loops.

The tdsRNA may be rIₙ•ribo(C₁₂U)ₙ and Rugged dsRNA.

The Rugged dsRNA may have the following characteristics: The Rugged dsRNA has a single strand comprised of r(C₄₋₂₉U); and an opposite strand comprised of r(I); wherein the two strands do not base pair the position of the uracil base, and wherein said strands are partially hybridized. The Rugged dsRNA has at least one selected from the group consisting of: a molecular weight of 250 kDa to 500 kDa; each strand of the Rugged dsRNA is from 400 to 800 basepairs in length; and the tdsRNA has 30 to 60 helical turns of duplexed RNA. The Rugged dsRNA may be a double-stranded RNA with two strands, wherein each strand of the Rugged dsRNA is of a length from 380 bases to 450 bases, wherein a single strand of the Rugged dsRNA comprises poly(ribocytosinic4-29uracilic acid); wherein an opposite strand of the Rugged dsRNA comprised of poly(riboinosinic acid), and wherein the two strands do not base pair the position of the uracil base, wherein the two strands base pair the position of the cytosine base, and wherein said strands are partially hybridized.

The methods can further comprise a step of administering an interferon to the subject, wherein the step of administering the tdsRNA and the step of administering the interferon are performed in any order.

Also disclosed herein is a composition for treating post-viral fatigue or relieving a symptom thereof in a subject comprising any tdsRNA described anywhere in this disclosure.

The post-viral fatigue is Long COVID. The composition may further comprise at least one interferon selected from the group consisting of: recombinant or natural interferon, Alferon, alpha-interferon species, recombinant or natural interferon alpha, recombinant or natural interferon alpha 2a, recombinant or natural interferon beta, recombinant or natural interferon beta 1b, and recombinant, and natural interferon gamma. The composition may be used in any of the methods of this disclosure. As an example, the composition may comprise rIₙ•ribo(C₁₁₋₁₄U)ₙ and Rugged dsRNA; or it may comprise rIₙ•ribo(C₁₁₋₁₄U)ₙ. The post-viral fatigue may be from any virus discussed in this disclosure such as, for example, the viruses discussed above.

Examples of various embodiments of this disclosure are provided. Each part or subpart of an embodiment should also be considered an embodiment. Each of the embodiments of this disclosure may be combined with one or more other embodiments of the disclosure and the combination is also an embodiment of this disclosure. In any part of this disclosure and in any embodiment, the terms "comprise," "comprising," "consist," "consisting," "consist essentially of," or "consisting essentially of" can be replaced with each other and each of these replacements is also an embodiment of the disclosure. That is, the interchange of one of the above-listed terms of this paragraph with another, even if they have different meanings, is also envisioned. In this disclosure, the term "disease" may be substituted with the term "disorder," and the term "disorder" may be substituted with the term "disease."

Various aspect of the above disclosure is discussed in more detail below.

### BRIEF DESCRIPTION OF THE FIGURES

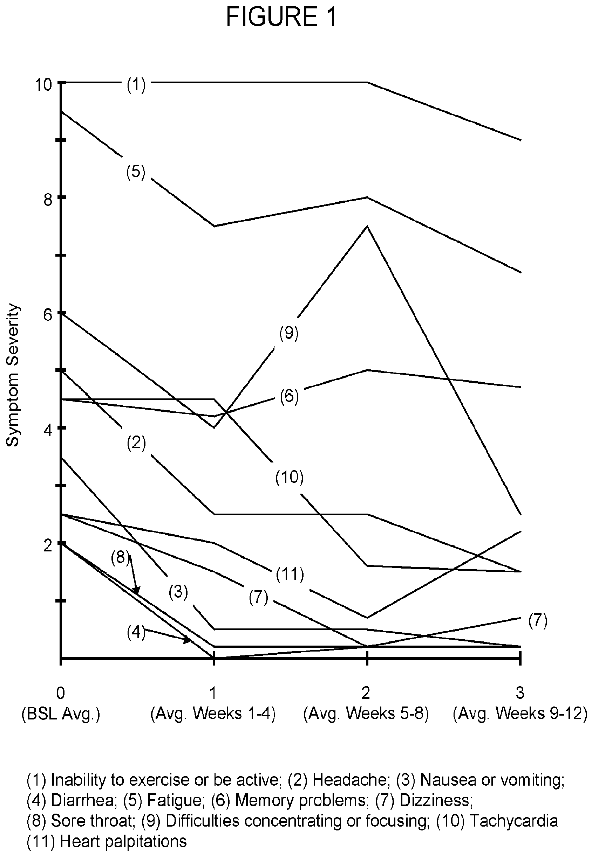

- Figure 1: depicts a graphic summary of patient symptoms over time showing significant improvement.

### DETAILED DESCRIPTION

### 1. OVERVIEW

COVID-19 is a recently emergent disease caused by infection by a newly discovered virus SARS-CoV-2. COVID-19 can manifest a wide severity spectrum from asymptomatic to fatal forms. In addition to its devastating death toll, COVID-19 also has at least one long-term detrimental effect - Long Covid. Long Covid has been referred to previously by many names such as Post COVID-19 Fatigue Syndrome ("PCCF"), Long Haul Covid, long-hauler syndrome.

This disclosure refers to compositions and methods for treating Long COVID which are a set of symptoms that persist long after a SARS-CoV-2 infection. Long COVID refers to a post COVID-19 viral fatigue syndrome. These Long COVID symptoms develop during or after COVID-19 (SARS-CoV-2 infection) and persist even after some or all of the primary symptoms of COVID-19 have subsided or reduced in severity. The primary symptoms of COVID-19 include, for example, fever, chills, cough, shortness of breath, difficulty breathing, fatigue, muscle aches, body aches, headache, loss of taste, loss of smell, sore throat, congestion, runny nose, nausea, vomiting, diarrhea. That is, these Long COVID symptoms persist even after a COVID-19 patient is no longer at risk for serious adverse effects and hospitalization is no longer required.

For example, Long COVID can persist even after a SARS-CoV-2 infection, even after the level of SARS-CoV-2 is declining or is very low, and even after a subject no longer has detectable levels of SARS-CoV-2 virus. We note a subset of COVID-19 patients may suffer from Long Covid for the rest of their lives and would require continual therapy.

For example, a subject may be released from the hospital and may have no detectable levels of SARS-CoV-2 by an antibody test or by a PCR-based (e.g., including RT-PCR) test and still have symptoms of Long COVID. Long COVID symptoms can include, for example, muscle pain, cognitive problems, fatigue, severe fatigue, brain fog, problems with concentration, problems with thinking, chills, sweats, and sleep problems, and less exercise treadmill tolerance (ETT) relative to a subject without Long COVID. Some of these symptoms are less severe than COVID-19 symptoms and do not require hospitalization but are nevertheless debilitating enough that a subject suffering from Long COVID may not be able to work or work as productively as before the onset of Long COVID. Subjects with Long COVID may be unable to live an active lifestyle, unable to work, and even unable to perform everyday tasks necessary for survival.

We have found that tdsRNA (discussed in the section below), by itself or in the form of a composition, is effective in reducing one or more symptoms of Long COVID in a subject. In a preferred embodiment, tdsRNA is effective for a 25% increase in exercise treadmill tolerance (ETT). Without wishing to be bound by theory, we postulate that tdsRNA reduces the one or more symptoms by modulating the immune system of a subject with Long COVID.

### 2. DEFINITIONS

"COVID-19" is a disease caused by an infection of the "SARS-CoV-2" virus. These names were standardized by the World Health Organization (WHO) and the International Committee on Taxonomy of Viruses (ICTV). Because of novelty and the rapidity of SARS-CoV-2 spread worldwide, a number of names have been used for SARS-CoV-2 including: COVID-19, COVID-19 virus, 2019-nCoV, Novel coronavirus pneumonia, Wuhan coronavirus, and severe acute respiratory syndrome coronavirus 2 (SARS-CoV-2). A subject with the disease of COVID-19 would have an infection of SARS-CoV-2 virus and, conversely, a subject with an infection of SARS-CoV-2 has the disease COVID-19. However, a subject with a SARS-CoV-2 infection may be asymptomatic or may have not yet developed all the symptoms.

This disclosure relates to, inter alia, tdsRNA. tdsRNA can also be called "therapeutic dsRNA," or "therapeutic double-stranded RNA" and these terms have the same meaning.

"r" and "ribo" have the same meaning and refer to ribonucleic acid or the nucleotides or nucleosides that are the building block of ribonucleic acid.

RNA consists of a chain of linked units called nucleotides. This disclosure relates mostly to RNA and, therefore, unless otherwise specified, the nucleotides and bases expressed refers to the ribo form of the nucleotide or base (i.e., ribonucleotide with one or more phosphate groups). Therefore "A" refers to rA or adenine, "U" refers to rU or uracil, "C" refers to rC or cytosine, "G" refers to rG or guanine, "I" refers to rI or inosine, "rN" refers to rA, rU, rC, rG or rI. Each of these (i.e., A, U, C, G, I) may have one or more phosphate groups as discussed above depending on whether they are part of a chain (i.e., RNA) or free (nucleoside, nucleotide, etc.).

"n" is a positive number and refers to the length of a ssRNA or dsRNA or to the average length of a population of ssRNA or dsRNA. "n" can be a positive integer when referring to one nucleic acid molecule or it can be any positive number when it is an average length of a population of nucleic acid molecules.

An RNA may have a ratio of nucleotides or bases. For example, r(C₁₂U)ₙ denotes a single RNA strand that has, on average 12 C bases or nucleotides for every U base or nucleotide. As another example, r(C₁₁₋₁₄U)ₙ denotes a single RNA strand that has, on average 12 C bases or nucleotides for every U base or nucleotide.

Formulas: As an example, the formula "rIₙ•r(C₁₂U)ₙ" can be expressed as riboIₙ•ribo(C₁₂U)ₙ, rIₙ•ribo(C₁₂U)ₙ, or riboIₙ•r(C₁₂U)ₙ, refers to a double-stranded RNA with two strands. One strand (rIₙ) is poly ribo-inosine of n bases in length. The other strand is ssRNA of random sequence of C and U bases, the random sequence ssRNA is n bases in length, and a ratio of C bases to U bases in the random sequence ssRNA is about 12 (i.e., mean 12 C to 1 U). The terms "r" and "ribo" have the same meaning in the formulas of the disclosure. Thus, rI, riboI, r(I) and ribo(I) refer to the same chemical which is the ribose form of inosine. Similarly, rC, riboC, r(C) and ribo(C) all refer to cytidine in the ribose form which is a building block of RNA. rU, riboU, r(U) and ribo(U) all refer to Uracil in the ribose form which is a building block of RNA.

The "•" symbol indicates that one strand of the dsRNA is hybridized (hydrogen-bonded) to the second strand of the same dsRNA. Therefore, rIₙ•r(C₁₂U)ₙ is double-stranded RNA comprising two ssRNA. One ssRNA is poly(I) and the other ssRNA is poly(C₁₂U). It should be noted that while we referred to the two strands being hybridized, not 100% of the bases form base pairing as there are some bases that are mismatched. Also, because rU does not form base pairing with rI as well as rC form base paring with rI, rU provides a focus of hydrodynamic instability in rIₙ•r(C₁₂U)ₙ at the locations of the U bases.

As another example, the formula "rIₙ•r(C₁₁₋₁₄U)" refers to the same dsRNA except that a ratio of C bases to U bases one strand is 11 to 14. That is, the ratio can be 11, 12, 13 or 14 or any value between 11 and 14. For example, when half of the strands are r(C₁₂U)ₙ and half of the strands are r(C₁₃U)ₙ, the formula would be r(C_{12.5}U)ₙ.

The dsRNA (tdsRNA) and ssRNA of this disclosure are homopolymers (e.g., a single-stranded RNA where every base is the same) or heteropolymers (e.g., a single-stranded RNA where the bases can be different) of limited base composition. The tdsRNAs are not mRNA and are distinct from mRNA in structure. For example, the ssRNA and dsRNA are preferably missing one or all of the following: (1) 5' cap addition, (2) polyadenylation, (3) start codon, (4) stop codon, heterogeneous protein-coding sequences, and (5) spice signals.

As used herein, the term "substantially free" is used operationally in the context of analytical testing of the material. Preferably, purified material is substantially free of one or more impurities. In a preferred embodiment, the tdsRNA of this disclosure is substantially free (e.g., more than 0% to less than 0.1%) or completely free (0%) of dI/dI dsRNA or dCdU/dCdU dsRNA. In other words, the tdsRNA is substantially free or completely free (0%) of homodimers of polymer 1 or homodimers of polymer 2. Substantially free in this context would be considered to be more than 0% but less than 1%, less than 0.5%, less than 0.2%, less than 0.1%, or less than 0.01% of a contaminant such as (1) dI/dI (polymer 1/polymer 1) dsRNA, dCdU/dCdU (polymer 2/polymer 2) dsRNA.

The terms "intranasal" or "intranasally," "instillation," "instillation of a liquid," "instillation using a sprayer" as used herein, refers to a route of delivery of an active compound to a patient by inhalation to the nasal mucosa, the airway, the lung or a combination thereof.

As used herein, the term "nebulizer" refers to a drug delivery device used to administer medication such as tdsRNA in the form of a mist inhaled into the central nervous system through the nose. Nebulizers may use oxygen, compressed air, ultrasonic power, mechanical means, etc., to break up medical solutions and suspensions into small aerosol droplets that can be directly inhaled from the device.

As used herein, the term "aerosol" refers to a mixture of gas and liquid particles, and the best example of a naturally occurring aerosol is mist, formed when small vaporized water particles mixed with hot ambient air are cooled down and condense into a fine cloud of visible airborne water droplets. In one embodiment of the present disclosure, an aerosol may be produced through an aerosol spray or a sprayer. As used herein, the term "aerosol spray" or "a sprayer" refers to a type of dispensing system which creates an aerosol mist of liquid particles. This is used with a can or bottle that contains a liquid under pressure. When the container's valve is opened, the liquid is forced out of a small hole and emerges as an aerosol or mist. As the gas expands to drive out the payload, only some propellant evaporates inside the can to maintain an even pressure. Outside the can, the droplets of propellant evaporate rapidly, leaving the payload suspended as very fine particles or droplets. An atomizer is a similar device that is pressurized by a hand-operated pump rather than by stored gas.

A particle, droplet, or an aerosol, and the like in this description may be a liquid suspension particle or a dry particle. A dry particle may be dry as it is produced, as it is administered, and/or as it exits an apparatus. It can be a dry particle even if the particle, droplet, or an aerosol starts out as a liquid because the liquid may be a fast evaporating liquid. Therefore, by the time the particle, droplet, or an aerosol contacts the subject, it will be dry. Also, it is possible a liquid particle, droplet, or an aerosol will dry after contact with a subject, for example, by rapid evaporation of the liquid component.

The terms "intranasal" or "intranasally," "instillation," "instillation of a liquid," "instillation using a sprayer" as used herein, refers to a route of delivery of an active compound to a patient by inhalation to the nasal mucosa, the airway, the lung or a combination thereof. Inhalation may be by breathing through the mouth or through a stoma as a result of a tracheostomy.

Active ingredients or active agents are used interchangeably and include any active ingredient or active agent described in this disclosure including, at least, tdsRNA. Other active agents include, at least, interferons such as Alferon.

### 3. tdsRNA

The double-stranded RNAs described in this disclosure are therapeutic double-stranded RNA, abbreviated as "tdsRNA." tdsRNA includes, at least, Rintatolimod which is a tdsRNA of the formula rIₙ•r(C₁₂U)ₙ). tdsRNA may be stored or administered in a pharmaceutically acceptable solution such as Phosphate Buffered Saline (PBS).

The tdsRNA may be a tdsRNA produced by any of the methods of this disclosure - referred to herein as the "tdsRNA Product" or "tdsRNA" - the two terms have the same meaning. tdsRNA is represented by rIₙ•r(C₁₂U)ₙ, or a combination of rIₙ•r(C₁₂U)ₙ and rugged dsRNA. Where there is no subscript next to a base, the default value is "1." For example, in the formula rIₙ•r(C₁₂U)ₙ, there is no subscript following "U," it is understood that rIₙ•r(C₁₂U)ₙ is the same as rIₙ•r(C₁₂U₁)ₙ and the formula is meant to convey that for the strand denoted as r(C₁₂U₁)ₙ, there are 12 rC base for every rU base. The length of the tdsRNA strand is denoted as a lowercase "n" (e.g., rIₙ•r(C₁₂U)ₙ). The subscript n is also the length of each individual single-stranded nucleic acid. Since tdsRNA is double-stranded, n is also the length of the double-stranded nucleic acid - i.e., the length of the tdsRNA. For example, rIₙ•r(C₁₂U)ₙ indicates, inter alia, a double-stranded RNA with each strand with a length of n.

In an aspect, at least 70 %, at least 80 %, or at least 90 % of the tdsRNA may have a molecular weight of between 400,000 Daltons to 2,500,000 Daltons. Where the term percent ("%") is used, the percent may be weight percent or molar percent.

In another aspect, the tdsRNA comprises a first ssRNA and a second ssRNA and each of these first ssRNA or second ssRNA may contain one or more strand breaks.

In another aspect, the tdsRNA has the property that greater than 90%, greater than 95%, greater than 98%, greater than 99%, or 100% of the bases of the RNA are in a double-stranded configuration.

In any aspect, the tdsRNA may be in a therapeutic composition comprising, for example, a tdsRNA, and a pharmaceutically acceptable excipient (carrier).

One embodiment of tdsRNA is directed to rintatolimod, which is a tdsRNA of the formula rIₙ•r(C₁₂U)ₙ and which is also denoted by the trademark AMPLIGEN^{®}.

tdsRNA (e.g., Rintatolimod) has undergone extensive clinical and preclinical testing. It has been well-tolerated in clinical trials enrolling over 1,200 patients with over 100,000 doses administered and there have been no drug-related deaths. Two placebo-controlled, randomized studies show no increase in serious adverse events compared to placebo. Favorable safety profiles have been seen for intraperitoneal, intravenous, and intranasal routes of administration of tdsRNA.

### 3.1 LENGTH OF tdsRNA

The length of the tdsRNA, may be represented by bases for one strand of the tdsRNA or in basepairs for both strands for the tdsRNA. It is understood that in some embodiments that not all of the bases (e.g., U and I) are in basepaired configuration. For example, rU bases do not pair as well as rC bases to inosine.

The length of the tdsRNA may be measured by (1) bases or basepairs, (2) molecular weight which is the weight of the double-stranded tdsRNA (e.g., Daltons) or (3) turns of the double-stranded RNA. These measurements can be easily interconverted. For example, it is generally accepted that there are about 629 Daltons per base pair.

"n" represents length in units of basepair or basepairs (abbreviated as bp regardless of whether it is singular or plural) for double-stranded nucleic acid. "n" can also represent bases for single-stranded RNA. Because "bp" represents singular or plural, it is the same as "bps" which is another representation of basepairs.

The tdsRNA can have the following values for its length "n" (in bases for single strand or basepairs for double strands): 40-40,000, 40-50,000, 40-500, 50-500, 100-500, 380-450, 400-430, 400-800 or a combination thereof. Expressed in molecular weight, the tdsRNA may have the following values: 30 kDa to 300 kDa, 250 kDa to 320 kDa, 270 kDa to 300 kDa or a combination thereof. Expressed in helical turns, the tdsRNA may have 4.7 to 46.7 helical turns of duplexed RNA, 30 to 38 helical turns of duplexed RNA, 32 to 36 helical turns of duplexed RNA or a combination thereof.

The length may be an average basepair, average molecular weight, or an average helical turns of duplexed RNA and can take on integer or fractional values.

### 3.2 RUGGED DSRNA (A FORM OF tdsRNA)

Rugged dsRNA is an isolated double stranded ribonucleic acid (dsRNA) enzymatically active under thermal stress comprising: each strand with a molecular weight of 250 kDa to 500 kDa, 400 800 basepairs, or 30 to 60 helical turns of duplex RNA; a single strand comprised of poly(ribocytosinic4-29 uracilic acid) and an opposite strand comprised of poly(riboinosinic acid), wherein the two strands do not base pair the position of the uracil base, wherein the two strands base pair the position of the cytosine base, and wherein said strands are partially hybridized.

A rugged dsRNA can be an isolated double-stranded ribonucleic acid (dsRNA) which is resistant to denaturation under conditions that are able to separate hybridized poly(riboinosinic acid) and poly(ribocytosinic acid) strands, wherein only a single strand of said isolated dsRNA comprises one or more uracil or guanine bases that are not basepaired to an opposite strand and wherein said single strand is comprised of poly(ribocytosinic₃₀₋₃₅uracilic acid). Further, the single strand may be partially hybridized to an opposite strand comprised of poly(riboinosinic acid). Rugged dsRNA may be an isolated double-stranded ribonucleic acid (dsRNA) which is resistant to denaturation under conditions that are able to separate hybridized poly(riboinosinic acid) and poly(ribocytosinic acid) strands.

In another aspect, Rugged dsRNA, has at least one of the following: r(Iₙ)•r(C₄₋₂₉U)ₙ, r(Iₙ)•r(C₁₂U)ₙ, 1(Iₙ)•r(C₁₁₋₁₄U)ₙ, r(Iₙ)•r(C₃₀U)ₙ, or r(Iₙ)•r(C₃₀₋₃₅U)ₙ. In another aspect, Rugged dsRNA may have a size of 400 bps to 430 bps, 250 kDa to 320 kDa molecular weight, 270 kDa to 300 kDa molecular weight, 30 to 38 helical turns of duplexed RNA, 32 to 36 helical turns of duplexed RNA, and a combination thereof.

In another aspect, Rugged dsRNA is produced by isolating the 5 minute HPLC peak of a tdsRNA preparation.

### 3.3 RUGGED DSRNA PREPARATION

The starting material for making Rugged dsRNA may be dsRNA prepared in vitro using conditions of this disclosure. For example, the specifically configured dsRNA described in U.S. Patents 4,024,222, 4,130,641, and 5,258,369 are generally suitable as starting materials after selection for rugged dsRNA. tdsRNA (or preparations of tdsRNA) described in this disclosure is also useful as starting material.

After procuring starting material, Rugged dsRNA may be isolated by at least subjecting the partially hybridized strands of a population of dsRNA to conditions that denature most dsRNA (more than 10 wt% or mol%, more than 20 wt% or mol%, more than 30 wt% or mol%, more than 40 wt% or mol%, more than 50 wt% or mol%, more than 60 wt% or mol%, more than 70 wt% or mol%, more than 80 wt% or mol%, more than 90 wt% or mol%, more than 95 wt% or mol%, or more than 98 wt% or mol%) in the population, and then selection negatively or positively (or both) for dsRNA that remain partially hybridized. The denaturing conditions to unfold at least partially hybridized strands of dsRNA may comprise an appropriate choice of buffer salts, pH, solvent, temperature, or any combination thereof. Conditions may be empirically determined by observation of the unfolding or melting of the duplex strands of ribonucleic acid. The yield of rugged dsRNA may be improved by partial hydrolysis of longer strands of ribonucleic acid, then selection of (partially) hybridized stands of appropriate size and resistance to denaturation.

The purity of rugged dsRNA, which functions as tdsRNA, may thus be increased from less than 0.1-10 mol% (e.g., rugged dsRNA is present in at least 0.1 mol % or 0.1 wt percent but less than 10 mol% or 10 wt percent) relative to all RNA in the population after synthesis to a higher purity. A higher purity may be more than 20 wt% or mol%, more than 30 wt% or mol%, more than 40 wt% or mol%, more than 50 wt% or mol%, more than 60 wt% or mol%, more than 70 wt% or mol%, more than 80 wt% or mol%, more than 90 wt% or mol%, more than 98 wt% or mol%, or between 80 to 98 wt% or mol%. All wt% or mol% is relative to all RNA present in the same composition.

Another method of isolating Rugged dsRNA is to employ chromatography. Under analytical or preparative high-performance liquid chromatography, Rugged dsRNA can be isolated from a preparation (e.g., the starting material as described above) to produce poly(I):poly(C₁₂U)ₙ (e.g., poly(I):poly(C₁₁₋₁₄U)ₙ) as a substantially purified and pharmaceutically-active molecule with an HPLC peak of about 4.5 to 6.5 minutes, preferably between 4.5 and 6 minutes and most preferably 5 minutes.

Rugged dsRNA and the method of making rugged dsRNA are described in U.S. Patents 8,722,874 and 9,315,538.

### 3.4 STABILIZING POLYMERS

The tdsRNA may be complexed with a stabilizing polymer such as: polylysine, polylysine plus carboxymethylcellulose (lysine carboxy methyl cellulose), polyarginine, polyarginine plus carboxymethylcellulose, or a combination thereof. Some of these stabilizing polymers are described, for example, in US Patent 7,439,349.

### 3.5 MODIFIED BACKBONE

The tdsRNA may comprise one or more alterations in the backbone of the nucleic acid. For example, configured tdsRNA may be made by modifying the ribosyl backbone of poly(riboinosinic acid) r(Iₙ), for example, by including 2'-O-methylribosyl residues. Specifically configured dsRNA may also be modified at the molecule's ends to add a hinge(s) to prevent slippage of the base pairs, thereby conferring specific bioactivity in solvents or aqueous environments that exist in human biological fluids.

### 4. ADDITIONAL AGENTS

The tdsRNA of this disclosure may be in a compound or in a combination with a number of additional agents which are described herein.

### 4.1 Interferons

One optional component of the composition is interferon. As used herein, the term "interferon" (abbreviated "IFN") refers collectively to type 1 and type 2 interferons and including deletion, insertion, or substitution variants thereof, biologically active fragments thereof, and allelic forms thereof. As used herein, interferon refers collectively to type 1 and type 2 interferons. Type 1 interferon includes interferons alpha, beta, omega and their subtypes. Human interferon alpha has at least 14 identified subtypes while interferon beta has 3 identified subtypes.

The interferon may be at least one selected from the group consisting of: interferon, interferon mixture, Alferon, alpha-interferon species, recombinant or natural interferon alpha, recombinant or natural interferon alpha 2a, recombinant or natural interferon beta, recombinant or natural interferon beta 1b, recombinant, and natural interferon gamma.

The interferon is optionally an alpha-interferon. One preferred alpha interferon is ALFERON N Injection^{®} the only approved natural, multi-species, α-interferon available in the United States. Reverse-phase HPLC studies show that ALFERON N Injection^{®} is a consistent mixture of at least seven species of alpha interferon (α2, α4, α7, α8, α10, α16 and α17). This natural-source interferon has unique antiviral properties distinguishing it from genetically engineered interferons. The high purity of ALFERON N Injection^{®} and its advantage as a natural mixture of seven interferon species, some of which, like species 8b, have greater antiviral activities than other species, for example, species 2b, which is the only component of INTRON A^{®}. The superior antiviral activities, for example, in the treatment of chronic hepatitis C virus (HCV) and HIV infection, and tolerability of ALFERON N Injection^{®} compared to other available recombinant interferons, such as INTRON A^{®} and ROFERON A^{®}, have been reported.

The interferon may be interferon species purified as a mixture of at least seven species of alpha-interferon produced by human white blood cells. The seven species may be, for example, interferon alpha 2; interferon alpha 4; interferon alpha 7; interferon alpha 8; interferon alpha 10; interferon alpha 16; and interferon alpha 17.

The recommended dosage of the components will depend on the clinical status of the patient and the experience of the clinician in treating similar conditions. As a general guideline, a dosage of ALFERON N Injection^{®} utilized for systemic infections is 7 IU/kg 3 IU/pound) to 22 million IU/kg (10 million IU/pound) (e.g., subcutaneous injection) three times weekly. Experience to date is with dosages above 7 IU/ky of patient body weight (3 IU/lb. of patient body weight). Oral α-interferon (ALFERON LDO^{®}) has been administered as a liquid solution in the range of 500-10,000 IU/day and calculated on the basis of a 68.0 kg (150-pound) human this is from 7.3 to 146 IU/kg per day (3.3 to 66.0 IU/lb. per day). In one preferred embodiment, beneficial results are obtained at dosage levels of α-interferon in excess of 450 IU, which is greater than 7 IU/kg body weight (3 IU/pound body weight). A healthcare provider would be able, however, to determine the optimal dose and schedule of low dose oral α-interferon (or any interferon) to achieve a desired antiviral effect.

### 4.2 CARRIER OR VEHICLE

Suitable agents may include a suitable carrier or vehicle for intranasal mucosal delivery. As used herein, the term "carrier" refers to a pharmaceutically acceptable solid or liquid filler, diluent or encapsulating material. In one aspect, the carrier is a suitable carrier or vehicle for intranasal mucosal delivery including delivery to the air passages and to the lungs of a subject.

A water-containing liquid carrier can contain pharmaceutically acceptable additives such as acidifying agents, alkalizing agents, antimicrobial preservatives, antioxidants, buffering agents, chelating agents, complexing agents, solubilizing agents, humectants, solvents, suspending and/or viscosity-increasing agents, tonicity agents, wetting agents or other biocompatible materials. A tabulation of ingredients listed by the above categories may be found in the U.S. Pharmacopeia National Formulary, 1857-1859, (1990). One commonly used pharmaceutically acceptable carrier is phosphate-buffered saline (PBS).

### 4.3 ABSORPTION-PROMOTING AGENTS

Suitable agents may include any suitable absorption-promoting agents. The suitable absorption-promoting agents may be selected from small hydrophilic molecules, including but not limited to, dimethyl sulfoxide (DMSO), dimethylformamide, ethanol, propylene glycol, and the 2-pyrrolidones. Alternatively, long-chain amphipathic molecules, for example, deacyl methyl sulfoxide, azone (1-dodecylazacycloheptan-2-one or laurocapram), sodium lauryl sulfate, oleic acid, and the bile salts, may be employed to enhance mucosal penetration of the tdsRNA. In additional aspects, surfactants (e.g., polysorbates) are employed as adjunct compounds, processing agents, or formulation additives to enhance intranasal delivery of the tdsRNA.

### 4.4 DELIVERY-ENHANCING AGENTS

As used herein, the term "delivery-enhancing agents" refers to any agents which enhance the release or solubility (e.g., from a formulation delivery vehicle), diffusion rate, penetration capacity and timing, uptake, residence time, stability, effective half-life, peak or sustained concentration levels, clearance and other desired intranasal delivery characteristics (e.g., as measured at the site of delivery, or at a selected target site of activity such as the bloodstream) of tdsRNA or other biologically active compound(s).

Enhancement of intranasal delivery can thus occur by any of a variety of mechanisms, for example by increasing the diffusion, transport, persistence or stability of tdsRNA, increasing membrane fluidity, modulating the availability or action of calcium and other ions that regulate intracellular or paracellular permeation, solubilizing mucosal membrane components (e.g., lipids), changing non-protein and protein sulfhydryl levels in mucosal tissues, increasing water flux across the mucosal surface, modulating epithelial junctional physiology, reducing the viscosity of mucus overlying the mucosal epithelium, reducing mucociliary clearance rates, and other mechanisms.

### 4.5 MUCOLYTIC OR MUCUS CLEARING AGENTS

The present formulations may also comprise other suitable agents such as mucolytic and mucus-clearing agents. The term "mucolytic and mucus-clearing agents," as used herein, refers to any agents which may serve to degrade, thin or clear mucus from intranasal mucosal surfaces to facilitate absorption of intranasally administered biotherapeutic agents including tdsRNA. Based on their mechanisms of action, mucolytic and mucus clearing agents can often be classified into the following groups: proteases (e.g., pronase, papain) that cleave the protein core of mucin glycoproteins, sulfhydryl compounds that split mucoprotein disulfide linkages, and detergents (e.g., Triton X-100, Tween 20) that break noncovalent bonds within the mucus. Additional compounds in this context include, but are not limited to, bile salts and surfactants, for example, sodium deoxycholate, sodium taurodeoxycholate, sodium glycocholate, and lysophosphatidylcholine. Other effective agents that reduce mucus viscosity or adhesion to enhance intranasal delivery according to the methods of the disclosure include, e.g., short-chain fatty acids, and mucolytic agents that work by chelation, such as N-acylcollagen peptides, bile acids, and saponins (the latter function in part by chelating Ca²⁺ and/or Mg²⁺ which play an important role in maintaining mucus layer structure).

### 4.6 CILIOSTATIC AGENTS

The present formulations may comprise ciliostatic agents. As used herein, the term "ciliostatic agents" refers to any agents which are capable of moving a layer of mucus along the mucosa to removing inhaled particles and microorganisms. For use within the disclosure, the foregoing ciliostatic factors, either specific or indirect in their activity, are all candidates for successful employment as ciliostatic agents in appropriate amounts (depending on concentration, duration and mode of delivery) such that they yield a transient (i.e., reversible) reduction or cessation of mucociliary clearance at a mucosal site of administration to enhance the delivery of tdsRNA and other biologically active agents without unacceptable adverse side effects.

Within more detailed aspects, a specific ciliostatic factor may be employed in a combined formulation or coordinate administration protocol with tdsRNA, and/or other biologically active agents disclosed herein. Various bacterial ciliostatic factors isolated and characterized in the literature may be employed within the disclosure. Ciliostatic factors from the bacterium Pseudomonas aeruginosa include a phenazine derivative, a pyo compound (2-alkyl-4-hydroxyquinolines), and a rhamnolipid (also known as a hemolysin).

### 4.7 PENETRATION OR PERMEATION-PROMOTING AGENT

The intranasal mucosal therapeutic and prophylactic formulations of the present disclosure may be supplemented with any suitable penetration-promoting agent that facilitates absorption, diffusion, or penetration of tdsRNA across mucosal barriers. The penetration promoter may be any promoter that is pharmaceutically acceptable. Thus, another aspect relates to compositions comprising tdsRNA and one or more penetration-promoting agents selected from sodium salicylate and salicylic acid derivatives (acetyl salicylate, choline salicylate, salicylamide, etc.), amino acids and salts thereof (e.g., monoaminocarboxlic acids such as glycine, alanine, phenylalanine, proline, hydroxyproline, etc., hydroxyamino acids such as serine, acidic amino acids such as aspartic acid, glutamic acid, etc., and basic amino acids such as lysine, etc. --inclusive of their alkali metal or alkaline earth metal salts), and N-acetylamino acids (N-acetylalanine, N-acetylphenylalanine, N-acetylserine, N-acetylglycine, N-acetyllysine, N-acetylglutamic acid, N-acetylproline, N-acetylhydroxyproline, etc.) and their salts (alkali metal salts and alkaline earth metal salts).

Also provided as penetration-promoting agents within the methods and compositions of the disclosure are substances that are generally used as emulsifiers (e.g., sodium oleyl phosphate, sodium lauryl phosphate, sodium lauryl sulfate, sodium myristyl sulfate, polyoxyethylene alkyl ethers, polyoxyethylene alkyl esters, etc.), caproic acid, lactic acid, malic acid and citric acid and alkali metal salts thereof, pyrrolidonecarboxylic acids, alkylpyrrolidones carboxylic acid esters, N-alkylpyrrolidones, proline acyl esters, and the like.

The present formulation may also comprise other suitable agents such as nitric oxide donor agents. As used herein, the term "nitric oxide donor agents" refers to any suitable agents which are capable of releasing nitric oxide. The release of nitric oxide may have a vasodilating effect. A nitric oxide (NO) donor may be selected as a membrane penetration-enhancing agent to enhance mucosal delivery of tdsRNA, and other biologically active agents disclosed herein. Various NO donors are known in the art and are useful in effective concentrations within the methods and formulations of the disclosure. Exemplary NO donors include, but are not limited to, nitroglycerine, nitroprusside, NOC5 [3-(2-hydroxy-1-(methyl-ethyl)-2-nitrosohydrazino)-1-propanamine], NOC12 [N-ethyl-2-(1-ethyl-hydroxy-2-nitrosohydrazino)-ethanamine], SNAP [S-nitroso-N-acetyl-DL-penicillamine], NORI and NOR4. Within the methods and compositions of the disclosure, an effective amount of a selected NO donor may be coordinately administered or combinatorically formulated with tdsRNA, and/or other biologically active agents disclosed herein, into or through the mucosal epithelium.

Non-limiting examples of other permeation enhancers useful in the instant disclosure are the simple long-chain esters that are Generally Recognized As Safe (GRAS) in the various pharmacopeial compendia. These may include simple aliphatic, unsaturated or saturated (but preferably fully saturated) esters, which contain up to medium-length chains. Non-limiting examples of such esters include isopropyl myristate, isopropyl palmitate, myristyl myristate, octyl palmitate, and the like. The enhancers are of a type that is suitable for use in a pharmaceutical composition. The artisan of ordinary skill will also appreciate that those materials that are incompatible with or irritating mucous membranes should be avoided.

For nasal administration, the enhancer is present in the composition in a concentration effective to enhance penetration of the pharmaceutically active agent that is to be delivered through the nasal mucosa. Various considerations should be taken into account in determining the amount of enhancer to use. Such considerations include, for example, the amount of flux (rate of passage through the membrane) achieved and the stability and compatibility of the components in the formulations. The enhancer is generally used in an amount of about 0.001 to about 40 (w/w) % of the composition. Specific ranges include, about 0.01% to about 30 (w/w), about 0.1 to about 25% (w/w), about 1% to about 15% (w/w), about 5 to 10% (w/w). Alternatively, the amount of the enhancer may range from about 1.0 to about 3% (w/w) or about 10 to about 20% (w/w).

In forming an emulsion in which the water-insoluble enhancer is a normally solid material, the enhancer is dissolved in a suitable solvent. If the enhancer is a normally liquid material that is water-immiscible, a suitable solvent for the enhancer may or may not be used, as appropriate. The enhancer may be dissolved, dispersed, suspended, or solubilized in a suitable solvent(s) such as alcohols, oils, glycerol, ethylene glycol, propylene glycol, hexane, acetone, freon, water, other polar or non-polar solvents, or a mixture, which is then added to a composition comprising an effective amount of the desired antigen admixed with a pharmaceutical carrier. In some cases, when the enhancers are in liquid form, a "neat" solution of enhancer can be directly incorporated in the antigen, pharmaceutical carrier, and enhancer mixture, in which the concentration of enhancer ranges from about 0.1% to about 50% (w/w).

Any of the above permeation enhancers are useful, especially in nasal administration.

### 4.8 VASODILATOR OR VASOCONSTRICTOR AGENTS

The present formulation may also comprise other suitable agents such as vasodilator agents. As used herein, the term "vasodilator agents" refers to any agents which are vasoactive. A vasodilator agent may function within the disclosure to modulate the structure and physiology of the submucosal vasculature, increasing the transport rate of tdsRNA, and other biologically active agents into or through the mucosal epithelium and/or to specific target tissues or compartments (e.g., the systemic circulation). Vasodilator agents for use within the disclosure typically cause submucosal blood vessel relaxation by either a decrease in cytoplasmic calcium, an increase in nitric oxide (NO) or by inhibiting myosin light chain kinase. They are generally divided into 9 classes: calcium antagonists, potassium channel openers, ACE inhibitors, angiotensin-II receptor antagonists, alpha-adrenergic and imidazole receptor antagonists, beta-1-adrenergic agonists, phosphodiesterase inhibitors, eicosanoids and NO donors. Within certain methods and compositions of the disclosure, a selected vasodilator agent may be coordinately administered (e.g., systemically or intranasally, simultaneously or in combinatorically effective temporal association) or combinatorically formulated with tdsRNA and other biologically active agent(s) in an amount effective to enhance the mucosal absorption of the active agent(s) to reach a target tissue or compartment in the subject.

The present formulation may also comprise other suitable agents such as vasoconstrictor agents. As used herein, the term "vasoconstrictor agents" refers to any substances which may cause vasoconstriction. Vasoconstrictor agents may also be called vasoconstrictors, vasopressors, or simply "pressors." Vasoconstrictor agents may usually cause an increase in systemic blood pressure, but when they are administered in specific tissues, localized blood flow may be reduced. The extent of vasoconstriction may be slight or severe depending on the substance of vasoconstrictor agents. Many vasoconstrictor agents may also cause pupil dilation. Vasoconstrictor agents may include any suitable substances such as antihistamines, decongestants and stimulants that are used to treat ADHD. Suitable vasoconstrictor agents have been previously described by Dhuria, Hanson, et al. (Dhuria, Hanson, et al., 2009).

### 4.9 RNASE INHIBITORY AGENT AND ENZYME INHIBITOR

In some cases, for example, nasal vaccines, the disclosure encompasses the delivery of a protein, peptide or another nucleic acid in addition to tdsRNA. Therefore, the compositions of the present disclosure may contain an enzyme inhibitor. As is well known to practitioners in nucleic acid, peptide and protein biochemistry, these biopolymers tend to be very sensitive to the presence of enzymes, such as RNase and proteolytic enzymes, that rapidly degrade the biopolymer when present in even minute amounts. Typical enzyme inhibitors that are commonly employed and that may be incorporated into the present disclosure may be, for example, leupeptin, aprotinin, and the like. Enzyme inhibitors also include nuclease inhibitors such as DNase inhibitors and RNase inhibitors. RNase inhibitors are commonly used as a precautionary measure in enzymatic manipulations of RNA to inhibit and control RNase. These are commercially available from a number of sources such as, for example, Invitrogen (SUPERase, In RNase Inhibitor, RNaseOUT, RNAsecure, and RNase Inhibitor).

### 4.10 SELECTIVE TRANSPORT-ENHANCING AGENTS

The present formulation may also comprise other suitable agents such as selective transport-enhancing agents. As used herein, the term "selective transport-enhancing agent" refers to any agent that facilitates transport of tdsRNA and/or one or more biologically active agents including vaccines. The compositions and delivery methods of the disclosure may optionally incorporate a selective transport-enhancing agent that facilitates transport of one or more biologically active agents. These transport-enhancing agents may be employed in a combinatorial formulation or coordinate administration protocol with tdsRNA disclosed herein, to coordinately enhance the delivery of one or more additional biologically active agent(s). Alternatively, the transport-enhancing agents may be employed in a combinatorial formulation or coordinate administration protocol to directly enhance mucosal delivery of tdsRNA, with or without enhanced delivery of an additional biologically active agent.

Exemplary selective transport-enhancing agents for use within this aspect of the disclosure may include glycosides, sugar-containing molecules, and binding agents such as lectin binding agents, and stabilizers. For example, specific "bioadhesive" ligands, including various plant and bacterial lectins, which bind to cell surface sugar moieties by receptor-mediated interactions can be employed as carriers or conjugated transport mediators for enhancing mucosal, e.g., nasal delivery of biologically active agents within the disclosure. Certain bioadhesive ligands for use within the disclosure will mediate transmission of biological signals to epithelial target cells that trigger selective uptake of the adhesive ligand by specialized cellular transport processes (endocytosis or transcytosis). These transport mediators can therefore be employed as a "carrier system" to stimulate or direct selective uptake of one or more tdsRNA or functionally equivalent fragment proteins, analogs, mimetics, and other biologically active agent(s) into and/or through mucosal epithelia. These and other selective transport-enhancing agents significantly enhance mucosal delivery of macromolecular biopharmaceuticals (particularly peptides, proteins, oligonucleotides and polynucleotide vectors) within the disclosure.

Additional intranasal mucosal delivery-enhancing agents that are useful within the coordinated administration and processing methods and combinatorial formulations of the disclosure may also include, but are not limited to, mixed micelles, enamines, nitric oxide donors (e.g., S-nitroso-N-acetyl-DL-penicillamine, NOR1, NOR4--which are preferably co-administered with a nitric oxide scavenger such as carboxy-PITO or diclofenac sodium), sodium salicylate, glycerol esters of acetoacetic acid (e.g., glyceryl-1,3-diacetoacetate or 1,2-isopropylideneglycerine-3-acetoacetate), and other release-diffusion or intra- or trans-epithelial penetration-promoting agents that are physiologically compatible for intranasal mucosal delivery. Other absorption-promoting agents may be selected from a variety of carriers, bases and excipients that enhance mucosal delivery, stability, activity or trans-epithelial penetration of the tdsRNA. These include, inter alia, cyclodextrins and beta-cyclodextrin derivatives (e.g., 2-hydroxypropyl-beta-cyclodextrin and heptakis(2,6-di-O-methyl-beta-cyclodextrin). These compounds, optionally conjugated with one or more of the active ingredients and further optionally formulated in an oleaginous base, enhance bioavailability in the intranasal mucosal formulations. Yet additional absorption-enhancing agents adapted for intranasal mucosal delivery may also include medium-chain fatty acids, including mono- and diglycerides (e.g., sodium caprate--extracts of coconut oil, CAPMUL), and triglycerides (e.g., amylodextrin, Estaram 299, Miglyol 810).

### 4.11 STABILIZING DELIVERY VEHICLE, CARRIER, SUPPORT OR COMPLEX-FORMING SPECIES

The present formulation may also comprise other suitable agents such as a stabilizing delivery vehicle, carrier, support or complex-forming species. The coordinate administration methods and combinatorial formulations of the instant disclosure may optionally incorporate effective lipid or fatty acid-based carriers, processing agents, or delivery vehicles to provide improved formulations for mucosal delivery of tdsRNA or functionally equivalent fragment proteins, analogs and mimetics, and other biologically active agents. For example, formulations and methods for mucosal delivery can comprise one or more of these active agents, such as a peptide or protein, admixed or encapsulated by, or coordinately administered with, a liposome, mixed micellar carrier, or emulsion, to enhance chemical and physical stability and increase the half-life of the biologically active agents (e.g., by reducing susceptibility to proteolysis, chemical modification and/or denaturation) upon mucosal delivery.

Within certain aspects of the disclosure, specialized delivery systems for biologically active agents may comprise small lipid vesicles known as liposomes or micelles. These are typically made from natural, biodegradable, non-toxic, and non-immunogenic lipid molecules, and can efficiently entrap or bind drug molecules, including peptides and proteins, into, or onto, their membranes. The attractiveness of liposomes as a nucleic acid delivery system is increased by the fact that the encapsulated tdsRNA can remain in their preferred aqueous environment within the vesicles, while the liposomal membrane protects them against nuclease and other destabilizing factors.

Additional delivery vehicles carrier, support or complex-forming species for use within the disclosure may include long and medium-chain fatty acids, as well as surfactant mixed micelles with fatty acids. Most naturally occurring lipids in the form of esters have important implications with regard to their own transport across mucosal surfaces. Free fatty acids and their monoglycerides which have polar groups attached have been demonstrated in the form of mixed micelles to act on the intestinal barrier as penetration enhancers. This discovery of barrier modifying function of free fatty acids (carboxylic acids with a chain length varying from 12 to 20 carbon atoms) and their polar derivatives has stimulated extensive research on the application of these agents as mucosal absorption enhancers.

For use within the methods of the disclosure, long-chain fatty acids, especially fusogenic lipids (unsaturated fatty acids and monoglycerides such as oleic acid, linoleic acid, linoleic acid, monoolein, etc.) provide useful carriers to enhance mucosal delivery of tdsRNA, and other biologically active agents disclosed herein. Medium-chain fatty acids (C₆ to C₁₂) and monoglycerides have also been shown to have enhancing activity in intestinal drug absorption and can be adapted for use within the mucosal delivery formulations and methods of the disclosure. In addition, sodium salts of medium and long-chain fatty acids are effective delivery vehicles and absorption-enhancing agents for mucosal delivery of biologically active agents. Thus, fatty acids can be employed in soluble forms of sodium salts or by the addition of non-toxic surfactants, e.g., polyoxyethylated hydrogenated castor oil, sodium taurocholate, etc. Other fatty acid and mixed micellar preparations that are useful within the disclosure include, but are not limited to, Na caprylate (C8), Na caprate (C10), Na laurate (C12) or Na oleate (C18), optionally combined with bile salts, such as glycocholate and taurocholate.

### 5. ADMINISTRATION (DELIVERY)

Administration to the subject or administering to the subject may be in any known form including: systemic administration; intravenous administration; intradermal administration; subcutaneous administration; intramuscular administration; intranasal administration (pulmonary airway administration); intranasal administration and oral administration; intraperitoneal administration; intracranial administration; intravesical administration; oral administration (through the mouth, by breathing through the mouth); topical administration; inhalation administration; aerosol administration; intra-airway administration; tracheal administration; bronchial administration; instillation; bronchoscopic instillation; intratracheal administration; mucosal administration; dry powder administration; spray administration; contact administration; swab administration; intratracheal deposition administration; intrabronchial deposition administration; bronchoscopic deposition administration; lung administration; nasal passage administration; respirable solid administration; respirable liquid administration; dry powder inhalants administration.

Intranasal administration may be administering to nasal passages; administering to nasal epithelium; administering to lung; administering by inhalation; administering to the larynx; administering to bronchi; administering to alveoli; administering by inhalation; administering by nasal instillation; and a combination thereof.

Administering or administration may be administering to at least one tissue or cell selected from the group consisting of: an airway tissue; nose tissue; oral tissue; alveoli tissue; pharynx tissue; trachea tissue; bronchi tissue; carina tissue; bronchi tissue; bronchioles tissue; lung tissue; lobe of a lung tissue; alveoli tissue; nasal passage tissue; nasal epithelium tissue; larynx tissue; bronchi tissue; inhalation tissue; an epithelium cell; an airway epithelium cell; a ciliated cell; a goblet cell; a non-ciliated cell; a basal cell; a lung cell; a nasal cell; a tracheal cell; a bronchial cell; a bronchiolar epithelial cell; an alveolar epithelial cell; and a sinus cell.

As another example, administering may be performed by a delivery system or medical device comprising the tdsRNA. The delivery system or medical device may be a nebulizer; a sprayer; a nasal pump; a squeeze bottle; a nasal spray; a syringe sprayer or plunger sprayer (a syringe providing pressure to an attached sprayer or nozzle); a nasal aerosol device; a controlled particle dispersion device; a nasal nebulization device; a pressure-driven jet nebulizer; ultrasonic nebulizer; a breath-powered nasal delivery device; an atomized nasal medication device; an inhaler; a powder dispenser; a dry powder generator; an aerosolizer; an intrapulmonary aerosolizer; a sub-miniature aerosolizer; a propellant based metered-dose inhaler; a dry powder inhalation device; an instillation device; an intranasal instillation device; an intravesical instillation device; a swab; a pipette; a nasal irrigation device; a nasal rinse; an aerosol device; a metered aerosol device; a pressurized dosage device; a powdered aerosol device; a spray aerosol device; a spray device; a metered spray device; a suspension spray device; and a combination thereof.

The composition of the present disclosure may exist in various forms, for example, an oil-in-water emulsion, a water-in-oil emulsion, and a water-in-oil-in-water emulsion. The active compounds of the present disclosure, including where tdsRNA is in combination with other agents, may exist in either the continuous or the dispersed phase or in both phases depending upon whether the compounds are hydrophilic, lipophilic, or amphiphilic.

The composition of the present disclosure may also comprise an emulsifying agent for use in aiding the formation of an emulsion. Any suitable hydrocolloid emulsifying agent, typically a solid material, or a mixture of two or more such emulsifying agents can be used in the practice of the present disclosure. Hydrocolloid emulsifying agents include: vegetable derivatives, for example, acacia, tragacanth, agar, pectin, and carrageenan; animal derivatives, for example, gelatin, lanolin, cholesterol, and lecithin; semi-synthetic agents, for example, methylcellulose and carboxymethylcellulose; and synthetic agents, for example, acrylic emulsifying agents such as carbomers. The hydrocolloid emulsifying agent forms hydrocolloids (hydrated lyophilic colloids) around the emulsified liquid droplets of the emulsion. The hydrocolloid serves as a protective layer around each emulsified droplet which physically repulses other droplets, thus hindering Ostwald ripening (the tendency of emulsified droplets to aggregate).

In contrast, other emulsifying agents typically protect the emulsified droplets by forming a liquid crystalline layer around the emulsified droplets. In compositions that employ a liquid crystalline layer-forming emulsifying agent, the hydrophilic-lipophilic balance (HLB) of the oil phase of the emulsion must be matched with that of the emulsifying agent to form a stable emulsion and, often, one or more additional emulsifying agents (secondary emulsifying agents) must be added to further stabilize the emulsion. The aforementioned liquid crystalline layer also retards the release of the compounds of the dispersed phase upon contact with the target substrate.

The liquid compositions are particularly suited for nasal administration.

### 5.1 ADMINISTRATION ROUTE

Preferably, all of this disclosure (administrations, formulations, medicaments, compositions, dosages) relates to and describes at least to their application to a subject that is human. Additional non-human subjects are described below.

The pharmaceutical composition comprising one or more active agents (e.g., tdsRNA) of this disclosure may be administered to a subject by any local or systemic route or method known in the art. The preferred route may vary with the age, condition, gender, or health status of the subject; the nature of the disease, the number and severity of symptoms, chosen active ingredient, or the presence of other pathological conditions.

The most preferred methods include intravenous administration; intraperitoneal administration; or intranasal administration (including, e.g., breathing through the mouth or airway - e.g., through a stoma made by tracheostomy). Intravenous administration or intraperitoneal administration is commonly performed with a needle. Other administration methods include, at least, intradermal administration; subcutaneous administration; intramuscular administration; intraperitoneal administration; intracranial administration; intravesical administration; oral administration (through the mouth, by breathing through the mouth); topical administration; inhalation administration; aerosol administration; intra-airway administration; tracheal administration; bronchial administration; instillation administration; bronchoscopic instillation administration; intratracheal administration; mucosal administration; dry powder administration; spray administration; contact administration; swab administration; intratracheal deposition administration; intrabronchial deposition administration; bronchoscopic deposition administration; lung administration; nasal passage administration; respirable solid administration; respirable liquid administration; dry powder inhalants administration; and a combination thereof. It is noted where more than one active ingredient (e.g., different tdsRNAs, etc.) is administered; the active ingredients may be administered by the same route or different routes.

Some forms of administration (administering) may be described by one or more of the above categories and some administration methods may be grouped differently or may be referred to by broader terms. For example, enteral administration may refer to oral administration, feeding tube administration, or enema administration; topical administration may be by a device such as a nebulizer for inhalation through the respiratory system, by skin patch acting epicutaneously or transdermally, or by suppository methods. Parenteral administration may take the form of subcutaneous administration, intravenous administration, intramuscular administration, intradermal administration, or intraperitoneal administration; buccal administration, sublingual administration, transmucosal administration; inhalation administration, instillation administration, instillation administration, intranasally administration, instillation administration, or intratracheal administration.

Nasal administration refers to any administration through the airway and is another term for pulmonary airway administration. Nasal administration may include administration to the airway through the mouth (i.e., through breathing through the mouth or through a stoma made by tracheostomy).

Nasal administration includes administration to a tissue of the airway. This includes at least one tissue selected from the group consisting of: airway tissue; nose tissue; oral tissue; alveoli tissue; pharynx tissue; trachea tissue; bronchi tissue; carina tissue; bronchi tissue; bronchioles tissue; lung tissue; tissue in the lobe of a lung; alveoli tissue; nasal passage tissue; nasal epithelium tissue; larynx tissue; bronchi tissue; inhalation tissue; and a combination thereof. It follows that nasal administration may include administration to cells and tissues such as: an epithelium cell; an airway epithelium cell; a ciliated cell; a goblet cell; a non-ciliated cell; a basal cell; a lung cell; a nasal cell; a tracheal cell; a bronchial cell; a bronchiolar epithelial cell; an alveolar epithelial cell; a sinus cell; and a combination thereof.

Administration may also be from any known delivery system. A delivery system may be at least one selected from the group consisting of: a pill, a capsule, a needle, a cannula, an implantable drug depot, an infusion system (e.g., a device similar to an insulin pump); a nebulizer; a sprayer; a nasal pump; a squeeze bottle; a nasal spray; a syringe sprayer, a plunger sprayer (a syringe providing pressure to an attached sprayer or nozzle); a nasal aerosol device; a controlled particle dispersion device; a nasal aerosol device; a nasal nebulization device; a pressure-driven jet nebulizer; an ultrasonic nebulizer; a breath-powered nasal delivery device; an atomized nasal medication device; an inhaler; a powder dispenser; a dry powder generator; an aerosolizer; an intrapulmonary aerosolizer; a sub-miniature aerosolizer; a propellant based metered-dose inhalers; a dry powder inhalation devices; an instillation device; an intranasal instillation device; an intravesical instillation device; a swab; a pipette; a nasal irrigation device; a nasal rinse; an aerosol device; a metered aerosol device; a pressurized dosage device; a powdered aerosol; a spray aerosol; a spray device; a metered spray device; a suspension spray device; and a combination thereof.

### 5.2 FORMULATIONS AND DOSAGE

Formulations for administration (i.e., pharmaceutical compositions) may include a pharmaceutically acceptable carrier with the tdsRNA.

Pharmaceutical carriers include suitable non-toxic vehicles in which a composition of the disclosure is dissolved, dispersed, impregnated, or suspended, such as water or other solvents, fatty materials, celluloses and their derivatives, proteins and their derivatives, collagens, gelatine, polymers, adhesives, sponges, fabrics, and the like and excipients which are added to provide better solubility or dispersion of the drug in the vehicle. Such excipients may include non-toxic surfactants, solubilizers, emulsifiers, chelating agents, binding materials, lubricants, softening agents, and the like. Pharmaceutically acceptable carriers may be, for example, aqueous solutions, syrups, elixirs, powders, granules, tablets, and capsules which typically contain conventional excipients such as binding agents, fillers, lubricants, disintegrants, wetting agents, suspending agents, emulsifying agents, preservatives, buffer salts, flavoring, coloring, and/or sweetening agents.

A liquid carrier may be present in the composition in a concentration effective to serve as a suitable vehicle for the compositions of the present disclosure. In general, the carrier is used in an amount of 40 to 98 wt. %, or 50 to 98 wt. % of the composition. Preferred forms of compositions are compositions for use as nasal sprays.

The liquid carrier may be water or any other suitable liquid, solvent, or mixture thereof. The water may contain suitable buffering agents to result in a pH wherein the particular antigen is delivered optimally, or it may contain other carriers, such as glycerin, propylene glycol, polyethylene glycols of various sizes, amino acid modifiers, such as arginine and the like, and other suitable soluble excipients, as is known to those who are proficient in the art of compounding or pharmaceutics. One preferred liquid carrier is phosphate-buffered saline (PBS).

The tdsRNA comprises rIₙ•r(C₁₂U)_{n,} rI_{n•}r(C₁₂U)ₙ, and Rugged dsRNA .

### 5.3 NASAL COMPOSITIONS

A composition for enhancing intranasal delivery may includes a combination of tdsRNA and active compounds prepared for nasal delivery. The combination of tdsRNA and active compounds may be applied in a subsequent manner or a simultaneous manner. Preferably, the mixture will be in the form of an aqueous solution. The mixture may also be a powder or a dried, powdered, or lyophilized form of the mixture. These forms may be re-hydrated before delivery.

### 5.4 NASAL FORMULATIONS

In one aspect, the present disclosure relates to formulations for nasal delivery of tdsRNA. In one aspect, tdsRNA is the sole active compound and may be free of any other active compounds. In another aspect, the tdsRNA may be co-administered with one or more additional active compounds.

Each of the agents and chemicals described herein, including any combinations thereof, may be added to a tdsRNA for any form of administration, including nasal administration, to a subject.

### 5.5 MEDICAMENT

In another aspect, a medicament (e.g., a pharmaceutical composition) containing the tdsRNA is provided. Optional other components of the medicament include excipients and a vehicle (e.g., aqueous buffer or water for injection) packaged aseptically in one or more separate containers (e.g., nasal applicator or injection vial). Further aspects will be apparent from the disclosure and claims herein.

### 5.6 DOSAGE FOR THE AVERAGE SUBJECT

The dosages are generally applicable to a subject as described in another section of this disclosure. Preferably, the subject is human.

For a subject the dose of tdsRNA per day may be at least one selected from the group consisting of: 0.1 µg to 1,000,000 µg, 0.1 µg to 25,000 µg, 0.4 to 400,000 µg, 0.5 µg to 5,000 µg, 0.5 mg to 60 mg, 5 mg to 40 mg, 5 mg to 400 mg, 10 mg to 20 mg, 10 mg to 800 mg, 25mg to 700 mg, 20 mg to 200 mg, 50 mg to 150 mg, 80 mg to 140 mg, and a combination thereof.

A subject may be a human of about 68.0 kg (150 lb.) or 70 Kg in weight, and the appropriate dosage per body weight may be calculated.

### 5.7 DOSE IN KILOGRAM PER DAY

In another aspect, the tdsRNA is administered in a dose per day selected from the group consisting of 0.1 mg/kg, 0.2 mg/kg, 0.3 mg/kg, 0.5 mg/kg, 0.7 mg/kg, 0.8 mg/kg, 1 mg/kg, 2 mg/kg, 3 mg/kg, 4 mg/kg, 5 mg/kg, 6 mg/kg, 7 mg/kg, 8 mg/kg, 9 mg/kg, 10 mg/kg, 15 mg/kg, 20 mg/kg, 0.1 - 1 mg/kg, 0.1 - 2 mg/kg, 0.1 - 3 mg/kg, 0.1 - 4 mg/kg, 0.1 - 5 mg/kg, 0.1 - 6 mg/kg, 0.1 - 7 mg/kg, 0.1 - 8 mg/kg, 0.1 - 10 mg/kg, 0.1 - 20 mg/kg, 0.2 - 3 mg/kg, 0.3 - 3 mg/kg, 0.4 - 3 mg/kg, 0.6 - 3 mg/kg, and 0.8 - 3 mg/kg.

### 5.8 AMOUNT PER UNIT DOSE

The amount per unit dose of tdsRNA may be at least one selected from 0.1 mg/kg, 0.2 mg/kg, 0.4 mg/kg, 0.6 mg/kg, 0.8 mg/kg, 1 mg/kg, 2 mg/kg, 3 mg/kg, 4 mg/kg, 5 mg/kg, 6 mg/kg, 8 mg/kg, 10 mg/kg.

### 5.9 SPECIFIC EXAMPLES

The tdsRNA may be administered at a dose from 1 mg/kg to 10 mg/kg biweekly. As another example, the administration may be in 50-1400 milligrams every other day, leading to an average daily dosage of 25-700 milligrams per day. The tdsRNA may be administered at a dose from 0.50 mg/kg to 10 mg/kg every other week. 50-1400 milligrams every other day, leading to an average daily dosage of 25-700 milligrams per day.

### 5.10 DOSE FREQUENCY

The tdsRNA may be administered at a frequency selected from the group consisting of: one dose per day, one dose every 2 days, one dose every 3 days, one dose every 4 days, one dose every 5 days, 4 doses a week, 3 doses a week, 2 doses a week, 1 dose a week, one dose every two weeks, one dose every three weeks, one dose every four weeks, and one dose every month.

### 5.11 NUMBER OF DOSES AND DOSING PERIOD

The tdsRNA may be administered as a single dose, in two doses, in three doses, in four doses, in five doses, or in 6 or more doses. The dosage may be continued indefinitely. Continuous dosage may be used under some circumstances, for example, if the subject is already using an insulin pump the tdsRNA may be admixed with the insulin.

A dosing period is usually about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30 days, 1 month, 2 months, 3 months, 4 months, 6 months, 9 months or one year. In certain cases, multiple (for example, 2, 3, 4, 5, 6, 7, 8, 9, 10 or more) doses of a tdsRNA are administered to a subject in need of treatment. It is envisioned that for some subjects, the dosing periods may be 1 year, 2 years, 3 years, 5 years, 10 years or continuous.

### 5.12 NASAL DOSAGE

tdsRNA may be administered at the same dose in nasal administration as for any other form of administration. Nonlimiting specific examples of nasal administration (which is also applicable for any other form of administration) include a dose of 5 µg to 10 µg; 10 µg to 20 µg; 20 µg to 50 µg; 50 µg to 100 µg; 100 µg to 200 µg; 200 µg to 500 µg; 500 µg to 1000 µg; 1000 µg to 1500 µg; 1500 µg to 2000 µg; or any combination thereof.

Unless otherwise specified, "composition," "a composition," or "the composition" includes, at least, a composition of the disclosure or includes at least tdsRNA. Compositions may be optionally filtered and sterilized to enhance safety, stability and solubility. The composition may be formulated to enhance the delivery method. For example, the formulation may be formulated to enhance i.v. delivery, intraperitoneal delivery or nasal delivery.

### 5.13 COMPOSITIONS AND METHODS THAT ARE GENERALLY APPLICABLE AND PARTICULARLY APPLICABLE FOR NASAL ADMINISTRATION

A composition for enhancing intranasal delivery includes tdsRNA and optionally active compounds prepared for nasal delivery. The combination of tdsRNA and active compounds may be applied in a subsequent (sequential) manner or a simultaneous (parallel) manner. Preferably, the mixture will be in the form of an aqueous solution. The mixture be a powder or a dried, powdered, or lyophilized form of the mixture. These forms may be re-hydrated before delivery. The composition may be in solid, liquid or any other form such as gels and liposomes.

A composition of the disclosure (e.g., tdsRNA) that is used in nasal administration is considered a nasal composition. Compositions of the disclosure are not limited to nasal administration. That is, any composition of the disclosure may be used as a nasal composition. Similarly, nasal compositions may be used for any other purposes such as non-nasal administration.

Simultaneous administration (also called parallel administration) may also comprise the administration of two or more compositions at the same time. For example, two or more separate nasal nozzles and sprayers can each dispense a different composition for simultaneous administration. Simultaneous administration may also dispense compositions of different forms. For example, a dry powder and a liquid may be dispensed together in separate sprayers at the same time.

Each of the agents and chemicals described herein, including any combinations thereof, may be administered together with a composition of the disclosure (e.g., tdsRNA), nasally or otherwise, to a subject. Non-limiting examples of other compounds for nasal administration include RNA, DNA, adjuvants, proteins, interferons, or parts thereof.

We note that tdsRNA is stable as a solid or dissolved in water and therefore any additional component, such as phosphate-buffered saline, is optional. Other components may benefit from additional ingredients described herein.

In certain embodiments, the therapeutic agent is administered with an agent that disrupts, e.g., transiently disrupts, tight junctions, such as EGTA (see U.S. Pat. No. 6,855,549).

Furthermore, since nasal administration may be perceived by a sense of smell in the subject, additives that improve the fragrances or nasal acceptance or reduce irritation may be added. These include buffers and preservatives if the composition is not made sterile, for example, methyl hydroxybenzoate, antioxidants, flavoring agents, volatile oils, buffering agents and surfactants.

### 5.14 SPECIFIC EXAMPLES OF COMPOSITIONS

Aerosol compositions can be made with liquid and dried compositions of the disclosure to be administered via inhalation. These aerosol compositions can be placed into pressurized acceptable propellants, such as dichlorodifluoromethane, propane, and nitrogen. Compositions may be formulated as pharmaceuticals for non-pressured preparations, such as in a nebulizer or an atomizer. For compositions to be administered from multiple-dose containers, antimicrobial agents can be added.

Liquid solutions may be suitable for any administration including nasal administration. Liquid compositions may include diluents, such as water and alcohols, for example, ethanol, benzyl alcohol, propylene glycol, glycerin, and the polyethylene alcohols, either with or without the addition of a pharmaceutically acceptable surfactant, suspending agent, or emulsifying agent. The composition of the disclosure can be administered in a physiologically acceptable diluent in a pharmaceutically acceptable carrier, such as a sterile liquid or mixture of liquids, including water, saline, aqueous dextrose and related sugar solutions, an alcohol, such as ethanol, isopropanol, or hexadecyl alcohol, glycols, such as propylene glycol or polyethylene glycol such as poly(ethyleneglycol) 400, glycerol ketals, such as 2,2-dimethyl-1,3-dioxolane-4-methanol, ethers, an oil, a fatty acid, a fatty acid ester or glyceride, or an acetylated fatty acid glyceride with or without the addition of a pharmaceutically acceptable surfactant, such as a soap or a detergent, suspending agent, such as pectin, carbomers, methylcellulose, hydroxypropylmethylcellulose, or carboxymethylcellulose, or emulsifying agents and other pharmaceutical adjuvants.

The compositions may be formulated as dry, semidry, or liquid particles. The particulate pharmaceutical composition may optionally be combined with a carrier to aid in dispersion or transport. A suitable carrier such as a sugar (i.e., dextrose, lactose, sucrose, trehalose, mannitol) may be blended with the active compound or compounds in any suitable ratio.

Specific examples of compositions forms include at least the following: aerosol of liquid, aerosol suspension of respirable solid, dry powder inhalants, metered-dose inhalants, liquid/liquid suspensions, emulsions, suspensions, oil in water emulsion, and water in oil emulsions.

In reference to particles or droplets, it is envisioned that a particle or a droplet may be a solid, a liquid, or other types of particle such as a gel, a liposome, and the like. Also, it is envisioned that a composition may be dispensed as one type of particle but is delivered to a subject as a second type of particle. For example, a composition may be dispensed as a liquid particle with a high evaporation rate such that the liquid is transformed into a solid by the time the particle reaches the subject.

Certain devices require the use of various compositions suitable for the dispensing of some compositions of the present disclosure. Typically, each composition is specific to the type of device employed and may involve the use of an appropriate propellant material, in addition to the usual diluents, adjuvants and/or carriers useful in therapy. Also, the use of liposomes, microcapsules or microspheres, inclusion complexes, or other types of carriers is contemplated. Chemically modified systems may also be prepared in different compositions depending on the type of chemical modification or the type of device employed.

Compositions suitable for use with a nebulizer may also include a buffer and a simple sugar (e.g., for stabilization of the composition and regulation of osmotic pressure). The carrier is typically water (and most preferably sterile, pyrogen-free water) or a dilute aqueous alcoholic solution, preferably made isotonic, but may be hypertonic with body fluids by the addition of, for example, sodium chloride. The nebulizer composition may also contain a surfactant to reduce or prevent surface-induced aggregation caused by atomization of the solution in forming the aerosol. Optional additives include preservatives if the composition is not made sterile, for example, methyl hydroxybenzoate, antioxidants, flavoring agents, volatile oils, buffering agents and surfactants.

Compositions for use with a metered-dose inhaler device may generally comprise a finely divided powder (a composition of the disclosure) suspended in a propellant with the aid of a surfactant. The propellant may be any conventional material employed for this purpose, such as a chlorofluorocarbon, a hydrochlorofluorocarbon, a hydrofluorocarbon, or a hydrocarbon, including trichlorofluoromethane, dichlorodifluoromethane, dichlorotetrafluoroethanol, and 1,1,1,2-tetrafluoroethane, or combinations thereof. Suitable surfactants include sorbitan trioleate and soya lecithin. Oleic acid may also be useful as a surfactant.

Compositions for dispensing from a powder inhaler device may comprise a finely divided dry powder containing a composition as described herein, and may also include a bulking agent, such as lactose, sorbitol, sucrose, or mannitol in amounts that facilitate dispersal of the powder from the device, e.g., 50 to 90% by weight of the composition. The composition may be prepared in particulate form with an average particle size of less than 10 mm (or microns), most preferably 0.5 to 5 mm, for the most effective delivery to the distal lung.

Non-limiting specific examples of nasal (pulmonary) administration include at least one or more of the administration methods such as oral administration (through the mouth, by breathing through the mouth); intranasal administration (e.g., by nose drops); inhalation administration; aerosol administration; intra-airway (e.g., tracheal or bronchial) administration; bronchoscopic instillation; intratracheal administration; mucosal administration; dry powder administration; respiratory administration; instillation administration.

Another example of nasal administration includes any deposition to any part of the airway, including, for example, by spray, by a swab, intratracheal deposition, intrabronchial deposition and bronchoscopic deposition, nasal rinse, nasal lavage, a temporary or permanent depot implant.

Administration by "inhalation" may be performed using a composition of the disclosure of a size sufficiently small to pass through the mouth or nose and larynx, past the oropharyngeal region, upon inhalation and into the bronchi and alveoli of the lungs. In general, particles (droplets, liquid or solid) ranging from about 1 to 10 microns in size (more particularly, less than about 5 microns in size) are respirable and suitable for administration by inhalation. The particles can be solid or liquid. Such preparations may have a mean particle size of 4, 5, 6, 7, 8, 9, 10, 11, 12, or 13 microns.

Preparations for inhaled or aerosol delivery may be formulated as a dry powder. Preparations for inhaled or aerosol delivery maybe formulated as a wet powder, for example through the inclusion of a wetting agent. The wetting agent may be at least one selected from the group consisting of water, saline, or other liquid of physiological pH. The particles may be a liquid.

Administration by intranasal administration may be performed by particles of a larger size formulated and delivered to topically treat the nasal epithelium. Particles or droplets used for intranasal administration generally have a diameter that is larger than those used for administration by inhalation. For intranasal administration, a particle size in the range of 10-500 microns is preferred to ensure retention in the nasal cavity.

Particles for inhalation and particles for intranasal administration may be administered together. That is, particles of 1 to 500 microns are used. Particles of 1-10 or 1-13 microns may be selected for or enriched. Particles of 10-500 microns, or 15 to 500 microns may be selected for or enriched.

The compositions of the disclosure may be administered as a plurality of drops to the nasal or buccal cavity. A dose may be, for example, 1-100, 1-50, 1-20, 1-10, 1-5, drops.

Administering may comprise using a device that delivers a metered dosage of composition.

Aerosols of liquid particles of the compositions of the disclosure may be produced by any suitable means, such as with a nebulizer, pressure-driven jet nebulizer, an ultrasonic nebulizer, or other means. Aerosols of solid particles comprising the composition of the disclosure may likewise be produced with any solid particulate therapeutic aerosol generator. One illustrative type of solid particulate aerosol generator is an insufflator. Suitable compositions for administration by insufflation include finely comminuted powders which may be delivered by means of an insufflator or taken into the nasal cavity in the manner of a snuff. In the insufflator, the powder (e.g., a metered-dose thereof effective to carry out the treatments described herein) is contained in capsules or cartridges, typically made of gelatin or plastic, which are either pierced or opened in situ and the powder delivered by air drawn through the device upon inhalation or by means of a manually-operated pump. The powder employed in the insufflator consists either solely of the composition of the disclosure or of a powder blend comprising the composition and a suitable powder diluent, such as lactose, and an optional surfactant. The composition of the disclosure typically comprises from 0.1% to 100% w/w of the composition.

Another type of illustrative aerosol generator comprises a metered-dose inhaler. Metered-dose inhalers are pressurized aerosol dispensers, typically containing a suspension or solution composition of the tdsRNA in a liquefied propellant. During use these devices discharge the composition through a valve adapted to deliver a metered volume, typically from 10 µl to 200 µl, to produce a fine particle spray containing the tdsRNA. Suitable propellants include certain chlorofluorocarbon compounds, for example, dichlorodifluoromethane, trichlorofluoromethane, dichlorotetrafluoroethane and mixtures thereof. The composition may additionally contain one or more co-solvents, for example, ethanol, surfactants, such as oleic acid or sorbitan trioleate, antioxidant and suitable flavoring agents.

The preferred route and mode of administration will vary with the condition and age of the recipient, the nature of the infection or condition, and the chosen active ingredient.

### 6. NASAL ADMINISTRATION DEVICES

A device or delivery system, encompassing a composition of the disclosure is also disclosed.

The composition of the disclosure may be delivered by any nasal administration device or combination of devices. A combination refers to a composition that is both administered by two different devices or a device having the feature of two devices. Non-limiting examples of suitable devices that can be use individually or together include at least one selected from the group consisting of: a nebulizer; a sprayer (e.g., a spray bottle such as "Nasal Spray Pump w/Safety Clip, Pfeiffer SAP #60548; a squeeze bottle (e.g., bottle commonly used for nasal sprays, including ASTELIN (azelastine hydrochloride, Medpointe Healthcare Inc.) and PATANASE (olopatadine hydrochloride, Alcon, Inc.); a nasal pump spray (e.g., APTAR PHARMA nasal spray pump); a controlled particle dispersion devices (e.g., VIANASE electronic atomizer); a nasal aerosol device (e.g., ZETONNA nasal aerosol); a nasal nebulization device (e.g., EASYNOSE nebulizer, a pressure-driven jet nebulizer, or an ultrasonic nebulizer); a powder nasal delivery devices (e.g., OPTINOSE breath-powered nasal delivery device); an atomized nasal medication device (e.g., LMA MAD NASAL device); an instillation device; an inhalation device (e.g., an inhaler); a powder dispenser; a dry powder generator; an aerolizer (e.g., intrapulmonary aerosolizer or a sub-miniature aerosolizer, metered aerosol, powdered aerosol, spray aerosol); a spray; a metered spray; a metered dose inhalers (e.g., a propellant based metered-dose inhaler); a dry powder inhalation device; an intranasal instillation device; an intravesical instillation device; an insufflation device.

An application device for application to mucous membranes, such as, that of the nose, throat, and/or bronchial tubes (i.e., inhalation). This can be a swab, a pipette or a device for nasal irrigation, nasal rinse, or nasal lavage.

Another example is a syringe or plunger-activated sprayer. This could be, for example, a sprayer head (or nozzle) attached, for example, via a Luer lock, to a syringe. The syringe applies pressure to a composition that flows through the sprayer head and produces a spray or an aerosol.

### 6.1 MORE SPECIFIC EXAMPLES OF NASAL DEVICES

Aerosol: A product that is packaged under pressure and contains therapeutically active ingredients that are released upon activation of an appropriate valve system. For use as aerosols, the compounds of the present disclosure in solution or suspension may be packaged in a pressurized aerosol container together with suitable propellants, for example, hydrocarbon propellants like propane, butane, or isobutane with conventional adjuvants. The materials of the present disclosure also may be administered in aerosol but in a non-pressurized form such as in a nebulizer or atomizer.

Metered Aerosol: A pressurized dosage form comprised of metered-dose valves, which allow for the delivery of a uniform quantity of spray upon each activation.

Powdered Aerosol: A product that is packaged under pressure and contains therapeutically active ingredients in the form of a powder, which are released upon activation of an appropriate valve system.

Spray aerosol: An aerosol product that utilizes a compressed gas as the propellant to provide the force necessary to expel the product as a wet spray.

Spray: A liquid minutely divided as by a jet of air or steam. Nasal spray drug products contain therapeutically active ingredients dissolved or suspended in solutions or mixtures of excipients in non-pressurized dispensers.

Metered spray: A non-pressurized dosage form consisting of valves that allow the dispensing of a specified quantity of spray upon each activation.

Suspension spray: A liquid preparation containing solid particles dispersed in a liquid vehicle and in the form of course droplets or as finely divided solids.

Some non-limiting specific examples of commercially available devices are the Ultravent nebulizer, manufactured by Mallinckrodt, Inc., St. Louis, Mo.; the Acorn II nebulizer, manufactured by Marquest Medical Products, Englewood, Colo.; the Ventolin metered dose inhaler, manufactured by Glaxo Inc., Research Triangle Park, N.C.; and the Spinhaler powder inhaler, manufactured by Fisons Corp., Bedford, Mass.

One illustrative type of solid particulate aerosol generator is an insufflator. Suitable compositions for administration by insufflation include finely comminuted powders which may be delivered by means of an insufflator or taken into the nasal cavity in the manner of a snuff. In the insulator, the powder (e.g., a metered-dose thereof effective to carry out the treatments described herein) is contained in capsules or cartridges, typically made of gelatin or plastic, which are either pierced or opened in situ and the powder delivered by air drawn through the device upon inhalation or by means of a manually-operated pump. The powder employed in the insufflator consists either solely of the active ingredient or of a powder blend comprising the active ingredient, a suitable powder diluent, such as lactose, and an optional surfactant. The active ingredient typically comprises from 0.1 to 100 w/w of the composition.

A second type of illustrative aerosol generator comprises a metered-dose inhaler. Metered-dose inhalers are pressurized aerosol dispensers, typically containing a suspension or solution composition of the active ingredient in a liquefied propellant. During use these devices discharge the composition through a valve adapted to deliver a metered volume, typically from 10 to 200 ul, to produce a fine particle spray containing the active ingredient. Suitable propellants include certain chlorofluorocarbon compounds, for example, dichlorodifluoromethane, trichlorofluoromethane, dichlorotetrafluoroethane and mixtures thereof. The composition may additionally contain one or more co-solvents, for example, ethanol, surfactants, such as oleic acid or sorbitan trioleate, antioxidant and suitable flavoring agents.

It is noted that while some of the devices may dispense a liquid, the liquid may be a rapidly evaporating liquid that would turn into a dry powder before contact with a patient. Therefore, in effect, the spray can be considered a dry powder administration.

Any of the listed devices may be incorporated into an administration device embodiment of this disclosure.

### 7. DISCUSSION OF FURTHER FEATURES

### 7.1 SUBJECT OR PATIENT

As used herein, a "subject" has the same meaning as a "patient" and is a mammal, preferably, a human. In addition to humans, categories of mammals within the scope of the present disclosure include, for example, farm animals, domestic animals, laboratory animals, etc. Some examples of farm animals include cows, pigs, horses, goats, etc. Some examples of domestic animals include dogs, cats, etc. Some examples of laboratory animals include primates, rats, mice, rabbits, guinea pigs, etc. Other examples of subjects include any animal such as civet cats, swine, cattle, horses, camels, cats, dogs, rodents, birds, bats, rabbits, ferrets, mink, snake, and the like. As used herein, the terms "patient" or "subject" are used interchangeably.

### 7.2 DEVICES AND KITS

In another aspect, the present disclosure relates to and comprises a therapeutic device for intranasal delivery. The therapeutic device may comprise any suitable devices charged with a preparation of tdsRNA and optionally, another biologically active agent such as a vaccine or antigen. These devices are described in more detail below.

### 7.3 ADDITIONAL METHODS AND COMPOSITIONS

In any aspect of this disclosure, the method may comprise a further step of administering to the subject one or more compound or agent selected from the group consisting of: antiviral, interferon, interferon mixture, Alferon, alpha-interferon species, recombinant or natural interferon-alpha, recombinant or natural interferon-alpha-2a, recombinant or natural interferon - beta, recombinant or natural interferon - beta-1b, and recombinant or natural interferon-gamma.

As used herein, the term "interferon" (abbreviated "IFN") refers collectively to type 1 and type 2 interferons and including deletion, insertion, or substitution variants thereof, biologically active fragments thereof, and allelic forms thereof. As used herein, interferon refers collectively to type 1 and type 2 interferons. Type 1 interferon includes interferons alpha, beta, omega and their subtypes. Human interferon alpha has at least 14 identified subtypes while interferon beta has 3 identified subtypes.

The interferon may be at least one selected from the group consisting of: interferon, interferon mixture, Alferon, alpha-interferon species, recombinant or natural interferon alpha, recombinant or natural interferon alpha 2a, recombinant or natural interferon beta, recombinant or natural interferon beta 1b, recombinant, and natural interferon gamma.

The interferon is optionally an alpha-interferon. One preferred alpha interferon is ALFERON N Injection^{®} the only approved natural, multi-species, α-interferon available in the United States. It is the first natural source, multi-species interferon and is a consistent mixture of at least seven species of α-interferon. The interferon is preferably a natural cocktail of at least seven species of human α-interferon. In contrast, the other available α-interferons are single molecular species of α-interferon made in bacteria using DNA recombinant technology. These single molecular species of α-interferon also lack an important structural carbohydrate component because this glycosylation step is not performed during the bacterial process.

Unlike species of α-interferon produced by recombinant techniques, ALFERON N Injection^{®} is produced by human white blood cells that are able to glycosylate the multiple α-interferon species. Reverse-phase HPLC studies show that ALFERON N Injection^{®} is a consistent mixture of at least seven species of alpha interferon (α2, α4, α7, α8, α10, α16 and α17). This natural-source interferon has unique antiviral properties distinguishing it from genetically engineered interferons. The high purity of ALFERON N Injection^{®} and its advantage as a natural mixture of seven interferon species, some of which, like species 8b, have greater antiviral activities than other species, for example, species 2b, which is the only component of INTRON A^{®}. The superior antiviral activities, for example, in the treatment of chronic hepatitis C virus (HCV) and HIV infection, and tolerability of ALFERON N Injection^{®} compared to other available recombinant interferons, such as INTRON A^{®} and ROFERON A^{®}, have been reported. ALFERON N Injection^{®} is available as an injectable solution containing 5,000,000 international units (IU) per ml.

The interferon may be interferon species purified as a mixture of at least seven species of alpha-interferon produced by human white blood cells. The seven species may be, for example, interferon alpha 2; interferon alpha 4; interferon alpha 7; interferon alpha 8; interferon alpha 10; interferon alpha 16; and interferon alpha 17.

For internal or any administration, the α-interferon may, for example, be formulated in conventional manner for oral, nasal or buccal administration. Formulations for oral administration include aqueous solutions, syrups, elixirs, powders, granules, tablets and capsules which typically contain conventional excipients such as binding agents, fillers, lubricants, disintegrants, wetting agents, suspending agents, emulsifying agents, preservatives, buffer salts, flavoring, coloring and/or sweetening agents. α-Interferon may be administered by any method of administration of this disclosure. Preferably administration is by a suitable route including oral, nasal, parenteral (including injection) or topical (including transdermal, buccal and sublingual). It will be appreciated that the preferred route will vary with the condition and age of the recipient, the nature and severity of Long COVID and chosen tdsRNA.

The recommended dosage of the components will depend on the clinical status of the patient and the experience of the clinician in treating similar conditions. As a general guideline, a dosage of ALFERON N Injection^{®} utilized for systemic infections is 7 IU/kg (3 IU/pound), to 22 milion IU/kg (10 million IU/pound) (e.g., subcutaneous injection) three times weekly. Experience to date is with dosages above 7 IU/kg (3 IU/lb) of patient body weight. Oral α-interferon (ALFERON LDO^{®}) has been administered as a liquid solution in the range of 500-10,000 IU/day and calculated on the basis of a 68.0 kg (150 pound) human this is from 7.3 to 146 IU/kg per day (3.3 to 66.0 IU/lb per day). In one preferred embodiment, beneficial results are obtained at dosage levels of α-interferon in excess of 450 IU, that is, greater than 7 IU/kg body weight (3 IU/pound body weight). A healthcare provider would be able, however, to determine the optimal dose and schedule of low dose oral α-interferon (or any interferon) to achieve a desired antiviral effect.

In another aspect, the agent may be at least one selected from the group consisting of Remdesivir, chloroquine, hydroxychloroquine, oseltamivir, zanamivir, abacavir, zidovudine, zalcitabine, didanosine, stavudine, efavirenz, indinavir, ritonavir, nelfinavir, amprenavir, ribavirin, interleukin, IL-2, PD-L1, Anti-PD-L1, checkpoint inhibitor, peramivir, and neuraminidase inhibitors.

The compositions and methods of this disclosure may comprise any compound/agent discussed herein including, e.g., in this previous paragraph.

### 7.4 EFFECTIVE AMOUNT: THERAPEUTICALLY OR PROPHYLACTICALLY EFFECTIVE AMOUNT

The compositions are delivered in effective amounts. The term "effective amount" refers to the amount necessary or sufficient to realize a desired biological effect which is, for example, reducing, stopping the advance of, or reversing the symptoms of Long-COVID. In addition to the sample dosages and administration methods mentions, one of ordinary skill in the art can empirically determine the effective amount of the tdsRNA without necessitating undue experimentation. It is preferred that a maximum dose be used, that is, the highest safe dose according to medical judgment.

Effective dosage forms, modes of administration, and dosage amounts may be determined empirically, and making such determinations is within the skill of the art. It is understood by those skilled in the art that the dosage amount will vary with the route and mode of administration, the rate of excretion, the duration of the treatment, the identity of any other drugs (e.g., antiviral agent) being co-administered, the age, size, species of mammal (e.g., human patient), and other factors well known in the arts of medicine and veterinary medicine. In general, a suitable dose of any active agent disclosed herein or a composition containing the same will be that amount of the active agent (tdsRNA) or composition comprising the active agent, which is the lowest dose effective to produce the desired effect. The desired effect may be to reduce the severity or duration of a symptom of a viral infection or Long-COVID.

### 8. OTHER ASPECTS

In this specification, stating a numerical range, it should be understood that all values within the range are also described (e.g., one to ten also includes every value between one and ten as well as all intermediate ranges such as two to ten, one to five, and three to eight). The term "about" may refer to the statistical uncertainty associated with a measurement or the variability in a numerical quantity that a person skilled in the art would understand does not affect the operation of the disclosure or its patentability. A claim which recites "comprising" allows the inclusion of other elements to be within the scope of the claim. The disclosure is also described by such claims reciting the transitional phrases "consisting essentially of" (i.e., allowing the inclusion of other elements to be within the scope of the claim if they do not materially affect the operation of the disclosure) or "consisting of" (i.e., allowing only the elements listed in the claim other than impurities or inconsequential activities which are ordinarily associated with the disclosure) instead of the "comprising" term. Any of these three transitions can be used to claim the disclosure.

An element described in this specification should not be construed as a limitation of the claimed disclosure unless it is explicitly recited in the claims. Thus, the granted claims are the basis for determining the scope of legal protection instead of a limitation from the specification which is read into the claims. In contradistinction, the prior art is explicitly excluded from the disclosure to the extent of specific embodiments that would anticipate the claimed disclosure or destroy novelty.

Moreover, no particular relationship between or among limitations of a claim is intended unless such relationship is explicitly recited in the claim (e.g., the arrangement of components in a product claim or order of steps in a method claim is not a limitation of the claim unless explicitly stated to be so). All possible combinations and permutations of individual elements disclosed herein are considered to be aspects of the disclosure. Similarly, generalizations of the disclosure's description are considered to be part of the disclosure.

From the foregoing, it would be apparent to a person of skill in this art that the disclosure can be embodied in other specific forms.

In this disclosure, Long COVID refers to long-term effect after COVID-19 (SARS-CoV-2 infection) and has the same meaning as Post COVID-19 Fatigue Syndrome (PCFS) or Post COVID-19 Chronic Fatigue (PCCF).which is a term we used in our previous publications and patent applications. The terms have the same meaning.

### EXAMPLES

### Example 1 Experiment to Measure Treatment of Long COVID

The study is a double-blind trial with equal parallel cohorts conducted to evaluate the safety and efficacy of tdsRNA in patients with Long COVID. The Long COVID patients are tested for seropositivity for anti-SARS-CoV-2 antibody. In addition, the patients are tested for SARS-CoV-2 by PCR-based testing (e.g., RTPCR). Many of the Long COVID patients (also referred to herein as subjects) are unable to physically perform the standard Bruce sub-maximal exercise protocol commonly used for the evaluation of cardiac function. For patient safety reasons, a modified Bruce ME/CFS protocol is used that was similar in energy requirements to a Bruce protocol designed for the elderly. The primary endpoint is changed in exercise treadmill tolerance (ETT) from baseline to Week 40.

SAS (Version 9.2) statistical software (Cary, NC) is used to analyze the data. All statistical analyses were two-sided. Exercise treadmill duration and vertical rise are analyzed using the two-sided Student's T-test. A comparison of the proportion of patients who improved exercise treadmill tolerance (ETT) by at least 25% was analyzed using the Chi-squared test. Study participants are required to undergo exercise treadmill tolerance (ETT) testing using a modified Bruce protocol which incorporated progressive increases in the treadmill inclination from 0% to 21% in seven 3% increments. The exercise treadmill tolerance (ETT) testing protocol has a vertical component which is calculated for each of the inclination stages completed. The last stage attempted, which was usually only partially completed, is also included in the calculation based on the percentage of completion. The increase in vertical rise from baseline (before treatment) up to Week 40 (40 weeks of treatment) is calculated for each patient and is expressed as vertical feet "climbed."

### Example 2 Additional Monitoring Criteria

In addition to treadmill tolerance (ETT), patients are also monitored under the following criteria for Long COVID. Under this criteria, a patient should be positive under both (a) and (b) as follows:
Under (a), a patient should have at least one symptom from the following:
- fatigue include severe fatigue,
- inability to exercise or be active because of fatigue,
- low exercise tolerance, such as, for example, low exercise treadmill tolerance (ETT).

Under (b), a patient should have at least 4 symptoms selected from the group consisting of: shortness of breath or difficulty breathing, persistent chest pain or pressure, cough, heart palpitations, diarrhea, partial or complete loss of sense of smell, tachycardia, hair loss, blurry vision, neuropathy in feet and hands, partial or complete loss of sense of taste, nausea or vomiting, clogged ears, dry eyes, tremors or shakiness, floaters or flashes of light in vision, rash, tinnitus or humming in ears, changed sense of taste, dry or peeling skin, phantom smells, costochondritis, low blood oxygen, covid toes, thrush, dyspnea, phlegm in back of throat, constant thirst, muscle twitching, heat intolerance, abnormally low temperature, cold burning feeling in lungs, goiter or lump in throat, dry scalp or dandruff, anemia, elevated thyroid, sicca syndrome, red eyes, dysgeusia, sputum production, lack of appetite, vertigo, muscle pain, cognitive problems, brain fog, problems with concentration, problems with thinking, chills, sweats, sleep problems, muscle or body aches, difficulty concentrating or focusing, headache, difficulty sleeping, anxiety, memory problems, dizziness, joint pain, sore throat, night sweats, fever or chills, congested or runny nose, sadness, reflux or heartburn, changing symptoms, abdominal pain, lower back pain, shortness of breath or exhaustion from bending over, weight gain, calf cramps, sleeping more than normal, upper back pain, nerve sensations, sharp or sudden chest pain, confusion, feeling irritable, weight loss, post nasal drip, dry throat, high blood pressure, swollen hands or feet, mouth sores or sore tongue, neck muscle pain, hot blood rush, bone aches in extremities, feeling of burning skin, extreme pressure at base of head or occipital nerve, swollen lymph nodes, brain pressure, kidney pain, spikes in blood pressure, hand or wrist pain, bulging veins, mid-back pain at base of ribs, burning sensations, painful scalp, jaw pain, arrhythmia, cracked or dry lips, foot pain, eye stye or infection, low blood pressure, kidney issues or protein in urine, urinary tract infection, Hormone imbalances, drastic personality change, gastroesophageal reflux disease with excessive salivation, herpes infection, EBV infection, trigeminal neuralgia, bilateral neck throbbing around lymph nodes, syncope, sadness, chest pain, rhinitis, and myalgia.

### Example 3 Experiment to measure the treatment of Long COVID

The objective of this study will be to evaluate the efficacy of a tdsRNA for reducing the symptoms of patients suffering from Long COVID. The study will be in the form of a prospective, randomized, double-blind, placebo-controlled, parallel-group study using a formulation of tdsRNA. Male and female patients (over 18 years) who have been previously diagnosed with SARS-CoV-2 infection are included in this trial. The main exclusion criteria will include pregnant and lactating woman, patients suffering from other inflammatory rheumatological diseases (such as rheumatoid arthritis or collagenoses), severe neuropathies, clinically manifest endocrinopathies, severe cardial, renal or hepatic impairment and acute or chronic infections, hypothyroidism, sleep apnea and narcolepsy, and iatrogenic conditions such as side effects of medication.

Fifty patients will be randomly assigned to one of two study cohorts: placebo (25 patients; cohort 1) or tdsRNA (25 patients; cohort 2). The treatment duration will be eight weeks over which each patient will receive intravenous injections of placebo or a tdsRNA formulation. Before the commencement of the study, each patient in each cohort will receive a placebo injection of PBS solution (day -3) to provide an intra-patient placebo control and to allow the patient to practice completion of the diary and assess whether patients are capable of measuring their own in injection site reactions accurately.

Patients who are willing and able to proceed with the study will be injected with placebo (PBS solution, cohort 1) or tdsRNA in PBS (cohort 2) on each administration. Doses of placebo or tdsRNA will be about 5 mg/kg three times a week administered over an eight-week period. For cohort 1, a standard volume of 0.1 ml of PBS-solution will be injected. For cohort 2, a standard volume of 0.1 ml of a suspension containing tdsRNA will be injected. The tdsRNA may be Ampligen, or a combination of Ampligen and Rugged dsRNA.

On each dosing day and at the end of the treatment phase, routine safety assessments will be performed using, for example, physical examinations, urinalysis, electrocardiograms and hematological and biochemical blood tests.

In order to document daily the symptoms of Long COVID, patients will use a standardized diary and will record daily the parameters mentioned (see, e.g., Example 2 for a list of parameters). Optionally, exercise treadmill tolerance (ETT) test will be performed also to assess the treatments.

### Example 4 A Study Of tdsRNA for Treating Patients With Long Covid

While this Example has been written in the present tense, it should be understood that, for some patients, this study has reached 21 weeks after the first dose of tdsRNA was administered around January of 2021.

Rintatolimod (Ampligen^{®}), poly I:poly C₁₂U is a specific form of tdsRNA. Double-stranded RNAs act as modulators of lymphokines, molecules that mediate cellular immune and antiviral activities, including interferon, interleukin and tumor necrosis factor (TNF). Also, dsRNAs directly activate intracellular pathways associated with antiviral and immune enhanced states.

COVID-19 is a recently emergent disease caused by infection by a newly discovered virus SARS-CoV-2. Besides its devastating death toll, it is increasingly clear that COVID-19 has at least one additional long-term detrimental effect - a Long Covid with prolonged or permanent manifestation that is very debilitating. In one report of 143 patients, approximately 55% of the patients had 3 or more symptoms of COVID-19 still present after 60 days. In similar reports, of over 1500 responses to a poll, 100% of the patients continued to have fatigue after COVID infection. In addition, over 50% of the patients reported at least one of six symptoms, including the now common fatigue and breathing problems as well as muscle or body aches, inability to exercise or be active, headache, and difficulty concentrating or focusing.

In this study, Long Covid patients showing post-COVID fatigue were tested to see if they would be benefit from tdsRNA treatment. Evaluation of safety is a secondary objective. Several quality of life and activity measures including Karnofsky Performance Scores, SF-36 Health Questionnaire, Multi-Dimensional Fatigue Inventory, Symptom Specific Severity, and COVID-19, Long Covid / Long Hauler's Symptom Questionnaire will also be followed.

This ongoing study is a prospective, open-label, multi-center study to treat CFS patients with tdsRNA (poly I:poly C₁₂U, also called Ampligen^{®}). Baseline period will be up to twelve (12) weeks and patients will be treated: 1) for at least a minimum of 24 weeks or as long as the patient is clinically benefiting from the treatment or 2) until dose-limiting toxicity, if any, occurs. At this time, there is no indication of toxicity.

The study parameters include: (1) Karnofsky Performance Scores (KPS); (2) Safety Assessments; (3) SF-36, Health Questionnaire; (4) Specific Symptom Severity; (5) Multi-Dimensional Fatigue Inventory; (6) COVID-19 Symptom Questionnaire; and (7) Long Covid / Long Hauler's Symptom Questionnaire (for Long Covid patients only).

When patients are enrolled with Long-COVID symptoms. Inclusion Criteria includes:
(a) Diagnosis of Chronic Fatigue Syndrome as defined by the 1988 CDC case definition for Chronic Fatigue Syndrome (CFS) ongoing for > 6 months or as defined only by the 1994 CDC case definition for CFS (Fukuda et al., Ann Intern Med. 1994; 121:953-959). Other clinical conditions which could present with similar symptoms were excluded.
(b) Age Range: > 18 years old, < 70 years old.
(c) Males or non-pregnant, non-lactating females: Females must be of non-child bearing potential (either post-menopausal for two years or surgically sterile including tubal ligation) or using an effective means of contraception (birth control pills, intrauterine device, diaphragm). Alternatively, female patients with a male partner having a successful vasectomy (considered successful if a volunteer reports that a male partner has either documentation of azoospermia by microscopy or a vasectomy more than 2 years ago with no resultant pregnancy despite sexual activity post-vasectomy). Females who are less than two (2) years post-menopausal, those with tubal ligations and those using contraception must have a negative serum pregnancy test at baseline within the four (4) weeks prior to the first study medication infusion. Every four weeks, and at study termination a pregnancy test should be performed, either serum or urine stick test. However, if the urine result is positive, a serum pregnancy test will be performed. Females of childbearing potential agree to use an effective means of contraception from four (4) weeks prior to the baseline pregnancy test until four (4) weeks after the last study medication infusion. All male patients agree not to be a sperm donor and to use an effective means of contraception while on study medication and until 90 days after the last study medication infusion. Any pregnancy that occurs while taking Ampligen^{®} should be recorded as an AE and reported immediately to AIM ImmunoTech Inc.
(d) A reduced quality of life as determined by a Karnofsky Performance Score (KPS) of >= 20 and ≤ 60. The KPS must be rounded in increments of ten (10).
(e) Ability to provide written informed consent indicating a willingness to participate in this investigational study.
(f) Documentation (during baseline or historically following the onset of CFS) of a negative ANA or a negative anti-ds (double-stranded) DNA, a negative Rheumatoid Factor, and an erythrocyte sedimentation rate (ESR). Documentation during baseline of a normal T4 (or other laboratory evidence that subject is euthyroid) is also required.
(g) Laboratory confirmed negative SARS-CoV-2 (COVID-19) infection by a government-approved test/kit within 2 weeks prior to starting study drug dosing.
(h) Patients with Long Covid must meet the 1988 or 1994 CFS CDC Definition for Chronic Fatigue Syndrome (Criteria 4.1.1) except for the duration of the fatiguing illness which must have continued for at least 3 months and must not have preceded the onset of the COVID-19 symptoms. The patient must also have at least one of the following "Long Hauler" symptoms which must have persisted or recurred during 3 or more consecutive months of illness and must not have preceded the onset of the COVID-19 symptoms (fever or chills, cough, shortness of breath or difficulty breathing, new loss of taste or smell or chest pain). Since many patients with mild or no COVID-19 symptoms were not tested for the presence of SARS-CoV-2, many patients with Long Covid, also called "Long Haulers," will not have a history of a positive SARS-CoV-2 test result. A positive serum antibody test for SARS-CoV-2 will be sufficient in these cases.

Exclusion criteria include: (a) Inability to return for scheduled treatment and assessments. (b) Chronic or intercurrent acute medical disorder or disease making implementation or interpretation of the protocol or results difficult or unsafe. (c) Pregnant or lactating females (See 4.1.3). (d) Therapy with interferons, interleukins, or other cytokines or investigational drugs within 6 weeks of beginning study medication. Subjects must give written informed consent prior to discontinuation of investigational drugs.

The following drugs are prohibited during the study: (a) Interferons, interleukins or other cytokines. (b) Investigational drugs and experimental agents not yet approved for any use in the United States. (c) Other drugs deemed necessary for the patient's wellbeing may be administered with the consent of the Investigator. Investigators are discouraged from prescribing or recommending the use of any unapproved therapies for CFS during the study. All concomitant medications will be recorded in the patient's case report forms/eCRF.

Dosing Schedule: Patients will receive open label intravenous (IV) poly I:poly C₁₂U (Ampligen^{®}) twice weekly for at least 24 weeks (see Section 3.0). During the first two (2) weeks (doses 1 through 4) the patient will receive 200 mg (80 ml) doses of poly I:poly C₁₂U (Ampligen). If this dose is well-tolerated, the dose will be increased at week 3 (dose 5) to 400 mg (160 ml). The first infusion at each dose level will be given over 60 ± 5 minutes; if well tolerated without any significant infusion-related side effects (e.g., flu-like symptoms), the next infusion will be over 45 ± 5 minutes; if well tolerated, all remaining infusions will be given over 35 ± 5 minutes. If significant infusion-related side effects occur, the duration of the infusions will not be decreased until it is well tolerated and may be increased to mitigate infusion-related intolerance. Also, patients may be treated with Benadryl before the infusion to reduce the likelihood of significant infusion-related side effects such as flushing or pruritus. Ampligen is not infused in less than 30 minutes.

Patients with a history of increased sensitivity to medications in general or the investigator believes may have more sensitivity to Ampligen may receive the first dose at a reduced dosage and /or increased infusion duration. Subsequent doses shall be titrated up to the recommended dose of 400 mg given over 35 ±5 minutes or until no longer tolerated. However, based on the discretion of the principal investigator, a lower dose and/or frequency may be administered.

A dosage reduction to 25% (i.e., 100 mg), 50% (i.e., 200 mg) or 75% (i.e., 300 mg) or greater of the full (400 mg) dose will be permitted if significant infusion-related side effects continue with infusions given over 60 ± 5 minutes and the infusion duration may be increased to mitigate infusion-related intolerance. Also, patients who are restarting on Ampligen and have required reduced dosage levels during prior treatment cycles or patients with a history of increased sensitivity to medications in general or the investigator believes may have more sensitivity to Ampligen may be restarted on reduced dosage level (i.e., < 200 mg) of Ampligen and slowly escalated to a maximum of 400 mg twice weekly.

Poly I:poly C₁₂U (Ampligen^{®}) will be provided in doses of 200 mg (80 ml; concentration of 2.5 mg/mL) as a solution in glass bottles for intravenous infusion. Store Ampligen at temperatures from 2°C to 8°C.

### Evaluations and Assessments:

This section details the evaluations, procedures, and tests to be performed during thestudy as well as the frequency.

Medical History: A complete medical history will be conducted prior to study enrollment to confirm the diagnosis of CFS and determine eligibility for this study. Patient complaints and adverse experiences will be recorded at each study visit.

Physical Examination and Investigator Assessment of Signs and Symptoms (S/S): A complete physical examination, including an Investigator Assessment of Signs and Symptoms, will be performed at baseline, every 12 weeks and at termination. Vital signs, heart rate and blood pressure will be obtained before and after each infusion.

Weight: Patient's weight will be determined at baseline, every 12 weeks and at termination.

Chemistry Panel: The chemistry panel will consist of serum creatinine, electrolytes, and a biochemical profile (calcium, phosphate, glucose, blood urea nitrogen, uric acid, cholesterol, total protein, albumin, bilirubin [total], alkaline phosphatase, lactic dehydrogenase, SGOT, SGPT). The chemistry panel will be performed 2 times at baseline at least 1 week apart, then one time at week 4, 8, 16 and 24, every 8 weeks thereafter, and at termination. If no evidence of laboratory toxicity is observed in the 24 week period, the frequency of testing will be reduced to every 12 weeks. If there is evidence of toxicity the frequency will remain at every 8 weeks until there is a 24-week period that there is no toxicity.

Urinalysis: Urinalysis will be performed one time at baseline, then one time at week 4, 8, 16 and 24, every 8 weeks thereafter, and at termination. If no evidence of laboratory toxicity is observed in the 24 week period, the frequency of testing will be reduced to every 12 weeks. If there is evidence of toxicity the frequency will remain at every 8 weeks until there is a 24-week period that there is no toxicity.

Coagulation Panel: The coagulation battery (PT and PTT) will be performed one time at baseline, at week 4, 8, 16 and 24, every 8 weeks thereafter, and at termination. If no evidence of laboratory toxicity is observed in the 24 week period, the frequency of testing will be reduced to every 12 weeks. If there is evidence of toxicity the frequency will remain at every 8 weeks until there is a 24-week period that there is no toxicity.

Hematology Panel: The complete blood count (CBC) will include hematocrit, hemoglobin, total WBC with differential including neutrophils, lymphocytes, monocytes, eosinophils, basophils, platelet count. The complete blood count will be performed 2 times at baseline at least 1 week apart, then at week 4, 8 16 and 24 weeks, every 8 weeks thereafter, and at termination. If no evidence of laboratory toxicity is observed in the 24 week period, the frequency of testing will be reduced to every 12 weeks. If there is evidence of toxicity the frequency will remain at every 8 weeks until there is a 24-week period that there is no toxicity.

Thyroid Function Panel: The Thyroid Function Panel will consist of TSH, free T4, and free T3 and will be done once at baseline, at week 24, every 24 weeks, and at termination.

C reactive protein: C reactive protein will be performed 2 times at baseline at least 1 week apart, then at week 4, 8, 16 and 24 weeks, every 12 weeks thereafter, and at termination.

Direct Coombs Assay (Direct Anti-globin assay): Direct Coombs Assay will be performed once at baseline, then at week 24, every 24 weeks and at termination.

Pregnancy Test: Serum pregnancy test will be performed at baseline (within 4 weeks of starting study drug). Either serum or urine stick pregnancy test will be performed every 4 weeks and at termination on all female patients of childbearing potential while patient is receiving study medication. If the urine stick test is positive, a serum test will be performed. (See section 4.1.3).

Karnofsky Performance Scores (KPS): KPS will be evaluated and recorded by the Investigator at baseline (2 times, at least one week apart), week 8, week 16, week 24, every 12 weeks thereafter and at termination (Appendix E). KPS Questionnaire will be completed by study participants at baseline (2 times, at least one week apart), week 8, week 16, week 24, every 12 weeks thereafter and at termination.

Chest X-Ray (CXR): A CXR (PA and lateral) will be done at baseline or within 12 weeks of starting study drug infusions, at week 120 and at study termination.

Electrocardiogram (EKG): A 12-lead EKG will be recorded at baseline, every 12 weeks, at termination and when medically indicated.

Sera for Frozen Storage: A 22 ml blood sample will be obtained for serum collection twice (separated by at least 2 weeks) at baseline, then at week 8, week 16, week 24, every 12 weeks thereafter, and at termination. Samples will be stored for possible future anti-dsRNA antibody testing and/or SARS-CoV-2 antibody testing.

Health Questionnaire: Short Form-36 Health Questionnaire will be completed at baseline, week 12, week 24, every 12 weeks thereafter and at termination.

Specific Symptom Severity: Specific Symptom Severity Questionnaire will be completed at baseline, week 12, week 24, every 12 weeks thereafter and at termination.

Multi-Dimensional Fatigue Inventory: Multi-Dimensional Fatigue Inventory Questionnaire will be completed at baseline, week 12, week 24, every 12 weeks thereafter and at termination.

COVID-19 Symptoms: COVID-19 Symptoms will be evaluated at baseline and weekly. Patients who develop symptoms of COVID-19 will be tested and if positive for presence of SARS-CoV-2 virus, will be discontinued from treatment and should be followed until resolution of COVID-19 symptoms even with a negative test for SARS-CoV-2 virus. The safety follow-up of COVID-19 symptoms will occur on a weekly basis for the first month and then monthly thereafter until the resolution of symptoms.

Viral Testing for COVID-19 (SARS-CoV-2): Nasal swab samples (or another currently acceptable collection method for sample) will be performed at baseline within 2 weeks prior to starting study treatment.

Long Covid Symptom Questionnaire (for Long Covid patients only): Long Covid / Long Hauler's Symptom Questionnaire will be completed at baseline, weekly thereafter and at termination.

Pre-study analysis performed during the twelve (12) weeks prior to starting therapy includes the following: (1) Medical History. (2) Physical Examination (including the Investigator Assessment of Signs andSymptoms). (3) Weight. (4) Chemistry Panel (2 times at least 1 week apart); once within four (4) weeks prior to starting study treatment. (5) Hematology Panel (2 times at least 1 week apart); once within four (4) weeks priorto starting study treatment. (6) Thyroid Function Panel (1 time). (7) C Reactive protein (2 times at least 1 week apart); once within four (4) weeks priorto starting study treatment. (8) Direct Coombs Assay (Direct Anti-globin assay). (9) During baseline or historically following the onset of CFS; documentation of a negative ANA or a negative anti-ds (double-stranded) DNA, and a negative Rheumatoid Factor. (10) During baseline or historically following the onset of CFS; documentation of a normal erythrocyte sedimentation rate (ESR). (11) Urinalysis. (12) Coagulation Panel. (13) Pregnancy test (when applicable, within four (4) weeks before starting study treatment). (14) KPS (2 times at least 1 week apart) (Appendix E); once within four (4) weeks priorto starting study treatment. (15) EKG. (16) Chest x-ray (PA and lateral) (must be done within 12 weeks of starting study drug infusions). (17) Sera for Frozen Storage (2 times separated by at least 2 weeks). (18) Patient Questionnaires (SF-36, Multi-Dimensional Fatigue Inventory, Specific Symptom Severity, and Post-COVID-19 Chronic Fatigue (Long Covid Symptom Questionnaire (forLong Covid patients only) (2 times at least 1 week apart); once within four (4) weeks prior to starting study treatment. (19) COVID-19 Symptoms at study entry and prior to starting study treatment. (20) Viral Testing for COVID-19 (SARS-CoV-2) once within 2 weeks prior to starting study treatment.

Further analysis is performed as follows.

Every week: (a) COVID-19 Symptoms: Patients who develop symptoms of COVID-19 willbe tested and if positive for the presence of SARS-CoV-2 virus, will be discontinued from treatment and should be followed until resolution of COVID-19 symptoms even with a negative test for SARS-CoV-2 virus. The safety follow-up of COVID-19 symptoms will occur on a weekly basis for thefirst month and then monthly thereafter until the resolution of symptoms. (b) Long Covid Questionnaire (for Long Covid patients only). (c) Study drug administration twice (2) weekly.

Every 4 weeks: (a) Chemistry Panel. (b) Hematology Panel. (c) Coagulation Panel. (d) Urinalysis. (e) Pregnancy Test (when applicable).

Every 8 weeks: (a) Chemistry Panel. (b) Urinalysis. (c) Coagulation Panel. (d) Hematology Panel. (e) C Reactive Protein. (f) Sera for Frozen Storage. (g) KPS. (h) Pregnancy Test (when applicable).

Every 12 weeks: (a) Physical Examination (including Investigator Assessment of Signs andSymptoms). (b) Weight. (c) EKG. (d) Pregnancy Test (when applicable). (e) Patient Questionnaires (SF-36, Multi-Dimensional Fatigue Inventory, Specific Symptom Severity and Long Covid Symptom Questionnaire (for Long Covid patients only)).

Every 16 weeks: (a) Chemistry Panel. (b) Urinalysis. (c) Coagulation Panel. (d) Hematology Panel. (e) C Reactive Protein. (f) Sera for Frozen Storage. (g) KPS. (h) Pregnancy Test (when applicable).

Every 20 weeks: (a) Pregnancy test (when applicable).

Every 24 weeks: (a) Physical Examination (including Investigator Assessment of Signsand Symptoms). (b) Weight. (c) Chemistry Panel. (d) Urinalysis. (e) Coagulation Panel. (f) Hematology Panel. (g) Thyroid Function Panel. (h) C Reactive Protein. (i) Sera for Frozen Storage. (j) Direct Coombs Assay (Direct Anti-globin assay). (k) Pregnancy test (when applicable). (l) KPS. (m) EKG. (n) Patient Questionnaires (SF-36, Multi-Dimensional Fatigue Inventory, and Long Covid Symptom Questionnaire (for Long Covid patients only)).

Post week 24, every week thereafter: (a) COVID-19 Symptoms: Patients who develop symptoms of COVID-19 willbe tested and if positive for presence of SARS-CoV-2 virus, will be discontinued from treatment and should be followed until resolution of COVID-19 symptoms even with a negative test for SARS-CoV-2 virus. Thesafety follow-up of COVID-19 symptoms will occur on a weekly basis for thefirst month and then monthly thereafter until resolution of symptoms. The status of COVID-19 symptoms may be obtained via a telephone contact. (b) Long Covid Questionnaire (for Long Covid patients only). (c) Study drug administration twice (2 times) weekly.

Post week 24, every 4 weeks thereafter: (a) Pregnancy test (when applicable).

Post week 24, every 8 weeks thereafter: (a) Chemistry Panel (If there is evidence of toxicity the frequency will remain atevery 8 weeks until there is a 24-week period that there is no toxicity.). (b) Urinalysis (If there is evidence of toxicity the frequency will remain at every8 weeks until there is a 24-week period that there is no toxicity.). (c) Coagulation Panel (If there is evidence of toxicity the frequency will remain at every 8 weeks until there is a 24-week period that there is no toxicity.). (d) Hematology Panel (If there is evidence of toxicity the frequency will remainat every 8 weeks until there is a 24-week period that there is no toxicity.).

Post week 24, every 12 weeks thereafter: (a) Physical Examination (including Investigator Assessment of Signs andSymptoms). (b) Weight. (c) EKG. (d) KPS. (e) Chemistry Panel (If no evidence of laboratory toxicity is observed in the 24week period, the frequency of testing will be reduced to every 12 weeks.). (f) Urinalysis (If no evidence of laboratory toxicity is observed in the 24 weekperiod, the frequency of testing will be reduced to every 12 weeks.). (g) Coagulation Panel (If no evidence of laboratory toxicity is observed in the 24week period, the frequency of testing will be reduced to every 12 weeks.). (h) Hematology Panel (If no evidence of laboratory toxicity is observed in the 24week period, the frequency of testing will be reduced to every 12 weeks.). (i) C Reactive Protein. (j) Sera for frozen storage. (k) Patient Questionnaires (SF-36, Multi-Dimensional Fatigue Inventory andSpecific Symptom Severity).

Post week 24, every 24 weeks thereafter: (a) Direct Coombs Assay. (b) Thyroid Function Panel.

Post week 24, at 120 Weeks: (a) Chest x-ray (PA and lateral).

At the termination of study: (a) Physical Examination (including Investigator Assessment of Signsand Symptoms). (b) Weight. (c) Chemistry Panel. (d) Urinalysis. (e) Coagulation Panel. (f) Hematology Panel. (g) Thyroid Function Panel. (h) C Reactive Protein. (i) Sera for Frozen Storage. (j) Direct Coombs Assay (Direct Anti-globin assay). (k) Pregnancy test (when applicable). (l) KPS. (m) EKG. (n) Chest x-ray (PA and lateral) (If time on study is less than 3 months, termination chest x-ray is not required). (o) COVID-19 Symptoms. (p) Patient Questionnaires (SF-36, Multi-Dimensional Fatigue Inventory,Specific Symptom Severity and Long Covid Symptom Questionnaire (for Long Covid patients only)).

A schematic of testing and assessments is displayed in Table B. All assessments, examinations, and procedures will have a +/- 1 week window to allow the patient adequate timeto have the items completed.

Patients who fail to comply with the requirements of the study will be withdrawn. Reasons for withdrawals may include but are not limited to the following: (1) Lost to Follow-up. (2) Voluntary patient withdrawal/withdrawal by patient or guardian. (3) Protocol violation, such as failure to comply with the requirements of the study (i.e., failure to return for visits) or use of prohibited concomitant medications. (4) Physician decision, such as significant intercurrent illness or surgery, as determined by the Investigator which prevents the patient from taking the study medication, or which requires administration of drugs disallowed in this study. (5) Adverse event, such as toxicity grading of 3 or 4 or serious adverse event (SAE) felt by the investigator to be related to the study medication (see Appendix A). (6) Lack of efficacy. (7) Recovery.

Subjects who discontinue (voluntarily or involuntarily) from study participation prior to completing the first 24 weeks of treatment are considered premature discontinuations. If the subject discontinues any time after the initial 24 weeks of treatment, the subject will be considered to have completed the study. If a subject discontinues at the 24 week mark, they will be considered completers.

Statistical evaluation a the endpoint will be performed for (1) Karnofsky Performance Score (KPS). (2) SF-36, Health Questionnaire. (3) Specific Symptom Severity. (4) Multi-Dimensional Fatigue Inventory. (5) COVID-19 Symptoms. (6) Long Covid Questionnaire (for Long Covid patients only).

We will analyze the median KPS changes between progressive eight (8) and/or 12 week intervalsand baseline using the Wilcoxon signed-rank test. An ANOVA analysis with baseline as a covariate will also be performed. In addition, a distribution analysis will be utilized to evaluatedifferences between baseline and treatment, using the Cochran-Armitage test.

Analysis of changes in SF-36. Specific Symptom Severity and Multi-Dimensional Fatigue Inventory between treatment intervals and baseline will utilize both parametric (t-test and ANOVA with baseline as a covariate) and non-parametric (Wilcoxon signed-rank test) approaches. Frequency distribution will utilize the Cochran Armitage trend test.

The patients who enter this test after having previously tested positive for SARS-CoV-2 and having Long Covid at study entry will be analyzed separately. Since many patients with mild or no COVID-19 symptoms were not tested for the presence of SARS-CoV-2, many patients with PCCF also called "Long Haulers," will not have a history of a positive SARS-CoV-2 test result. A positive serum antibody test for SARS-CoV-2 will be sufficient in these cases.

**Table 1: Toxicity Criteria**

| | | **0** | **1** | **2** | **3** | **4** | |
|---|---|---|---|---|---|---|---|
| Leukopenia | WBC x 10¹. | ≥4.5. | 3.0-<4.5. | 2.0-<3.0. | 1.0-<2.0. | <1.0. | |
| | NEUT x 10³ | ≥1.9 | 1.5-<1.9 | 1.0-<1.5 | 0.5-<1.0 | <0.5 | |
| Thrombocytope nia | Plt x 10³ | >130 | 90-<130 | 50-<90 | 25-<50 | <25 | |
| Anemia | Hgb gm %. | ≥11. | 9.5-10.9. | <9.5. | · | | |
| | Hct % Clinical | ≥32 | 28-31.9 | <28. | Require transfusions | | |
| | | | | Symptoms of anemia | | | |
| Hemorrhage | | None | Minimal | Moderate-not debilitating | Debilitating | Life Threatening | |
| Infection | | None | No active treatment | Requires active treatment | Debilitating | Life Threatening | |
| GU* | BUN mg %. | ≤20. | 21-40. | 41-60. | >60. | Symptomatic Uremia. | |
| | Creatinine | ≤1.2. | 1.3-2.0. | 2.1-4.0. | >4.0. | With obst uropathy | |
| | Protein. Hematuria | Negati ve | 1+. | 2+-3+. | 4+. | | |
| | | | Micro-Cult-positive | Gross-Cult positive | Gross plus Clots | | |
| | | Negati ve | | | | | |
| Hepatic** | SGOT. | <1.5 x nl. | 1.5-2 x normal. | 2.1-5 x normal. | >5 x normal. | · | |
| | Alk Phos Bilirubin. | <1.5 x nl. | 1.5-2 x normal. | 2.1-5 x normal. | >5 x normal. | | |
| | | <1.5 x nl | 1.5-2 x normal | 2.1-5 x normal | >5 x normal | | |
| | Clinical | | | | | | |
| | | | | | Pre-coma | Hepatic coma | |
| N&V | | None | Nausea | Nausea and Vomiting. Controllable | Vomiting Intractable | | |
| Diarrhea | | None | No dehydration | Dehydration | Grossly bloody | | |
| Pulmonary*** | Clinical | Normal | Mild or transient symptoms. with <25% decrease in DCO or VC (if measured) | Moderate symptoms with 25-. 50% decrease in DCO or VC (ifmeasured) | Severe Sx-Intermittent O2 or. >50% decrease in DCO or VC | Assisted vent or continuous 02 | |
| Cardiac | | NI. | ST-T changes. | Atrial arrhythmias. | Mild CHF. | Severe or refractory | |
| | | NI | Sinus tachycardia. | Unifocal PVC's | Multifocal | Congestive Heart Failure. | |
| | | | > 110 at rest | | PVC's Pericarditis | Ventricul ar tachycard ia Tampona de | |
| Neuro | PN | None | Decreased DTR' s. | Absent DTR's. | Disabling sensory loss. | Respiratory dysfunction secondary to weakness. | |
| | | | Mild paresthesias | Severe paresthesias | Severe PN pain | | |
| | | | | Severe Constipation. | Obstipation Severe weakness. | Obstipation requiring surgery. | |
| | | | Mild Constipation | Mild weakness | | Paralysis - confining pt to bed/wheelchair | |
| | | | | | Bladder dysfunction | | |
| Neuro | CNS | None | Mild anxiety. | Severe anxiety. | Confused or manic. | Seizures. | |
| | | | Mild depression | Moderate depression | Severe depression | Suicidal Coma | |
| | | | Mild headache | Moderate headache | Severe headache | | |
| | | | Lethargy | Somnolence. | Cord dysfunction | | |
| | | | | Tremor. | Confined to bed due to. CNS dysfunction | | |
| | | | | Mild hyperactivity | | | |
| Coagulation | PT. | within normal limits. | 1.01-1.25 x normal. | 1.26-1.50 x normal. | 1.51-2.00 x normal. | > 2.00 x normal. | |
| | PTT | | 1.01-1.66 x normal | 1.67-2.33 x normal | 2.34-3.00 x normal | > 3.00 x normal | |
| | | within normal limits | | | | | |
| Skin & Mucosa | | NI | Transient erythema. | Vesiculation | Ulceration. | | |
| | | | Pigmentation atrophy | Subepider mal. fibrosis | Neurosis | | |
| Oral | Stomatitis | None | Soreness | Ulcers - can eat | Ulcers - cannot eat | | |
| Alopecia | | None | Alopecia-mild | Alopecia-severe | | | |
| Allergy | | None | Transient rash. | Urticaria. | Serum sickness. | Anaphylaxis | |
| | | | Drug Fever (≤38°C, ≤100.4°F) | Drug fever >38°C (>100.4°F)Mild bronchospasm | Bronchospasm - requiringparenteral medications | | |
| Fever**** | | ≤38°C | ≤39°C | >39°C | >40.3°C | Fever induced hypotension | |
| Local Toxicities | | None | Pain | Pain- Phlebitis | Ulceration | | |

| | | | | | | | |
|---|---|---|---|---|---|---|---|
| 1. The toxicity grade should reveal the most severe degree occurring during the evaluation period not an average. 2. When two criteria are available (or similar toxicities, e.g., leukopenia, neutropenia the more severe toxicity grade should be used).3.Toxicity grade = 5 if that toxicity caused the death of patient. 4.Refer toxicity gradation over to study chairman for toxicity not covered on this table. *Urinary tract infection should be graded under infection not GU. Hematuria resulting from thrombocytopenia is graded under hemorrhage. **Viral hepatitis should be recorded as infection rather than liver toxicity. ***Pneumonia is considered infection and not graded as pulmonary toxicity unless felt to be resultant from changes directly induced bytreatment. ****Fever felt to be caused by drug allergy should be graded as allergy. Fever due to infection is graded under infection only. | | | | | | | |

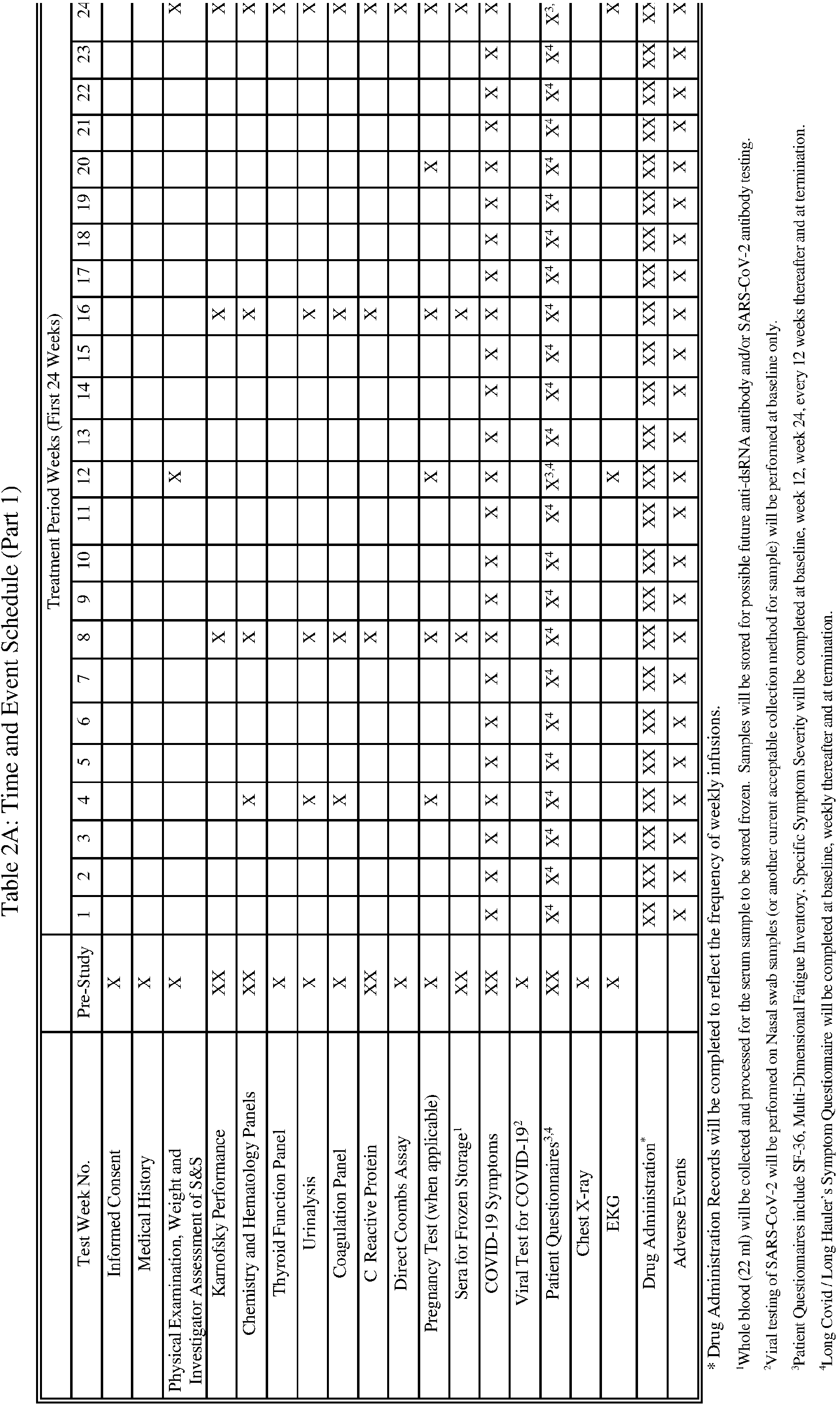

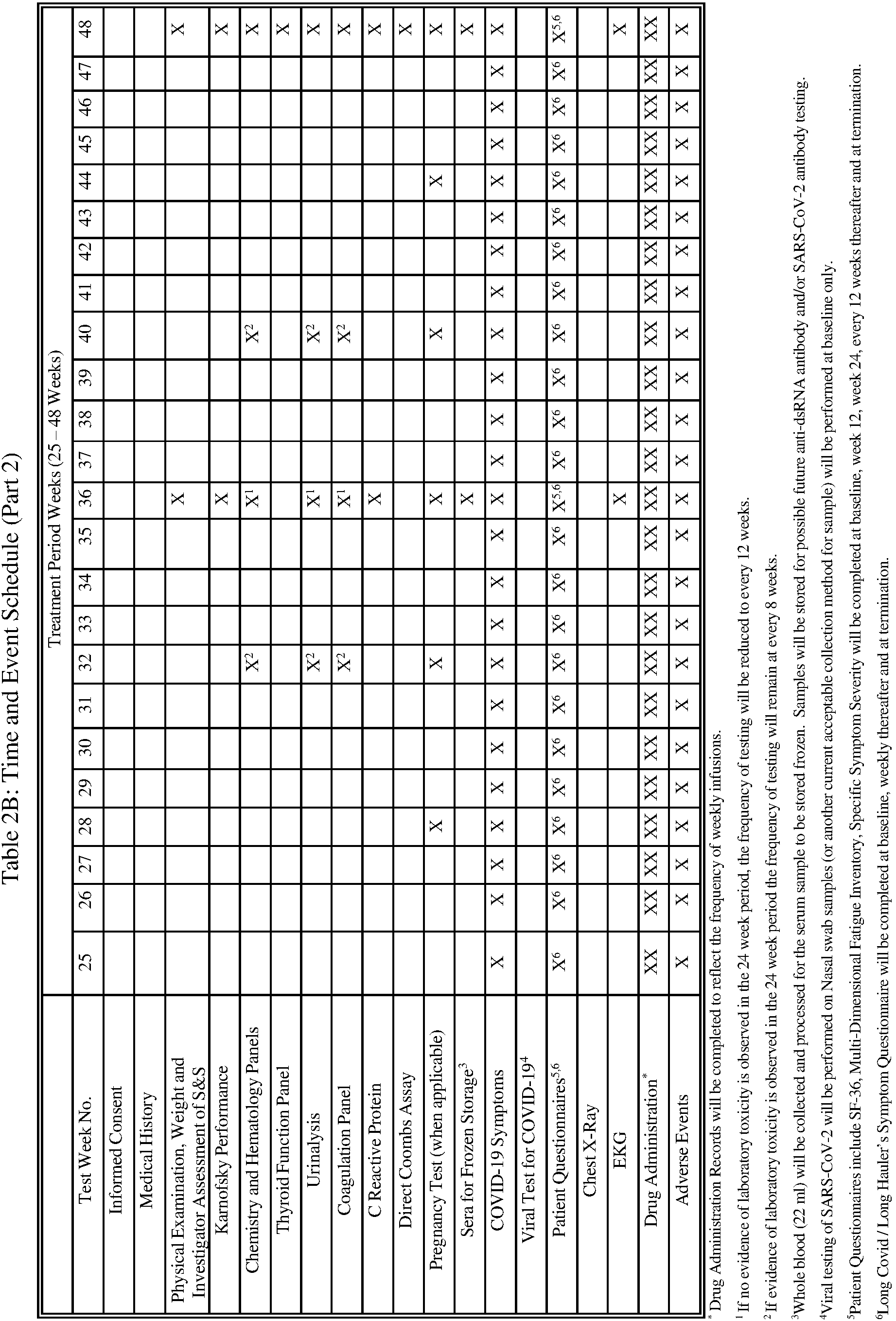

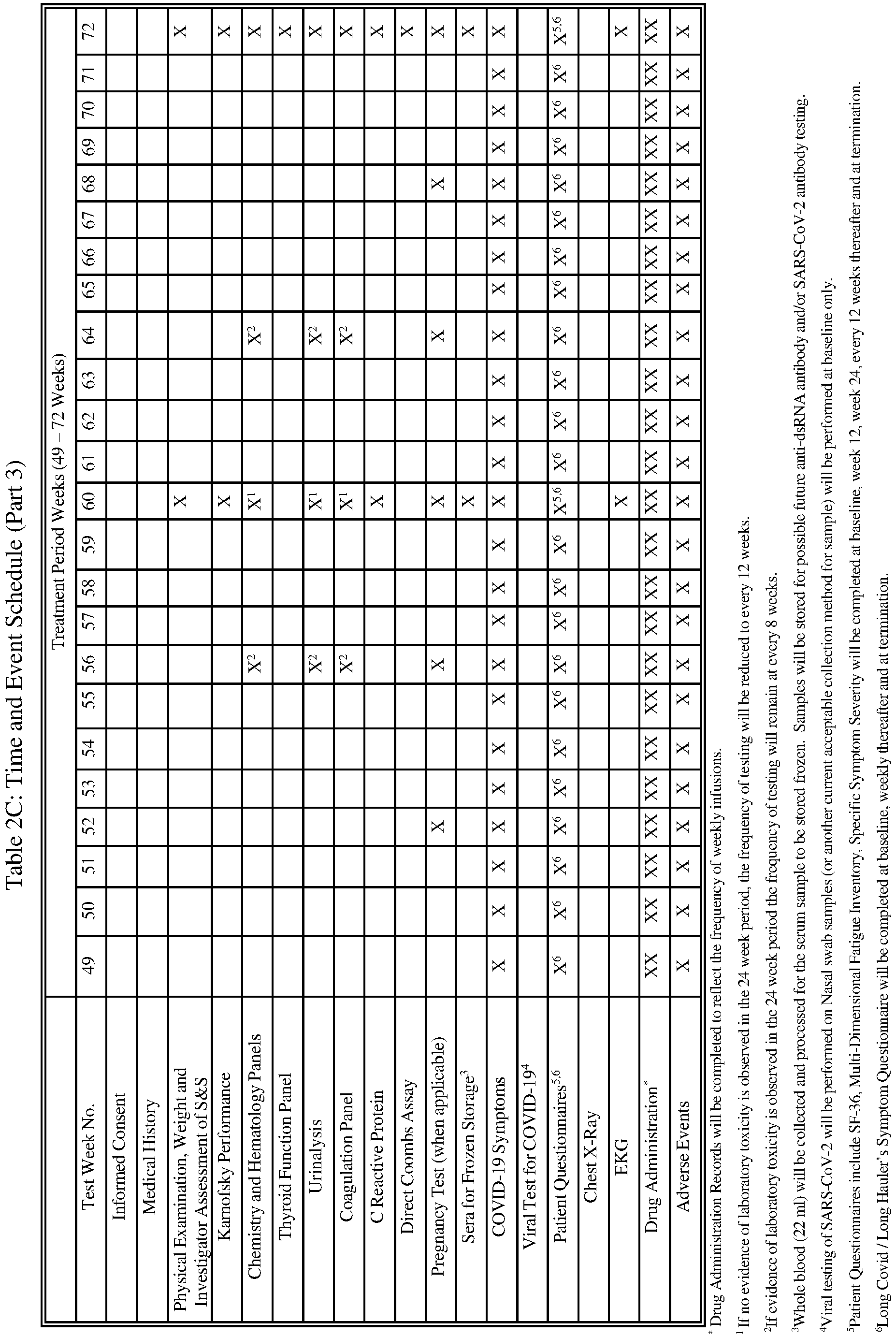

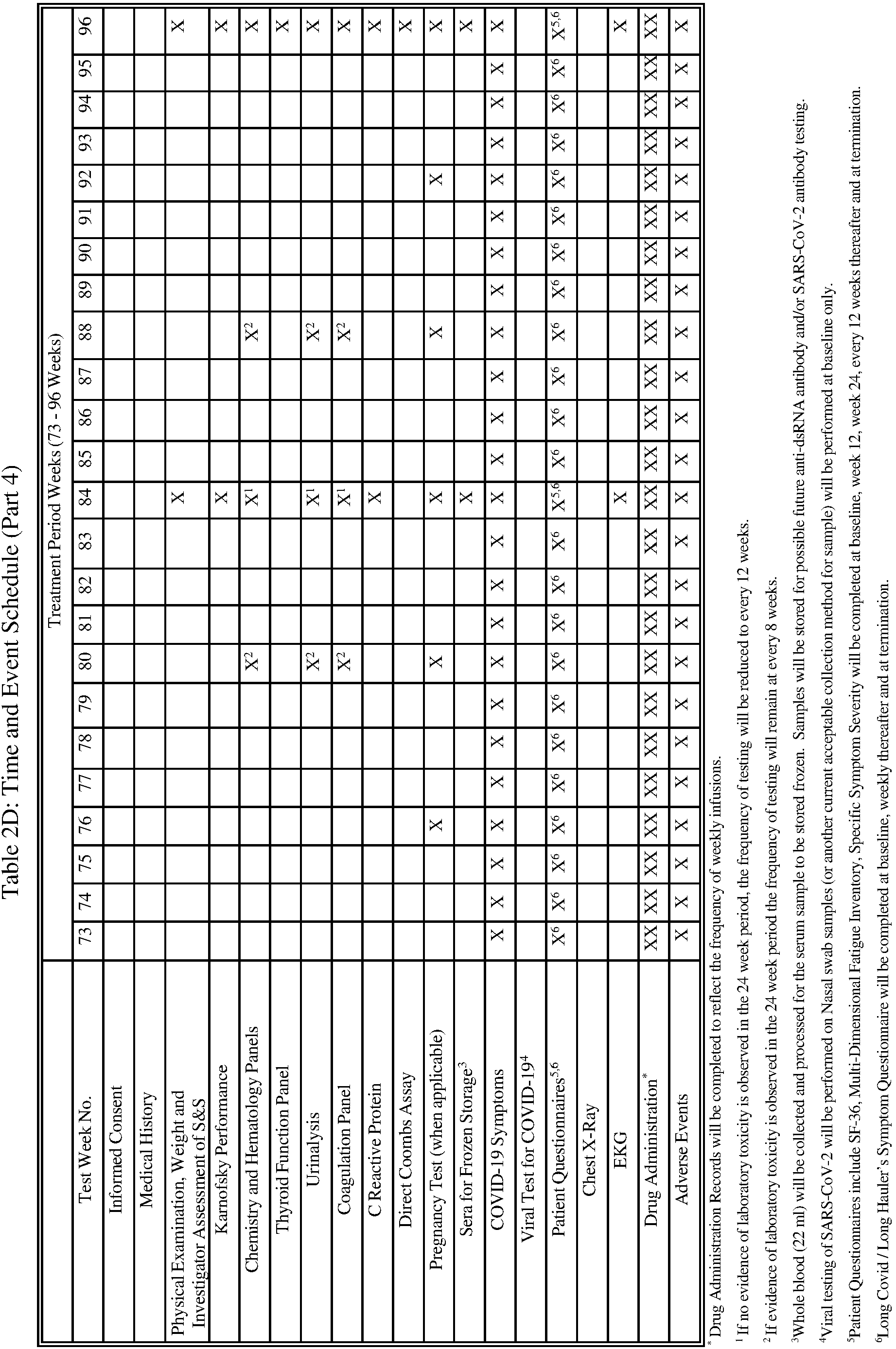

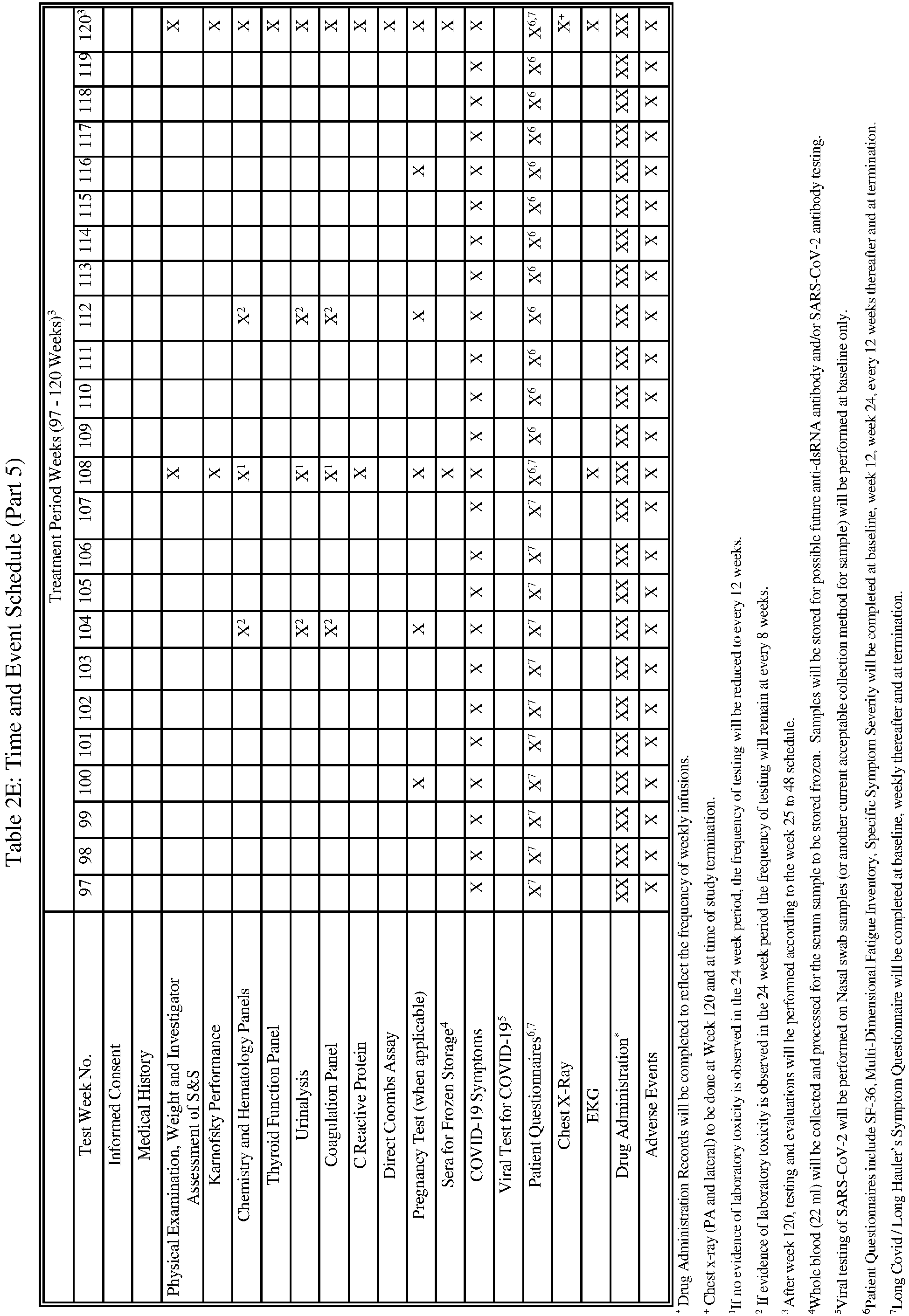

### 1988 CDC case definition of chronic fatigue syndrome (CFS):

This definition is intended to serve as the basis for epidemiologic and clinical studies of chronic fatigue syndrome. Although it may be a useful guide for the evaluation of a patient with a suggestive illness, the definition remains sufficiently nonspecific that it cannot confirm or deny the diagnosis of chronic fatigue syndrome in an individual patient. Chronic fatigue syndrome remains a diagnosis of exclusion, and physicians must continue to maintain a high level of suspicion throughout the course of the illness that other, more occult conditions may be causing the symptoms. A case of CFS must fulfill.

Major criteria 1 and 2, and the following minor criteria -- > 6 of the 11 symptom criteria PLUS > 2 of the 3 physical criteria; OR 8 of the 11 symptoms criteria.

### MAJOR CRITERIA:

(1) Acute or subacute onset of persistent or relapsing, debilitating fatigue or easy fatigability in a person who has no previous history of similar symptoms, that does not improve with rest and that is severe enough to reduce or impair average daily activity below 50% of the patient's premorbid activity level for a period of at least six (6) months.
(2) Reasonable exclusion of other clinical conditions that may produce similar symptoms, based upon history, physical examination, and appropriate laboratory findings: 1) malignancy; 2) autoimmune disease; 3) localized infections (such as occult abscess), 4) chronic or subacute bacterial diseases (such as endocarditis, Lyme disease, or tuberculosis), fungal diseases (such as histoplasmosis, blastomycosis, or coccidiomycosis), and parasitic diseases (such as toxoplasmosis, amebiasis, giardiasis, or helminthic infestations); 5) AIDS or AIDS-related complex (ARC); 6) chronic inflammatory diseases (such as sarcoidosis, Wegener's granulomatosis or chronic hepatitis); 7) neuromuscular diseases (such as multiple sclerosis or myasthenia gravis); 8) endocrine diseases (such as hypothyroidism, Addison's disease, Cushing's syndrome, or diabetes mellitus); 9) side effects of chronic medications or other toxic agents (such as chemical solvents or heavy metals); 10) other chronic pulmonary, cardiac, gastrointestinal, hepatic, renal, hematologic, neurological, musculoskeletal diseases; or 11) depression preceding onset of CFS. A recommended minimum laboratory evaluation for other possible causes should include the following: 1) complete blood count, differential, and platelet count; 2) erythrocyte sedimentation rate; 3) antinuclear antibody; 4) serum electrolytes; 5) glucose; 6) creatinine blood urea nitrogen; 7) calcium, phosphorous; 8) total bilirubin, alkaline phosphatase, SGOT (AST), SGPT (ALT); 9) thyroid-stimulating hormone level; 10) urinalysis; and 11) intermediate strength PPD.

### MINOR CRITERIA -SYMPTOM CRITERIA

To fulfill a symptom criteria, a symptom must have begun at or after the time of onset of increased fatigability, and must have persisted or recurred over a period of at least 6 months (the individual symptoms may or may not have occurred simultaneously). These symptoms include: (1) Mild fever (oral temperature between 37.4 °C (99.4 degrees F) and 38.3 °C (101 degrees F), if measured) and/or chills. Note: oral temperature of > 38.3 °C (101 degrees F) is not commonly associated with CFS and should prompt studies for other causes). (2) Sore throat. (3) Painful anterior or posterior cervical or axillary lymph nodes. (4) Unexplained generalized muscle weakness. (5) Prolonged (≥ 24 hours) generalized fatigue following levels of exercise that would have been easily tolerated in the patient's premorbid states. (6) Headaches (of a type, severity, or pattern that is different from headaches suffered in thepatient's premorbid state). (7) Muscle discomfort/myalgias. (8) Migratory arthralgias without joint swelling or redness. (9) Neuropsychological complaints (forgetfulness, excessive irritability, confusion,dizziness, difficulty thinking, inability to concentrate, depression). (10) Sleep disturbances (hypersomnia, insomnia, difficulty falling asleep, early morning awakening). (11) Description of the main symptom complex as initially developing over a few hours to afew days.

Physical Criteria which are documented in a physician's office on at least two (2) occasions, at least one month apart include: (1) Low-grade fever (oral temperature between 37.5 °C (99.5 degrees F) and 38.3 °C (101 degrees F), or rectal temperature 37.8 °C (100 degrees F) and 38.6 °C (101.5 degrees F)). (2) Non-exudative pharyngitis. (3) Palpable and/or tender anterior or posterior cervical or axillary lymphadenopathy (Note:lymph nodes > 2 cm in diameter suggest other etiologies. Further evaluation is warranted.).

### 1994 CASE DEFINITION FOR CHRONIC FATIGUE SYNDROME (CFS).

Inclusion criteria include: (1) Unexplained persistent or relapsing chronic fatigue that is of new or definite onset (i.e., not lifelong), is not the result of ongoing exertion, is not substantially alleviated by rest, and results insubstantial reduction in previous levels of occupational, educational, social, or personal activities. (2) The concurrent occurrence of four or more of the following symptoms, all of which must have persisted or recurred during 6 or more consecutive months of illness and must not have predated thefatigue. (A) Substantial impairment in short-term memory or concentration1 sore throat. (B) tender cervical or axillary lymph nodes muscle pain. (C) Multi-joint pain without swelling or redness headaches of a new type, pattern, or severity2 unrefreshing sleep. (D) post-exertional malaise lasting more than 24 hours.

Exclusion criteria include: (1) Any active medical condition that may explain the presence of chronic fatigue, such as untreatedhypothyroidism, sleep apnea and narcolepsy, and iatrogenic conditions such as side effects of medication. (2) Any previously diagnosed medical condition whose clinical doubt and whose continued activity may explain the chronic fatiguing illness. Such conditions may include previously treated malignancies and unresolved cases of hepatitis B or C virus infection. (3) Any past or current diagnosis of a major depressive disorder with: (3a) bipolar affective disorders. (3b) schizophrenia of any subtype. (3c) delusional disorders of any subtype. (3d) dementias of any subtype. (3e) anorexia nervosa. (3f) bulemia nervosa. (4) Alcohol or other substance abuse within 2 years before the onset of the chronic fatigue and at anytime afterward. (5) Severe obesity as defined by a body mass index equal to or greater than 45.

### KARNOFSKY PERFORMANCE SCALE AND PROCEDURES

### General Procedures for Determining Karnofsky Performance Scores (KPS).

The KPS is a global evaluation of the patient's ability to conduct daily activities, including work activities and self-care activities. The KPS is sensitive to effective therapeutic intervention in chronic disease states. Following two (2) assessments at baseline, a KPS is obtained at week 8, week 16, week 24 and every 12 weeks thereafter during the study, based upon a questionnaire (attached) completed by the patient and an interview which includes discussion of specific signs and symptoms, basic functional accomplishments (e.g., daily care activities), changes in activities and changes in medications taken. In addition, the assignment of the score may include discussions with a significant other (i.e., spouse, companion, or custodian needed to care for patient's daily needs). The KPS score will be assigned by the principal investigator or in (his/her) absence by only one additional designated individual at each site.

### Karnofsky Performance Scale is assigned as follows:

100 Normal activity; no complaints; no evidence of disease.
90 Able to carry on normal activity; minor signs or symptoms of disease.
80 Normal activity with effort; some signs and symptoms of disease.
70 Cares for self, unable to carry on normal activity or do active work.
60 Requires occasional assistance but is able to care for most of needs.
50 Requires considerable assistance for daily care.
40 Disabled; unable to care for self, requires special care and assistance.
30 Severely disabled, bedridden although death is not imminent.
20 Very sick; hospitalization and/or nursing care is necessary; active supportive treatment is necessary.
10 Moribund; fatal processes progressing rapidly.
0 Dead.

### DEFINITION OF LONG COVID / also called "LONG HAULERS".

1. Patients must have a diagnosis of COVID-19 by a laboratory-confirmed positive for SARS-CoV-2 (COVID-19), OR, a positive serum antibody test for SARS-CoV-2.
2. Patients must meet the Diagnosis of Chronic Fatigue Syndrome (CFS) as defined by the1988 CDC case definition for CFS OR as defined by the 1994 CDC case definition for CFS except for the duration of the fatiguing illness which must have continued for at least 3 months and must not have preceded the onset of the COVID-19 symptoms.
3. The concurrent occurrence of at least one or more of the following Long Hauler symptoms, allof which must have persisted or recurred during 3 or more consecutive months of illness andmust not have preceded the onset of the COVID-19 symptoms.

Long Hauler Symptoms, at least for this study, include: (1) Fever or chills. (2) Cough. (3) Shortness of breath or difficulty breathing. (4) New loss of taste or smell. (5) Chest pain.

A diagnosis of COVID-19 would not exclude the patient from meeting the CDC case definitions for CFS. Since many patients with mild or no COVID-19 symptoms were not tested for the presence of SARS-CoV-2, many patients with Long Covid also called "Long Haulers" will not have a history of a positive SARS-CoV-2 test result. A positive serum antibody test for SARS-CoV-2 will be sufficient in these cases.

### Results:

Our studies are ongoing and the first dose of tdsRNA in the form of AMPLIGEN^{®} was administered at least in January 2021. Our initial findings after 12 weeks are shown below in Table 3 as well as in Figure 1. In Table 3, "PCCF Symptoms" is the same as "Long Covid Symptoms." Long Covid Questionnaire Severity is as follows: 0 is none. 1 to 3 is considered mild. 4 to 6 is considered moderate and 7 to 10 is considered severe. Baseline questions were established with values greater than or equal to =2. The data is based on patient study data summary. The first dose was administered on January 4, 2021 and the current dosage is 400 mg administered twice weekly. The initial dose was 200 mg per dose administered twice weekly (400 mg/subject/week) on week 1 and 2. The dose escalated to 400 mg per dose administered twice weekly (800 mg/subject/week) from week 3, and the current dosage, 12 weeks into the study, is 400 mg per dose administered twice weekly (800 mg/subject/week).

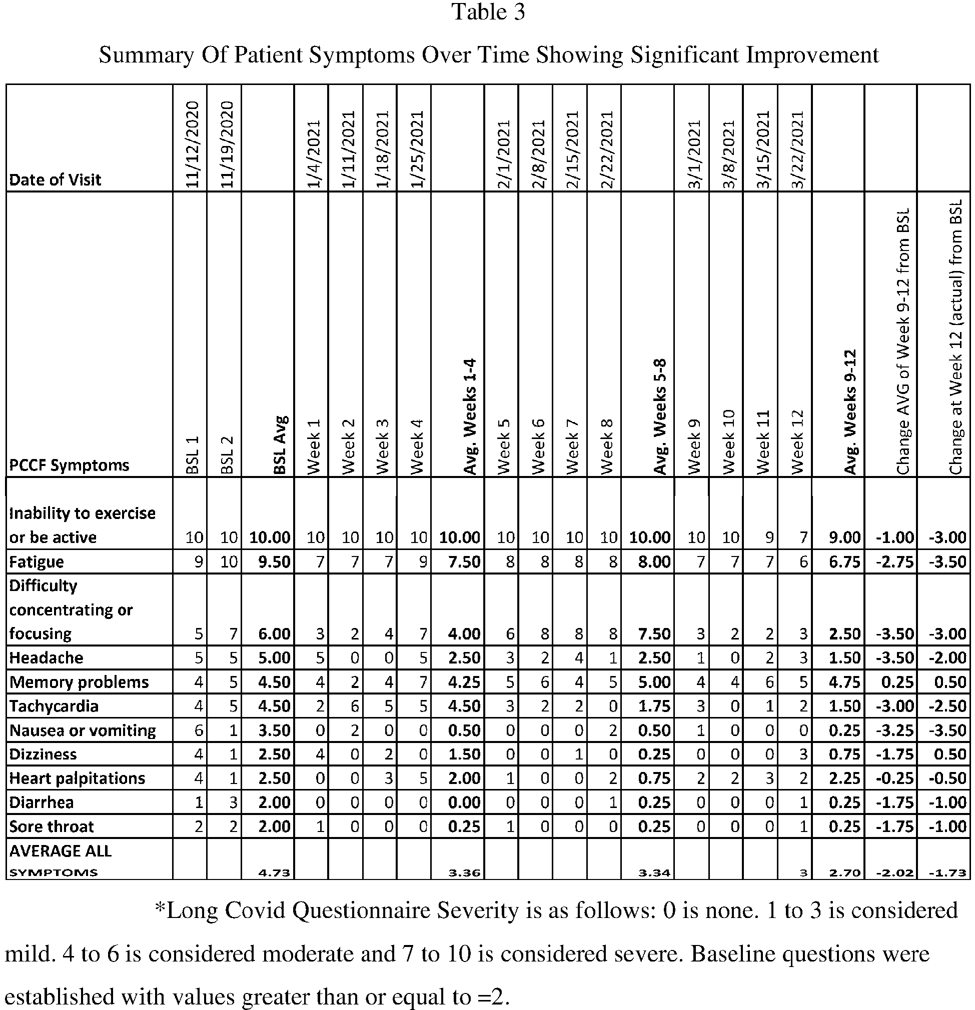

- Statistical analysis was performed on the data above and this analysis is shown in Table 4 below.

**Table 4**

| Statistical Analysis of Symptom Improvement at Week 12 |
|---|
| XLSTAT 2020.5.1.1078 - Comparison of two samples (Wilcoxon, Mann-Whitney, ...) - Start time: 03/31/2021 at 13:48:38 / End time: 03/31/2021 at 13:48:39 |
| Sample 1: Workbook = Patient SNV-288 AMP-511 Study Data 032521.xlsx / Sheet = PCCF Questionnaire sort / Range = 'PCCF Questionnaire sort'!$D$7:$D$18 / 11 rows and 1 column |
| Sample 2: Workbook = Patient SNV-288 AMP-511 Study Data 032521.xlsx / Sheet = PCCF Questionnaire sort / Range = 'PCCF Questionnaire sort'l$s$7:$s$18 / 11 rows and 1 column |
| Hypothesized difference (D): 0 |
| Significance level (%): 5 |
| p-value: Asymptotic p-value |
| Continuity correction: Yes |

### PCCF Questionnaire Baseline to [AVERAGE] Week 12 Value (Patient SNV-288)

| Summary statistics: | 11 Symptoms with Baseline value >/=2 | | | | | | |
|---|---|---|---|---|---|---|---|
| Variable | Observations | Obs. with missing data | Obs. without missing data | Minimum | Maximum | Mean | Std. deviation |
| BSL Avg | 11 | 0 | 11 | 2.000 | 10.000 | 4.727 | 2..805 |
| Avg. Weeks 9-12 | 11 | 0 | 11 | 0.250 | 9.000 | 2.705 | 2.919 |

### Mann-Whitney test / Two-tailed test:

| | |
|---|---|
| U | 91 |
| U (standardized) | 1.976 |
| Expected value | 60.500 |
| Variance (U) | 230.476 |
| p-value (Two-tailed) | 0.048 |
| alpha | 0.050 |

An approximation has been used to compute the p-value.

The continuity correction has been applied.

### Test interpretation:

H0: The difference of location between the samples is equal to β.
Ha: The difference of location between the samples is different from 0.

As the computed p-value is lower than the significance level alpha=0.05, one should reject the null hypothesis H0, and accept the alternative hypothesis Ha.

Ties have been detected in the data and the appropriate corrections have been applied.

Based on P-value (Two-tailed) of less than 0.05 for all 11 symptoms, that the results show a significant reduction in Long Covid symptoms in a Long Covid patient after a 12-week treatment.

The results of some individual symptoms are very dramatic. As can be seen in the data, by week 12, there was a significant decrease in the adverse symptoms of Long Covid. Comparing BSL average from before treatment to the values at 12 weeks, we see some significant improvements. For example, inability to exercise or be active was reduced from 10 to 7; fatigue was reduced from 9.5 to 6; difficulty concentrating or focusing was reduced from 6 to 3; headache was reduced from 5 to 3; tachycardia was reduced from 4.5 to 2; nausea or vomiting was reduced from 3.5 to 0; diarrhea was reduced from 2 to 1; and sore throat was reduced from 2 to 1. The average of all symptoms was reduced from 4.73 to 3. This data clearly show that administration of tdsRNA has a beneficial effect on reducing at least one Long Covid symptom in a Long Covid patient.

### Example 5 Nasal Administration of tdsRNA

Ampligen^{®} is a well-defined selective Toll-like receptor 3 (TLR3) agonist inducing innate immune antiviral responses. Ampligen has been administered intravenously in approximately 100,000 doses in clinical trials and compassionate use programs. Besides, intranasal administration of Ampligen as a universal flu adjuvant was found to be well tolerated.

TLR3 is expressed at a high level in human airway epithelial cells, including the nose and nasal pharynx. TLR3 serves as a pathogen recognition receptor to stimulate the innate immune response against many respiratory pathogens, including coronaviruses. As a highly specific TLR3 agonist, Ampligen stimulates the production of type I interferons, which exert both antiviral and immunomodulatory activity.

The route of human infection of SARS-CoV-2 is believed to be primarily by entry into the nasal epithelium. By dosing Ampligen every other day intranasally, it is believed that SARS-CoV-2 can be inhibited at the point of entry, and thus will be much less likely to progress to a pulmonary infection, or moderate COVID-19 disease. These characteristics make Ampligen a potent candidate to be developed for an early treatment strategy and (post-exposure) prophylaxis against COVID-19. Because Ampligen does not act by binding to proteins or specific nucleic acid sequences of viruses it can also be developed for potential future outbreaks with pathogenic coronaviruses (CoVs) or other respiratory viruses.

A phase I trial is underway to assess the safety, tolerability and biological activity of repeated administration of Ampligen intranasally every other day for 13 days (7 doses) in healthy volunteers. This study was performed for the further development of Ampligen as a potential treatment modality for COVID-19 and other pulmonary viral diseases.

Inclusion criteria were: 1. Signed informed consent prior to any study-mandated procedure. 2. Male or female subjects, 18 to 70 years of age, inclusive at screening. 3. Body mass index (BMI) between 18 and 32 kg/m2, inclusive at screening, and with a minimum weight of 50 kg. 4. Participant must be healthy, in the investigator's clinical judgment, as confirmed by medical history, physical examination, vital signs, ECG and laboratory assessments performed at screening. 5. Willing to comply with effective contraception during the study if subject is male or women of childbearing potential, up to 90 days after the last dose of study treatment. 6. Has the ability to communicate well with the investigator and willing to comply with the study restrictions.

Exclusion criteria were: 1. Evidence of any active or chronic disease or condition that could interfere with, or for which the treatment of might interfere with, the conduct of the study, or that would pose an unacceptable risk to the subject in the opinion of the investigator. 2. Clinically significant abnormalities, as judged by the investigator, in laboratory test results (including hepatic and renal panels, complete blood count, chemistry panel and urinalysis). 3. Positive hepatitis B surface antigen (HBsAg), hepatitis C antibody (HCV ab), or human immunodeficiency virus antibody (HIV ab) at screening. 4. Respiratory tract infection (including flu and common cold symptoms) or any febrile illness (>38°celsius) in the period of 3 days before first treatment administration. 5. Presence of respiratory viral infection as determined by respiratory panel on nasal swab at baseline (including positive SARS-CoV-2 PCR test). 6. History of chronic respiratory diseases (e.g., chronic obstructive pulmonary disease, emphysema, chronic rhinitis or sinusitis, asthma or other reactive airway diseases) in adulthood. Childhood asthma and non-active allergic rhinitis (including hay fever) will be permitted at the discretion of the investigator. 7. History of frequent nose bleeds. 8. Significant anatomical nasal abnormalities or other nasal abnormalities that might impact the study executions (including, but not limited to, nasal septal defects, cleft palate, nasal polyps, previous nasal cautery or surgery that impacts study assessments). 9. Immunocompromised (known or expected immune deficiency, disease, or use of medication that may affect the immune system) or evidence of autoimmune disorder (deemed clinically relevant by the investigator). 10. Participation in an investigational drug or device study (last dosing of previous study was within 90 days or 5 half-lives prior to first dosing of this study). 11. History of abuse of addictive substances (alcohol, illegal substances) or current use of more than 21 units of alcohol per week, drug abuse, or regular user of sedatives, hypnotics, tranquilizers, or any other addictive agent. 12. Positive test for drugs of abuse at screening or pre-dose. Drugs test may be repeated. 13. A routine smoker of tobacco products, currently or in the past year. No (incidental) smoking will be allowed in the two weeks prior to first dosing. 14. Use of immunomodulatory drug, including systemic corticosteroids as well as nasal preparations within 30 days before first dosing. 15. Receipt of any vaccine within 1 week prior to IMP administration, or planning to get vaccinated during the study. 16. Therapy with interferons, interleukins, or other cytokines within 6 weeks of first dosing. 17. Known hypersensitivity to Ampligen or its excipients. 18. If a woman, pregnant, or breastfeeding, or planning to become pregnant during the study. 19. Any known factor, condition, or disease that might interfere with treatment compliance, study conduct or interpretation of the results such as drug or alcohol dependence or psychiatric disease. 20. History of Bell's Palsy or other forms of facial paralysis. 21. Loss or donation of blood over 500 mL within three months (males) or four months (females) prior to screening, or donation of plasma within 14 days of screening or intention to donate blood or blood products during the study.

By dosing tdsRNA in the form of Ampligen^{®} every other day intranasally, it is believed that SARS-CoV-2 can be inhibited at the point of entry, and thus will be much less likely to progress to a pulmonary infection, or moderate COVID-19 disease. Also, this experiment will determine the efficacy of intranasal administration of tdsRNA.

Our findings at the halfway point are that there are no adverse events with the described dosage, and the administration of tdsRNA is well tolerated by all participants.

## Claims

1. Composition comprising therapeutic double-stranded RNA tdsRNA for use in treating Long COVID in a subject previously infected with a virus, wherein the virus is SARS-CoV-2, the treatment comprising administering to the subject a therapeutically effective amount of a composition comprising therapeutic double-stranded RNA tdsRNA,
wherein the tdsRNA is
rIₙ • r(C₁₂U)ₙ, or a combination of rIₙ • r(C₁₂U)ₙ and
rugged dsRNA, wherein the rugged dsRNA is an isolated double-stranded ribonucleic acid (dsRNA) enzymatically active under thermal stress comprising:
each strand with a molecular weight of 250 kDa to 500 kDa, 400-800 basepairs, or 30 to 60 helical turns of duplex RNA,
a single strand comprised of poly(ribocytosinic₄₋₂₉ uracilic acid) and an opposite strand comprised of poly(riboinosinic acid),
wherein the two strands do not base pair the position of the uracil base,
wherein the two strands base pair the position of the cytosine base, and
wherein said strands are partially hybridized,;
wherein n is a number with a value from 40 to 50 000,
wherein rIₙ represents a poly ribo-inosine strand of n bases in length, and
wherein r(C₁₂U)ₙ represents a ssRNA strand of random sequence of C and U bases being n bases in length and a ratio of C bases to U bases in the random sequence ssRNA is about 12.

2. Composition for use according to claim 1, wherein the SARS-CoV-2 is at least one selected from the group consisting of: a B.1.1.7 variant of SARS-CoV-2 (a.k.a. 20I/501Y.V1 Variant of Concern (VOC) 202012/01); a B.1.351 variant of SARS-CoV-2 (a.k.a. 20H/501Y.V2); a P.1 variant of SARS-CoV-2 (a.k.a. 20J/501Y.V3); B.1.427, and B.1.429.

3. Composition for use according to claim 1 or 2,
- wherein the subject exhibits Long COVID symptoms comprising:
(1) at least one symptom selected from the group consisting of:
fatigue including severe fatigue,
inability to exercise or be active because of fatigue, and
low exercise tolerance because of fatigue;
and
(2) at least 4 symptoms selected from the group consisting of:
shortness of breath or difficulty breathing, persistent chest pain or pressure, cough, heart palpitations, diarrhea, partial or complete loss of sense of smell, tachycardia, hair loss, blurry vision, neuropathy in feet and hands, partial or complete loss of sense of taste, nausea or vomiting, clogged ears, dry eyes, tremors or shakiness, floaters or flashes of light in vision, rash, tinnitus or humming in ears, changed sense of taste, dry or peeling skin, phantom smells, costochondritis, low blood oxygen, COVID toes, thrush, dyspnea, phlegm in back of throat, constant thirst, muscle twitching, heat intolerance, abnormally low temperature, cold burning feeling in lungs, goiter or lump in throat, dry scalp or dandruff, anemia, elevated thyroid, sicca syndrome, red eyes, dysgeusia, sputum production, lack of appetite, vertigo, muscle pain, cognitive problems, brain fog, problems with concentration, problems with thinking, chills, sweats, sleep problems, muscle or body aches, difficulty concentrating or focusing, headache, difficulty sleeping, anxiety, memory problems, dizziness, joint pain, sore throat, night sweats, fever or chills, congested or runny nose, sadness, reflux or heartburn, changing symptoms, abdominal pain, lower back pain, shortness of breath or exhaustion from bending over, weight gain, calf cramps, sleeping more than normal, upper back pain, nerve sensations, sharp or sudden chest pain, confusion, feeling irritable, weight loss, post nasal drip, dry throat, high blood pressure, swollen hands or feet, mouth sores or sore tongue, neck muscle pain, hot blood rush, bone aches in extremities, feeling of burning skin, extreme pressure at base of head or occipital nerve, swollen lymph nodes, brain pressure, kidney pain, spikes in blood pressure, hand or wrist pain, bulging veins, mid-back pain at base of ribs, burning sensations, painful scalp, jaw pain, arrhythmia, cracked or dry lips, foot pain, eye stye or infection, low blood pressure, kidney issues or protein in urine, urinary tract infection, hormone imbalances, drastic personality change, gastroesophageal reflux disease with excessive salivation, herpes infection, EBV infection, trigeminal neuralgia, bilateral neck throbbing around lymph nodes, syncope, sadness, chest pain, rhinitis, and myalgia; and/or
- wherein the subject has Long Covid as defined by:
(a) the subject has COVID-19, a previous diagnosed presence of SARS-CoV-2, or a positive serum antibody test for SARS-CoV-2;
(b) the subject meets the Diagnosis of Chronic Fatigue Syndrome (CFS) as defined by the 1988 or the 1994 CDC case definition for CFS for at least 3 months but CFS does not precede the (a);
(c) the subject has concurrent occurrence of at least one or more of Long Covid symptoms which persisted or recurred during 3 or more consecutive months of illness wherein the Long Covid symptoms do not precede (a), wherein the Long Covid symptoms is at least one selected from the group consisting of: fatigue; post-exertional malaise; headache; sleep disturbance; memory problems; problems with concentration; brain fog; fever; chills; cough; shortness of breath; difficulty breathing; loss of taste; loss of smell; and chest pain.

4. Composition for use according to any one of claims 1-3, wherein the subject
is seropositive for an anti-virus antibody;
is seronegative for the anti-virus antibody;
is not infected with the virus during the administering step;
is infected with the virus during the administering step; or
is not hospitalized for the virus infection during the administering step.

5. Composition for use according to any one of claims 1-4, wherein
- the subject did not have Long COVID symptoms before being infected with the virus,
preferably the subject did not have Long COVID symptoms for at least 1 year, 2 years, 3 years, 4 years, or 5 years before being infected with the virus; and/or
- the subject develops the symptoms of Long COVID during or after being infected with the virus.

6. Composition for use according to any one of claims 1-5, wherein the Long COVID symptoms have lasted from infection to the administering step, for a time selected from the group consisting of: 1 month, 2 months, 3 months, 4 months, 5 months, 6 months, 1 year, 2 years, and more than 2 years.

7. Composition for use according to any one of claims 1-6, wherein the subject is a mammal, preferably a host of the virus, and most preferably a human.

8. Composition for use according to any one of claims 1-7,
wherein at least 90 wt.% of the tdsRNA is larger than a size selected from the group consisting of: 40 basepairs; 50 basepairs; 60 basepairs; 70 basepairs; 80 basepairs; and 380 basepairs; or
wherein at least 90 wt.% of the tdsRNA is smaller than a size selected from the group consisting of: 50 000 basepairs; 10 000 basepairs; 9000 basepairs; 8000 basepairs; 7000 basepairs; and 450 basepairs.

9. Composition for use according to any one of claims 1-8, wherein n is a number with a value selected from the group consisting of: 40 to 40 000; 50 to 10 000; 60 to 9000; 70 to 8000; 80 to 7000; and 380 to 450.

10. Composition for use according to any one of claims 1-9,
wherein n is from 40 to 40 000;
wherein the tdsRNA has 4 to 4000 helical turns of duplexed RNA strands; or
wherein the tdsRNA has a molecular weight selected from the group consisting of:
2 kDa to 30 000 kDa;
25 kDa to 2500 kDa; and
250 kDa to 320 kDa.

11. Composition for use according to any one of claims 1-10, wherein the tdsRNA
- comprises 0.1-12 mol % rugged dsRNA, preferably the tdsRNA comprises 0.1-5 mol % rugged dsRNA; and/or
- is complexed with a stabilizing polymer, wherein the stabilizing polymer is preferably at least one selected from the group consisting of:
polylysine; polylysine and carboxymethylcellulose; polyarginine;
polyarginine and carboxymethylcellulose; and a combination thereof.

12. Composition for use according to any one of claims 1-11, wherein the composition comprises at least one pharmaceutically acceptable carrier.

13. Composition for use according to any one of claims 1-12,
- wherein the administration is performed after primary COVID-19 symptoms of acute illness have been resolved,
wherein the COVID-19 symptom of acute illness is preferably selected from the group consisting of: fever, chills, cough, shortness of breath, difficulty breathing, fatigue, muscle aches, body aches, headache, loss of taste, loss of smell, sore throat, congestion, runny nose, nausea, vomiting, diarrhea, and a combination thereof, and/or
- wherein administering is performed after the subject was infected with the virus for a time selected from the group consisting of 30 days, 50 days, 2 months, 3 months, 4 months, 5 months, 6 months, 1 year, 2 years, and more than 2 years; and/or
- wherein administering is performed by a delivery system comprising the tdsRNA,
wherein the delivery system or medical device is preferably at least one selected from the group consisting of: a nebulizer; a sprayer; a nasal pump; a squeeze bottle; a nasal spray; a syringe sprayer or plunger sprayer (a syringe providing pressure to an attached sprayer or nozzle); a nasal aerosol device; a controlled particle dispersion device; a nasal nebulization device; a pressure-driven jet nebulizer; ultrasonic nebulizer; a breath-powered nasal delivery device; an atomized nasal medication device; an inhaler; a powder dispenser; a dry powder generator; an aerosolizer; an intrapulmonary aerosolizer; a sub-miniature aerosolizer; a propellant based metered dose inhaler; a dry powder inhalation device; an instillation device; an intranasal instillation device; an intravesical instillation device; a swab; a pipette; a nasal irrigation device; a nasal rinse; an aerosol device; a metered aerosol device; a pressurized dosage device; a powdered aerosol device; a spray aerosol device; a spray device; a metered spray device; and a suspension spray device.

14. Composition for use according to any one of claim 1-13, wherein administering is at least one administering method selected from the group consisting of: systemic administration; intravenous administration; intradermal administration; subcutaneous administration; intramuscular administration; intranasal administration; intranasal administration and oral administration; intraperitoneal administration; intracranial administration; intravesical administration; oral administration; topical administration; inhalation administration; aerosol administration; intra-airway administration; tracheal administration; bronchial administration; instillation; bronchoscopic instillation; intratracheal administration; mucosal administration; dry powder administration; spray administration; contact administration; swab administration; intratracheal deposition administration; intrabronchial deposition administration; bronchoscopic deposition administration; lung administration; nasal passage administration; respirable solid administration; respirable liquid administration; and dry powder inhalants administration, wherein intranasal administration is preferably at least one selected from the group consisting of: administering to nasal passages; administering to nasal epithelium; administering to lung; administering by inhalation; administering to the larynx; administering to bronchi; administering to alveoli; administering by inhalation; and administering by nasal instillation.

15. Composition for use according to any one of claims 1-14, wherein administering is administering to at least one tissue or cell selected from the group consisting of: an airway tissue; nose tissue; oral tissue; alveoli tissue; pharynx tissue; trachea tissue; bronchi tissue; carina tissue; bronchi tissue; bronchioles tissue; lung tissue; lobe of a lung tissue; alveoli tissue; nasal passage tissue; nasal epithelium tissue; larynx tissue; bronchi tissue; inhalation tissue; an epithelium cell; an airway epithelium cell; a ciliated cell; a goblet cell; a non-ciliated cell; a basal cell; a lung cell; a nasal cell; a tracheal cell; a bronchial cell; a bronchiolar epithelial cell; an alveolar epithelial cell; and a sinus cell.

16. Composition for use according to any one of claims 1-15, wherein the tdsRNA is administered
- at a dosage of about 25 mg to 700 mg of tdsRNA per day; 20 mg to 200 mg of tdsRNA per day; 50 mg to 150 mg of tdsRNA per day; or 80 mg to 140 mg of tdsRNA per day; and/or
- at a rate selected from the group consisting of: one dose per day, one dose every 2 days, one dose every 3 days, one dose every 4 days, one dose every 5 days, one dose a week, two doses a week, three doses a week, one dose every two weeks, one dose every 3 weeks, one dose every 4 weeks, and one dose a month.

## Patentansprüche

1. Zusammensetzung, umfassend therapeutische doppelsträngige RNA tdsRNA zur Verwendung bei der Behandlung von Long COVID in einem zuvor mit einem Virus infizierten Subjekt, wobei das Virus SARS-CoV-2 ist, wobei die Behandlung Verabreichen einer therapeutisch wirksamen Menge einer Zusammensetzung, umfassend therapeutische doppelsträngige RNA tdsRNA, an das Subjekt umfasst,
wobei die tdsRNA
rIn • r(C 12U)n oder eine Kombination von rIn • r(C 12U)n und robuster dsRNA ist, wobei die robuste dsRNA eine isolierte doppelsträngige Ribonukleinsäure (dsRNA) ist, die unter thermischer Belastung enzymatisch aktiv ist, umfassend:
jeden Strang mit einem Molekulargewicht von 250 kDa bis 500 kDa, 400-800 Basenpaaren oder 30 bis 60 helikalen Windungen der Duplex-RNA,
einen Einzelstrang, umfasst von
Poly(ribocytosinischer₄₋₂₉-uracilischer Säure), und einen entgegengesetzten Strang, umfasst von Poly(riboinosinischer Säure),
wobei die zwei Stränge kein Basenpaar an der Position der Uracilbase bilden,
wobei die zwei Stränge an der Position der Cytosinbase ein Basenpaar bilden, und
wobei die Stränge teilweise hybridisiert sind,:
wobei n eine Zahl mit einem Wert von 40 bis 50 000 ist,
wobei rIn einen Poly-Ribo-Inosin-Strang mit einer Länge von n Basen darstellt, und
wobei r(C₁₂U)ₙ einen ssRNA-Strang mit zufälliger Sequenz von C-und U-Basen mit einer Länge von n Basen darstellt und ein Verhältnis von C-Basen zu U-Basen in der Zufallssequenz ssRNA etwa 12 ist.

2. Zusammensetzung zur Verwendung nach Anspruch 1, wobei das SARS-CoV-2 mindestens eines ist, ausgewählt aus der Gruppe bestehend aus: einer B.1.1.7-Variante von SARS-CoV-2 (auch bekannt als 20I/501Y.V1 Besorgniserregende Variante (*Variant of Concern* - VOC) 202012/01); einer B.1.351-Variante von SARS-CoV-2 (auch bekannt als 20H/501Y.V2); einer P.1-Variante von SARS-CoV-2 (auch bekannt als 20J/501Y.V3); B.1.427 und B.1.429.

3. Zusammensetzung zur Verwendung nach Anspruch 1 oder 2,
- wobei das Subjekt Long COVID-Symptome zeigt, umfassend:
(1) mindestens ein Symptom, ausgewählt aus der Gruppe bestehend aus:
Müdigkeit einschließlich starker Müdigkeit,
Unfähigkeit, sich aufgrund von Müdigkeit zu bewegen oder aktiv zu sein, und geringe Belastbarkeit aufgrund von Müdigkeit;
und
(2) mindestens 4 Symptome, ausgewählt aus der Gruppe bestehend aus:
Kurzatmigkeit oder Atembeschwerden, anhaltende Schmerzen oder Druck in der Brust, Husten, Herzklopfen, Durchfall, teilweiser oder vollständiger Verlust des Geruchssinns, Tachykardie, Haarausfall, verschwommenes Sehen, Neuropathie in Füßen und Händen, teilweiser oder vollständiger Verlust des Geschmackssinns, Übelkeit oder Erbrechen, verstopfte Ohren, trockene Augen, Zittern oder Zittrigkeit, Flimmern oder Lichtblitze in der Sicht, Ausschlag, Tinnitus oder Brummen in den Ohren, veränderter Geschmackssinn, trockene oder sich schälende Haut, Phantomgerüche, Costochondritis, geringer Blutsauerstoff, COVID-Zehen, Soor, Dyspnoe, Schleim im Rachen, ständiger Durst, Muskelzucken, Hitzeintoleranz, abnorm niedrige Temperatur, kaltes, brennendes Gefühl in der Lunge, Kropf oder Klumpen im Hals, trockene Kopfhaut oder Schuppen, Anämie, Schilddrüsenüberfunktion, Sicca-Syndrom, rote Augen, Dysgeusie, Sputumproduktion, Appetitlosigkeit, Schwindel, Muskelschmerzen, kognitive Probleme, Gehirnnebel, Konzentrationsprobleme, Denkprobleme, Schüttelfrost, Schweißausbrüche, Schlafprobleme, Muskel- oder Körperschmerzen, Konzentrations- oder Fokussierungsschwierigkeiten, Kopfschmerzen, Schlafstörungen, Angstzustände, Gedächtnisprobleme, Schwindel, Gelenkschmerzen, Halsschmerzen, Nachtschweiß, Fieber oder Schüttelfrost, verstopfte oder laufende Nase, Traurigkeit, Reflux oder Sodbrennen, wechselnde Symptome, Bauchschmerzen, Schmerzen im unteren Rückenbereich, Kurzatmigkeit oder Erschöpfung beim Bücken, Gewichtszunahme, Wadenkrämpfe, mehr Schlaf als normal, Schmerzen im oberen Rückenbereich, Nervenempfindungen, scharfe oder plötzliche Schmerzen in der Brust, Verwirrung, Gereiztheit, Gewichtsverlust, postnasaler Tropf, trockener Hals, hoher Blutdruck, geschwollene Hände oder Füße, wunde Stellen im Mund oder wunde Zunge, Nackenmuskelschmerzen, heißer Blutandrang, Knochenschmerzen in den Extremitäten, Gefühl brennender Haut, extremer Druck am Hinterkopf oder Hinterhauptnerv, geschwollene Lymphknoten, Hirndruck, Nierenschmerzen, Blutdruckspitzen, Hand- oder Handgelenksschmerzen, hervortretende Venen, Schmerzen im mittleren Rückenbereich an der Basis der Rippen, brennendes Gefühl, schmerzhafte Kopfhaut, Kieferschmerzen, Herzrhythmusstörungen, rissige oder trockene Lippen, Fußschmerzen, Augenstich oder -infektion, niedriger Blutdruck, Nierenprobleme oder Eiweiß im Urin, Harnwegsinfektion, Hormonstörungen, drastische Persönlichkeitsveränderung, gastroösophageale Refluxkrankheit mit übermäßigem Speichelfluss, Herpesinfektion, EBV-Infektion, Trigeminusneuralgie, beidseitiges Pochen im Nacken um die Lymphknoten, Synkope, Traurigkeit, Brustschmerzen, Rhinitis und Myalgie; und/oder
- wobei das Subjekt Long Covid hat, wie definiert durch:
(a) das Subjekt COVID-19 hat, ein früher diagnostiziertes Vorhandensein von SARS-CoV-2 oder einen positiven Serumantikörpertest auf SARS-CoV-2;
(b) das Subjekt der Diagnose eines chronischen Müdigkeitssyndroms *(Chronic Fatigue Syndrome -* CFS) gemäß der CDC-Falldefinition von 1988 oder 1994 für CFS für mindestens 3 Monate entspricht, aber CFS dem (a) nicht vorausgeht;
(c) das Subjekt ein gleichzeitiges Auftreten von mindestens einem oder mehreren Long-Covid-Symptomen zeigt, die während 3 oder mehr aufeinanderfolgenden Monaten der Erkrankung anhalten oder wiederkehren, wobei die Long-Covid-Symptome nicht vor (a) auftreten, wobei das Long-Covid-Symptom mindestens eines ist, ausgewählt aus der Gruppe bestehend aus: Müdigkeit; Unwohlsein nach der Anstrengung; Kopfschmerzen; Schlafstörungen; Gedächtnisproblemen; Konzentrationsproblemen; Gehirnnebel; Fieber; Schüttelfrost; Husten; Kurzatmigkeit; Atembeschwerden; Geschmacksverlust; Geruchsverlust; und Brustschmerzen.

4. Zusammensetzung zur Verwendung nach einem der Ansprüche 1 bis 3, wobei das Subjekt
seropositiv für einen Anti-Virus-Antikörper ist;
seronegativ für den Anti-Virus-Antikörper ist;
während des Verabreichungsschritts nicht mit dem Virus infiziert ist;
während des Verabreichungsschritts mit dem Virus infiziert ist; oder
während des Verabreichungsschritts nicht wegen der Virusinfektion hospitalisiert ist.

5. Zusammensetzung zur Verwendung nach einem der Ansprüche 1 bis 4, wobei
- das Subjekt keine Long-COVID-Symptome hatte, bevor es mit dem Virus infiziert wurde,
vorzugsweise, wobei das Subjekt mindestens 1 Jahr, 2 Jahre, 3 Jahre, 4 Jahre oder 5 Jahre lang keine Long-COVID-Symptome hatte, bevor es mit dem Virus infiziert wurde; und/oder
- das Subjekt die Long-COVID-Symptome während oder nach seiner Infektion mit dem Virus entwickelt.

6. Zusammensetzung zur Verwendung nach einem der Ansprüche 1 bis 5, wobei die Long-COVID-Symptome von der Infektion bis zum Verabreichungsschritt über einen Zeitraum angedauert haben, der ausgewählt ist aus der Gruppe bestehend aus: 1 Monat, 2 Monaten, 3 Monaten, 4 Monaten, 5 Monaten, 6 Monaten, 1 Jahr, 2 Jahren und mehr als 2 Jahren.

7. Zusammensetzung zur Verwendung nach einem der Ansprüche 1 bis 6, wobei das Subjekt ein Säugetier, vorzugsweise ein Wirt des Virus, und am stärksten bevorzugt ein Mensch ist.

8. Zusammensetzung zur Verwendung nach einem der Ansprüche 1 bis 7,
wobei mindestens 90 Gew.-% der tdsRNA größer als eine Größe ist, die ausgewählt ist aus der Gruppe bestehend aus: 40 Basenpaaren; 50 Basenpaaren; 60 Basenpaaren; 70 Basenpaaren; 80 Basenpaaren; und 380 Basenpaaren; oder
wobei mindestens 90 Gew.-% der tdsRNA kleiner als eine Größe ist, die ausgewählt ist aus der Gruppe bestehend aus 50 000 Basenpaaren; 10 000 Basenpaaren; 9000 Basenpaaren; 8000 Basenpaaren; 7000 Basenpaaren; und 450 Basenpaaren.

9. Zusammensetzung zur Verwendung nach einem der Ansprüche 1 bis 8, wobei n eine Zahl mit einem Wert ist, der ausgewählt ist aus der Gruppe bestehend aus 40 bis 40 000; 50 bis 10 000; 60 bis 9000; 70 bis 8000; 80 bis 7000; und 380 bis 450.

10. Zusammensetzung zur Verwendung nach einem der Ansprüche 1 bis 9,
wobei n von 40 bis 40 000 ist;
wobei die tdsRNA 4 bis 4000 helikale Windungen von Duplex-RNA-Strängen hat; oder
wobei die tdsRNA ein Molekulargewicht hat, ausgewählt aus der Gruppe bestehend aus:
2 kDa bis 30 000 kDa;
25 kDa bis 2500 kDa; und
250 kDa bis 320 kDa.

11. Zusammensetzung zur Verwendung nach einem der Ansprüche 1 bis 10, wobei die tdsRNA
- 0,1 bis 12 Mol-% robuste dsRNA umfasst, vorzugsweise wobei die tdsRNA 0,1 bis 5 Mol-% robuste dsRNA umfasst; und/oder
- mit einem stabilisierenden Polymer komplexiert ist, wobei das stabilisierende Polymer vorzugsweise mindestens eines ist, das ausgewählt ist aus der Gruppe bestehend aus:
Polylysin; Polylysin und Carboxymethylcellulose; Polyarginin; Polyarginin und Carboxymethylcellulose; und einer Kombination davon.

12. Zusammensetzung zur Verwendung nach einem der Ansprüche 1 bis 11, wobei die Zusammensetzung mindestens einen pharmazeutisch akzeptablen Träger umfasst.

13. Zusammensetzung zur Verwendung nach einem der Ansprüche 1 bis 12,
- wobei die Verabreichung durchgeführt wird, nachdem die primären COVID-19-Symptome einer akuten Erkrankung abgeklungen sind,
- wobei das COVID-19-Symptom einer akuten Erkrankung vorzugsweise ausgewählt ist aus der Gruppe bestehend aus Fieber, Schüttelfrost, Husten, Kurzatmigkeit, Atembeschwerden, Müdigkeit, Muskelschmerzen, Körperschmerzen, Kopfschmerzen, Geschmacksverlust, Geruchsverlust, Halsschmerzen, Verstopfung, laufender Nase, Übelkeit, Erbrechen, Durchfall und einer Kombination davon, und/oder
- wobei Verabreichen durchgeführt wird, nachdem das Subjekt mit dem Virus für einen Zeitraum infiziert war, der ausgewählt ist aus der Gruppe bestehend aus 30 Tagen, 50 Tagen, 2 Monaten, 3 Monaten, 4 Monaten, 5 Monaten, 6 Monaten, 1 Jahr, 2 Jahren und mehr als 2 Jahren; und/oder
- wobei Verabreichen durch ein Abgabesystem durchgeführt wird, umfassend die tdsRNA,
wobei das Verabreichungssystem oder die medizinische Vorrichtung vorzugsweise mindestens eine ist ausgewählt aus der Gruppe, bestehend aus: einem Vernebler; einem Zerstäuber; einer Nasenpumpe; einer Quetschflasche; einem Nasenspray; einem Spritzensprüher oder Kolbensprüher (eine Spritze, die Druck auf einen angebrachten Sprüher oder eine Düse ausübt); einer nasalen Aerosolvorrichtung; einer Vorrichtung zur kontrollierten Partikeldispersion; einer nasalen Vernebelungsvorrichtung; einem druckbetriebenen Düsenvernebler; einem Ultraschallvernebler; einer atmungsbetriebenen nasalen Verabreichungsvorrichtung; einer Vorrichtung zur Vernebelung nasaler Medikamente; einem Inhalator; einem Pulverspender; einem Trockenpulvergenerator; einem Aerosolisator; einem intrapulmonalen Aerosolisator; einem Subminiatur-Aerosolisator; einem treibgasbasierten Dosierinhalator; einer Trockenpulverinhalationsvorrichtung; einer Instillationsvorrichtung; einer intranasalen Instillationsvorrichtung; einer intravesikalen Instillationsvorrichtung; einem Tupfer; einer Pipette; einer Nasenspülvorrichtung; einer Nasenspülung; einer Aerosolvorrichtung; einer Dosier-Aerosolvorrichtung; einer Druckdosiervorrichtung; einer Pulver-Aerosolvorrichtung; einer Sprüh-Aerosolvorrichtung; einer Sprühvorrichtung; einer Dosier-Sprühvorrichtung; und einer Suspensions-Sprühvorrichtung.

14. Zusammensetzung zur Verwendung nach einem der Ansprüche 1 bis 13, wobei das Verabreichen mindestens ein Verabreichungsverfahren ist, ausgewählt aus der Gruppe bestehend aus: systemischer Verabreichung; intravenöser Verabreichung; intradermaler Verabreichung; subkutaner Verabreichung; intramuskulärer Verabreichung; intranasaler Verabreichung; intranasaler Verabreichung und oraler Verabreichung; intraperitonealer Verabreichung; intrakranieller Verabreichung; intravesikaler Verabreichung; oraler Verabreichung; topischer Verabreichung; Inhalationsverabreichung; Aerosolverabreichung; bronchialer Verabreichung; trachealer Verabreichung; bronchialer Verabreichung; Instillation; bronchoskopischer Instillation; intratracheale Verabreichung; mukosale Verabreichung; Trockenpulververabreichung; Sprühverabreichung; Kontaktverabreichung; Tupferverabreichung; intratracheale Depositionsverabreichung; intrabronchiale Depositionsverabreichung; bronchoskopische Depositionsverabreichung; Lungenverabreichung; Verabreichung über die Nasenwege; Verabreichung von lungengängigen Feststoffen; Verabreichung von lungengängigen Flüssigkeiten; und Verabreichung von Trockenpulverinhalationsmitteln, wobei die intranasale Verabreichung vorzugsweise mindestens eine ist, ausgewählt aus der Gruppe bestehend aus: Verabreichung an die Nasengänge; Verabreichung an das Nasenepithel; Verabreichung an die Lunge; Verabreichung durch Inhalation; Verabreichung an den Kehlkopf; Verabreichung an die Bronchien; Verabreichung an die Alveolen; Verabreichung durch Inhalation; und Verabreichung durch nasale Instillation.

15. Zusammensetzung zur Verwendung nach einem der Ansprüche 1 bis 14, wobei das Verabreichen Verabreichen an mindestens ein Gewebe oder eine Zelle ist, ausgewählt aus der Gruppe bestehend aus: Atemwegsgewebe; Nasengewebe; Mundgewebe; Alveolargewebe; Rachengewebe; Tracheagewebe; Bronchialgewebe; Carinagewebe; Bronchialgewebe; Bronchiolengewebe; Lungengewebe; Lungenlappen; Alveolargewebe; Nasenganggewebe; Nasenepithelgewebe; Kehlkopfgewebe; Bronchialgewebe; Inhalationsgewebe; einer Epithelzelle; einer Atemwegsepithelzelle; einer Flimmerzelle; einer Becherzelle; einer nichtflimmernden Zelle; einer Basalzelle; einer Lungenzelle; einer Nasenzelle; einer Trachealzelle; einer Bronchialzelle; einer bronchiolären Epithelzelle; einer alveolären Epithelzelle; und einer Sinuszelle.

16. Zusammensetzung zur Verwendung nach einem der Ansprüche 1 bis 15, wobei die tdsRNA verabreicht wird
- in einer Dosierung von etwa 25 mg bis 700 mg tdsRNA pro Tag; 20 mg bis 200 mg tdsRNA pro Tag; 50 mg bis 150 mg tdsRNA pro Tag; oder 80 mg bis 140 mg tdsRNA pro Tag; und/oder
- mit einer Rate, ausgewählt aus der Gruppe bestehend aus: einer Dosis pro Tag, einer Dosis alle 2 Tage, einer Dosis alle 3 Tage, einer Dosis alle 4 Tage, einer Dosis alle 5 Tage, einer Dosis pro Woche, zwei Dosen pro Woche, drei Dosen pro Woche, einer Dosis alle zwei Wochen, einer Dosis alle 3 Wochen, einer Dosis alle 4 Wochen und einer Dosis pro Monat.

## Revendications

1. Composition comprenant un ARN double brin thérapeutique ARNdbt destinée à être utilisée dans le traitement de la COVID longue chez un sujet précédemment infecté par un virus, dans laquelle le virus est le SARS-CoV-2, le traitement comprenant l'administration au sujet d'une quantité thérapeutiquement efficace d'une composition comprenant un ARN double brin thérapeutique ARNdbt,
dans laquelle l'ARNdbt est
rIₙ.r(C₁₂U)ₙ, ou une combinaison de rIₙ.r(C₁₂U)ₙ et d'ARNdb robuste, dans laquelle l'ARNdb robuste est un acide ribonucléique double brin (ARNdb) isolé actif enzymatiquement sous stress thermique comprenant:
chaque brin avec un poids moléculaire de 250 kDa à 500 kDa, 400 à 800 paires de bases, ou 30 à 60 tours d'hélice d'ARN duplex,
un simple brin composé de poly(acide ribocytosinique₄₋₂₉ uracilique) et un brin opposé composé de poly(acide riboinosinique),
dans laquelle les deux brins ne forment pas d'appariement de bases à la position de la base uracile,
dans laquelle les deux brins forment un appariement de bases à la position de la base cytosine, et
dans laquelle lesdits brins sont partiellement hybridés;
dans laquelle n est un nombre avec une valeur de 40 à 50 000,
dans laquelle rIₙ représente un brin de polyribo-inosine de n bases de longueur, et
dans laquelle r(C₁₂U)ₙ représente un brin d'ARNsb de séquence aléatoire de bases C et U de n bases de longueur et un rapport de bases C à bases U dans l'ARNsb de séquence aléatoire est d'environ 12.

2. Composition destinée à être utilisée selon la revendication 1, dans laquelle le SARS-CoV-2 est au moins un choisi dans le groupe consistant en: un variant B.1.1.7 du SARS-CoV-2 (également appelé variant préoccupant (VOC) 20I/501Y.V1 202012/01); un variant B.1.351 du SARS-CoV-2 (également appelé 20H/501Y.V2); un variant P.1 du SARS-CoV-2 (également appelé 203/501Y.V3); B.1.427 et B.1.429.

3. Composition destinée à être utilisée selon la revendication 1 ou 2,
- dans laquelle le sujet présente des symptômes de la COVID longue comprenant:
(1) au moins un symptôme choisi dans le groupe consistant en:
la fatigue, y compris la fatigue intense,
une inaptitude à l'exercice ou à être actif en raison de la fatigue, et
une faible tolérance à l'exercice en raison de la fatigue;
et
(2) au moins 4 symptômes choisis dans le groupe consistant en:
un essoufflement ou une difficulté à respirer, une douleur ou pression thoracique persistante, une toux, des palpitations cardiaques, une diarrhée, une perte partielle ou totale du sens de l'odorat, une tachycardie, une perte de cheveux, une vision trouble, une neuropathie dans les pieds et les mains, une perte partielle ou totale du sens du goût, des nausées ou vomissements, des oreilles bouchées, une sécheresse oculaire, des tremblements ou des secousses, des corps flottants ou des éclairs lumineux dans le champ visuel, une éruption cutanée, des acouphènes ou bourdonnements dans les oreilles, une altération du sens du goût, une peau sèche ou qui pèle, des odeurs fantômes, une costochondrite, un faible taux d'oxygène dans le sang, des orteils COVID, un muguet, une dyspnée, un phlegmon dans l'arrière de la gorge, une soif constante, des contractions musculaires, une intolérance à la chaleur, une température anormalement basse, une sensation de brûlure au froid dans les poumons, un goitre ou boule dans la gorge, un cuir chevelu sec ou des pellicules, une anémie, une thyroïde élevée, un syndrome sec, des yeux rouges, une dysgueusie, une production d'expectorations, un manque d'appétit, des vertiges, des douleurs musculaires, des troubles cognitifs, une confusion mentale, des troubles de la concentration, des troubles de la pensée, des frissons, des sueurs, des troubles du sommeil, des douleurs musculaires ou corporelles, des difficultés de concentration ou de focalisation, des maux de tête, des difficultés à dormir, une anxiété, des troubles de la mémoire, des vertiges, des douleurs articulaires, des maux de gorge, des sueurs nocturnes, une fièvre ou des frissons, un nez bouché ou qui coule, une tristesse, un reflux ou des brûlures d'estomac, des symptômes changeants, des douleurs abdominales, une lombalgie, un essoufflement ou un épuisement dû à la flexion, une prise de poids, des crampes aux mollets, un sommeil plus long que la normale, des douleurs dans le haut du dos, des sensations de malaise, une douleur thoracique aiguë ou soudaine, une confusion, une irritabilité, une perte de poids, un écoulement post-nasal, une sécheresse de la gorge, une hypertension artérielle, un gonflement des mains ou des pieds, des aphtes ou une langue douloureuse, des douleurs musculaires au niveau du cou, un échauffement, des douleurs osseuses aux extrémités, une sensation de peau qui brûle, une pression extrême à la base de la tête ou du nerf occipital, un gonflement des ganglions lymphatiques, une pression cérébrale, une douleur rénale, des pics de tension artérielle, des douleurs à la main ou au poignet, un gonflement des veines, des douleurs lombaires à la base des côtes, des sensations de brûlure, un cuir chevelu douloureux, une douleur à la mâchoire, une arythmie, des lèvres gercées ou sèches, une douleur aux pieds, un orgelet ou une infection oculaire, une hypotension artérielle, des problèmes rénaux ou des protéines dans les urines, une infection urinaire, des déséquilibres hormonaux, un changement radical de personnalité, un reflux gastro-œsophagien avec salivation excessive, une infection herpétique, une infection par EBV, une névralgie du trijumeau, une pulsation cervicale bilatérale autour des ganglions lymphatiques, une syncope, une tristesse, une douleur thoracique, une rhinite et une myalgie; et/ou
- dans laquelle le sujet a la Covid longue, comme défini par:
(a) le sujet a la COVID-19, une présence déjà diagnostiquée de SARS-CoV-2 ou un test sérique d'anticorps anti-SARS-CoV-2 positif;
(b) le sujet répond au diagnostic du syndrome de fatigue chronique (SFC) défini par la définition de cas des CDC de 1988 ou 1994 pour le SFC depuis au moins 3 mois, mais le SFC ne précède pas le (a);
(c) le sujet a simultanément au moins un ou plusieurs symptômes de Covid longue qui ont persisté ou sont réapparus pendant 3 mois consécutifs de maladie ou plus, dans laquelle les symptômes de Covid longue ne précédant pas (a), dans laquelle les symptômes de Covid longue sont au moins un choisi dans le groupe consistant en: la fatigue; un malaise post-exertionnel; les céphalées; les troubles du sommeil; les problèmes de mémoire; les problèmes de concentration; le brouillard cérébral; la fièvre; les frissons; la toux; l'essoufflement; les difficultés respiratoires; la perte du goût; la perte de l'odorat; et la douleur thoracique.

4. Composition destinée à être utilisée selon l'une quelconque des revendications 1 à 3, dans laquelle le sujet
est séropositif à un anticorps anti-virus;
est séronégatif à l'anticorps anti-virus;
est non infecté par le virus lors de l'étape d'administration;
est infecté par le virus lors de l'étape d'administration; ou
est non hospitalisé pour l'infection virale lors de l'étape d'administration.

5. Composition destinée à être utilisée selon l'une quelconque des revendications 1 à 4, dans laquelle
- le sujet n'avait pas de symptômes de COVID longue avant d'être infecté par le virus,
de préférence, le sujet n'avait pas de symptômes de COVID longue pendant au moins 1 an, 2 ans, 3 ans, 4 ans ou 5 ans avant d'être infecté par le virus; et/ou
- le sujet développe les symptômes de COVID longue pendant ou après l'infection par le virus.

6. Composition destinée à être utilisée selon l'une quelconque des revendications 1 à 5, dans laquelle les symptômes de COVID longue ont duré de l'infection à l'étape d'administration, pendant une durée choisie dans le groupe consistant en: 1 mois, 2 mois, 3 mois, 4 mois, 5 mois, 6 mois, 1 an, 2 ans et plus de 2 ans.

7. Composition destinée à être utilisée selon l'une quelconque des revendications 1 à 6, dans laquelle le sujet est un mammifère, de préférence un hôte du virus, et de manière particulièrement préférable un humain.

8. Composition destinée à être utilisée selon l'une quelconque des revendications 1 à 7, dans laquelle au moins 90 % en poids de l'ARNdbt est supérieur à une taille choisie dans le groupe consistant en: 40 paires de bases; 50 paires de bases; 60 paires de bases; 70 paires de bases; 80 paires de bases; et 380 paires de bases; ou
dans laquelle au moins 90 % en poids de l'ARNdbt est inférieur à une taille choisie dans le groupe consistant en: 50 000 paires de bases; 10 000 paires de bases; 9000 paires de bases; 8000 paires de bases; 7000 paires de bases; et 450 paires de bases.

9. Composition destinée à être utilisée selon l'une quelconque des revendications 1 à 8, dans laquelle n est un nombre avec une valeur choisie dans le groupe consistant en: 40 à 40 000; 50 à 10 000; 60 à 9000; 70 à 8000; 80 à 7000; et 380 à 450.

10. Composition destinée à être utilisée selon l'une quelconque des revendications 1 à 9,
dans laquelle n est de 40 à 40 000;
dans laquelle l'ARNdbt a 4 à 4000 tours d'hélice de brins d'ARN duplexé;
ou
dans laquelle l'ARNdbt a un poids moléculaire choisi dans le groupe consistant en:
2 kDa à 30 000 kDa;
25 Da à 2500 kDa; et
250 kDa à 320 kDa.

11. Composition destinée à être utilisée selon l'une quelconque des revendications 1 à 10, dans laquelle l'ARNdbt
- comprend 0,1 à 12 mol % d'ARNdb robuste, de préférence l'ARNdbt comprend 0,1 à 5 mol % d'ARNdb robuste; et/ou
- est complexé avec un polymère stabilisant, dans laquelle le polymère stabilisant est de préférence au moins un choisi dans le groupe consistant en:
la polylysine; la polylysine et la carboxyméthylcellulose; la polyarginine; la polyarginine et la carboxyméthylcellulose; et une combinaison de celles-ci.

12. Composition destinée à être utilisée selon l'une quelconque des revendications 1 à 11, dans laquelle la composition comprend au moins un véhicule pharmaceutiquement acceptable.

13. Composition destinée à être utilisée selon l'une quelconque des revendications 1 à 12,
- dans laquelle l'administration est effectuée après que les symptômes de COVID-19 primaires de maladie aiguë ont été résolus,
dans laquelle le symptôme de COVID-19 de maladie aiguë est de préférence choisi dans le groupe consistant en: la fièvre, les frissons, la toux, l'essoufflement, les difficultés respiratoires, la fatigue, les douleurs musculaires, les courbatures, les maux de tête, la perte de goût, la perte d'odorat, les maux de gorge, la congestion, l'écoulement nasal, les nausées, les vomissements, la diarrhée, et une combinaison de ceux-ci, et/ou
- dans laquelle l'administration est effectuée après que le sujet a été infecté par le virus pendant une durée choisie dans le groupe consistant en 30 jours, 50 jours, 2 mois, 3 mois, 4 mois, 5 mois, 6 mois, 1 an, 2 ans et plus de 2 ans; et/ou
- dans laquelle l'administration est effectuée par un système d'administration comprenant l'ARNdbt,
dans laquelle le système d'administration ou le dispositif médical est de préférence au moins un choisi dans le groupe consistant en: un nébuliseur; un pulvérisateur; une pompe nasale; un flacon souple; un spray nasal; un pulvérisateur à seringue ou un pulvérisateur à piston (une seringue exerçant une pression sur un pulvérisateur ou une buse fixé); un dispositif à aérosol nasal; un dispositif de dispersion contrôlée de particules; un dispositif de nébulisation nasale; un nébuliseur à jet sous pression; un nébuliseur à ultrasons; un dispositif d'administration nasale par la respiration; un dispositif de médication nasale atomisée; un inhalateur; un distributeur de poudre; un générateur de poudre sèche; un aérosoliseur; un aérosoliseur intrapulmonaire; un aérosoliseur sous-miniature; un inhalateur doseur à propulseur; un dispositif d'inhalation de poudre sèche; un dispositif d'instillation; un dispositif d'instillation intranasale; un dispositif d'instillation intravésicale; un écouvillon; une pipette; un dispositif d'irrigation nasale; un rinçage nasal; un dispositif à aérosol; un dispositif à aérosol doseur; un dispositif de dosage sous pression; un dispositif à aérosol en poudre; un dispositif à aérosol par pulvérisation; un dispositif de pulvérisation; un dispositif de pulvérisation dosée; et un dispositif de pulvérisation de suspension.

14. Composition destinée à être utilisée selon l'une quelconque des revendications 1 à 13, dans laquelle l'administration est au moins un procédé d'administration choisi dans le groupe consistant en: l'administration systémique; l'administration intraveineuse; l'administration intradermique; l'administration sous-cutanée; l'administration intramusculaire; l'administration intranasale; l'administration intranasale et l'administration orale; l'administration intrapéritonéale; l'administration intracrânienne; l'administration intravésicale; l'administration orale; l'administration topique; l'administration par inhalation; l'administration par aérosol; l'administration intra-voies aériennes; l'administration trachéale; l'administration bronchique; l'instillation; l'instillation bronchoscopique; l'administration intratrachéale; l'administration muqueuse; l'administration de poudre sèche; l'administration par pulvérisation; l'administration par contact; l'administration par écouvillon; l'administration par dépôt intratrachéal; l'administration par dépôt intrabronchique; l'administration par dépôt bronchoscopique; l'administration pulmonaire; l'administration dans les voies nasales; l'administration sous forme solide respirable; l'administration sous forme liquide respirable; et l'administration d'inhalants sous forme de poudre sèche, dans laquelle l'administration intranasale est de préférence au moins une choisie dans le groupe consistant en: l'administration aux voies nasales; l'administration à l'épithélium nasal; l'administration au poumon; l'administration par inhalation; l'administration au larynx; l'administration aux bronches; l'administration aux alvéoles; l'administration par inhalation; et l'administration par instillation nasale.

15. Composition destinée à être utilisée selon l'une quelconque des revendications 1 à 14, dans laquelle l'administration est l'administration à au moins un tissu ou cellule choisi dans le groupe consistant en: un tissu des voies respiratoires; un tissu nasal; un tissu buccal; un tissu alvéolaire; un tissu du pharynx; un tissu de la trachée; un tissu des bronches; un tissu de la carène trachéale; un tissu des bronches; un tissu des bronchioles; un tissu du poumon; un tissu d'un lobe du poumon; un tissu alvéolaire; un tissu des voies nasales; un tissu de l'épithélium nasal; un tissu du larynx; un tissu des bronches; un tissu d'inhalation; une cellule épithéliale; une cellule de l'épithélium des voies respiratoires; une cellule ciliée; une cellule caliciforme; une cellule non ciliée; une cellule basale; une cellule pulmonaire; une cellule nasale; une cellule trachéale; une cellule bronchique; une cellule épithéliale bronchiolaire; une cellule épithéliale alvéolaire; et une cellule sinusale.

16. Composition destinée à être utilisée selon l'une quelconque des revendications 1 à 15, dans laquelle l'ARNdbt est administré
- à une dose d'environ 25 mg à 700 mg d'ARNdbt par jour; 20 mg à 200 mg d'ARNdbt par jour; 50 mg à 150 mg d'ARNdbt par jour; ou 80 mg à 140 mg d'ARNdbt par jour; et/ou
- à un rythme choisi dans le groupe consistant en: une dose par jour, une dose tous les 2 jours, une dose tous les 3 jours, une dose tous les 4 jours, une dose tous les 5 jours, une dose par semaine, deux doses par semaine, trois doses par semaine, une dose toutes les deux semaines, une dose toutes les 3 semaines, une dose toutes les 4 semaines et une dose par mois.
